# FASCICULE DE BREVET EUROPEEN

(11) **EP 1 931 341 B1**
(45) Date de publication et mention de la délivrance du brevet: **04.11.2009**
(21) Numéro de dépôt: 06778790.3
(22) Date de dépôt: 06.07.2006
(51) Int. Cl.: A61K 31/4439, A61K 31/454, C07D 401/12, A61P 25/00

(54) **DERIVES DE 1H-INDOLE-PYRIDINECARBOXAMIDE ET DE 1H-INDOLE-PIPERIDINECARBOXAMIDE ET LEUR UTILISATION COMME INDUCTEURS DE TYROSINE HYDROXYLASE**
1H-INDOL-PYRIDINCARBONSÄUREAMID UND 1H-INDOL-PIPERIDINCARBONSÄUREAMID-DERIVATE UND IHRE VERWENDUNG ALS TYROSIN HYDROXYLASE-INDUKTOREN
1H-INDOLE-PYRIDINECARBOXAMIDE AND 1H-INDOLE-PIPERIDINECARBOXAMIDE DERIVATIVES AND THEIR USE AS TYROSINE HYDROXYLASE INDUCERS

(30) Priorité: 07.07.2005 FR 0507225
(43) Date de publication de la demande: 18.06.2008
(73) Titulaire: Les Laboratoires Servier, 92415 Courbevoie Cedex (FR); Centre National de la Recherche Scientifique, 75794 Paris Cedex 16 (FR); UNIVERSITE PARIS-SUD (PARIS 11), 91405 Orsay (FR)
(72) Inventeur: BRION, Jean-Daniel, F-95320 Saint Leu la Fôret (FR); BINTEIN, Fabrice, F-78140 Velizy (FR); RAZET, Rodolphe, F-68400 Ridiesheim (FR); RAZON, Patrick, F-75011 Paris (FR); RENKO, Zafiarisoa, Dolor, F-91940 Les Ulis (FR); LEVOIRIER, Eric, F-94600 Choisy Le Roi (FR); PUJOL, Jean-François, F-75007 Paris (FR); WEISSMANN, Dinah, F-75007 Paris (FR); LE RIDANT, Alain, FR-92200 Neuilly Sur Seine (FR); HARPEY, Catherine, F-75016 Paris (FR)
(86) Numéro de dépôt international: PCT/FR2006/001610
(87) Numéro de publication internationale: WO 2007/006922

(56) Documents cités:
- EP-A- 0 563 916
- US-A- 5 622 957

## Description

La présente invention concerne de nouveaux dérivés 1*H*-indole-pyridinecarboxamides et 1*H*-indole-pipéridinecarboxamide, leur procédé de préparation et les compositions pharmaceutiques qui les contiennent.

La littérature fournit de nombreux exemples de composés possédant une structure éburnane. C'est le cas notamment du brevet US 3 454 583 traitant de la vincamine ((3α,14β,16α)-(14,15-dihydro-14-hydroxy-éburnaménine-14-carboxylate de méthyle) et de ses dérivés pour leurs propriétés vasodilatatrices. Les demandes de brevets FR 2 433 528 et FR 2 381 048 présentent de nouveaux dérivés de 20,21-minorébumaménine et la demande EP 0 287 468, de nouveaux dérivés des 17-aza-20,21-dinorébumaménine. La demande de brevet EP 0 658 557 décrit des dérivés de l'éburnane modifiés en position 14 et 15 du squelette éburnane. La demande de brevet EP 0 563 916 décrit des dérivés de 1*H*-indole-cyclohexanecarboxamide.

Les composés de la présente invention outre le fait qu'ils soient nouveaux, présentent des propriétés pharmacologiques très intéressantes. Ils se révèlent notamment être de puissants inducteurs de tyrosine hydroxylase, de manière sélective ou non.

Plus particulièrement, la présente invention concerne les composés de formule (I) : 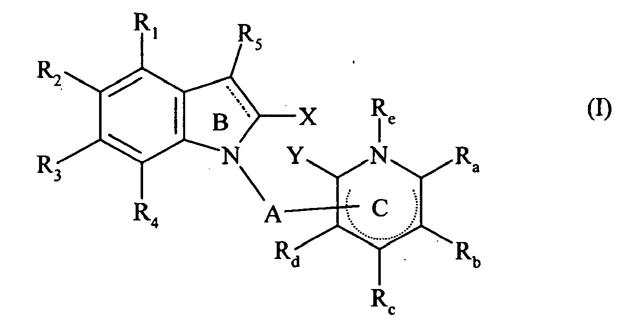 dans laquelle :
- A représente un radical bivalent : 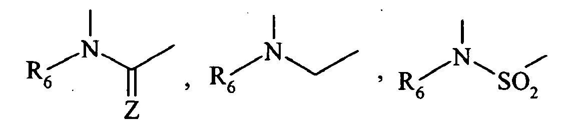 dans lesquels :
   Z représente un atome d'oxygène ou un atome de soufre,
   R₆ représente :
      un atome d'hydrogène,
      un groupement alkyle (C₁-C₆) linéaire ou ramifié, C(O)-AA dans lequel AA représente un radical amino-acide, alkoxycarbonyle (C₁-C₆) linéaire ou ramifié, CHR'-O-C(O)-R" dans lequel R' représente un atome d'hydrogène ou un groupement alkyle (C₁-C₆) linéaire ou ramifié et R" représente un alkyle (C₁-C₆) linéaire ou ramifié,
      alkényle (C₂-C₆) linéaire ou ramifié, aryle, arylalkyle (C₁-C₆) linéaire ou ramifié, polyhalogénoalkyle (C₁-C₆) linéaire ou ramifié,
      ou une chaîne alkyle (C₁-C₆) linéaire ou ramifié substituée par un ou plusieurs atomes d'halogène, un ou plusieurs groupements hydroxy, alkoxy (C₁-C₆) linéaire ou ramifié, ou amino éventuellement substitué par un ou deux groupements identiques ou différents, alkyle (C₁-C₆) linéaire ou ramifié,
- dans le cycle B,
   ----- représente une liaison simple ou une liaison double,
- dans le cycle C,
   ----- représente une liaison simple ou une liaison double, le cycle C ne contenant tout au plus qu'une seule liaison double,
- R₁, R₂, R₃, R₄, identiques ou différents, indépendamment les uns des autres, représentent:
   un atome d'hydrogène ou halogène,
   un groupement alkyle (C₁-C₆) linéaire ou ramifié, alkoxy (C₁-C₆) linéaire ou ramifié, hydroxy, cyano, nitro, polyhalogénoalkyle (C₁-C₆) linéaire ou ramifié, amino (éventuellement substitué par un ou deux groupements alkyle (C₁-C₆) linéaire ou ramifié,
   et/ou alkényle (C₂-C₆) linéaire ou ramifié, les groupements alkyle et alkényle pouvant être identiques ou différents),
   ou une chaîne alkyle (C₁-C₆) linéaire ou ramifiée substituée par un ou plusieurs atomes d'halogène, un ou plusieurs groupements hydroxy, alkoxy (C₁-C₆) linéaire ou ramifié, ou amino éventuellement substitué par un ou deux groupements, alkyle (C₁-C₆) linéaire ou ramifié, identiques ou différents,
- R₅ représente :
   un atome d'hydrogène,
   un groupement alkyle (C₁-C₆) linéaire ou ramifié, un aminoalkyle (C₁-C₆) linéaire ou ramifié, ou un groupement hydroxyalkyle (C₁-C₆) linéaire ou ramifié,
- X, Y, identiques ou différents, indépendamment les uns des autres, représentent : un atome d'hydrogène, ou un groupement alkyle (C₁-C₆) linéaire ou ramifié,
- Rₐ, R_{b}, R_{c}, R_{d}, identiques ou différents, indépendamment les uns des autres, représentent :
   un atome d'hydrogène, halogène,
   un groupement alkyle (C₁-C₆) linéaire ou ramifié, hydroxy, alkoxy (C₁-C₆) linéaire ou ramifié, cyano, nitro, polyhalogénoalkyle (C₁-C₆) linéaire ou ramifié, amino (éventuellement substitué par un ou deux groupements identiques ou différents, alkyle (C₁-C₆) linéaire ou ramifié),
   une chaîne alkyle (C₁-C₆) linéaire ou ramifiée substituée par un ou plusieurs groupements choisis parmi halogène, hydroxy, alkoxy (C₁-C₆) linéaire ou ramifié, ou amino éventuellement substitué par un ou deux groupements identiques ou différents, alkyle (C₁-C₆) linéaire ou ramifié,
   étant entendu que lorsque A est lié au cycle C par un des atomes de carbone qui porte l'un des substituants Rₐ, R_{b}, R_{c}, R_{d} ou Y, et que ledit atome de carbone de liaison porte aussi une double liaison, le substituant Rₐ, R_{b}, R_{c}, R_{d} ou Y correspondant est absent,
- Rₑ représente :
   un atome d'hydrogène,
   un groupement alkyle (C₁-C₆) linéaire ou ramifié, arylalkyle (C₁-C₆) linéaire ou ramifié, alkényle (C₂-C₆) linéaire ou ramifié, alkynyle (C₂-C₆) linéaire ou ramifié, une chaîne alkyle (C₁-C₆) linéaire ou ramifiée substituée par un ou plusieurs groupements choisis parmi hydroxy; amino (éventuellement substitué par un ou deux groupements identiques ou différents, alkyle (C₁-C₆) linéaire ou ramifié), alkoxy (C₁-C₆) linéaire ou ramifié, ou NR₇R₈ dans lequel R₇ et R₈ forment ensemble avec l'atome d'azote qui les porte, un hétérocycle de 4 à 8 chaînons éventuellement substitué, contenant éventuellement une ou plusieurs doubles liaisons au sein de l'hétérocycle et contenant éventuellement au sein du système cyclique un second hétéroatome choisi parmi l'atome d'oxygène et l'atome d'azote, une chaîne alkényle (C₂-C₆)linéaire ou ramifiée substituée par les mêmes groupements que la chaîne alkyle et une chaîne alkynyle (C₂-C₆) linéaire ou ramifiée substituée par les mêmes groupements que la chaîne alkyle,
leurs énantiomères, diastéréoisomères, N-oxydes, ainsi que leurs sels d'addition à un acide ou à une base pharmaceutiquement acceptable,
étant entendu que :
par hétérocycle de 4 à 8 chaînons éventuellement substitué, contenant éventuellement une ou plusieurs doubles liaisons au sein de l'hétérocycle et contenant éventuellement au sein du système cyclique, un second hétéroatome choisi parmi l'atome d'oxygène ou l'atome d'azote, on peut nommer à titre non limitatif la pyrrolidine, la pipéridine, l'azépane, la pipérazine, la morpholine, ces hétérocycles pouvant éventuellement être substitués, y compris sur le deuxième atome d'azote de la pipérazine par un ou plusieurs groupements, identiques ou différents, choisis parmi alkyle (C₁-C₆) linéaire ou ramifié, hydroxyalkyle (C₁-C₆) linéaire ou ramifié, alkoxy (C₁-C₆)alkyle(C₁-C₆) linéaire ou ramifié, CO₂Rᵥ, CO₂-R_{w}-NRᵥR'ᵥ, CO₂-R_{w}-ORᵥ (dans lesquels Rᵥ représente un atome d'hydrogène ou un groupement alkyle (C₁-C₆) linéaire ou ramifié, R'ᵥ a les mêmes définitions que Rᵥ et R_{w} représente une chaîne alkylène (C₁-C₆) linéaire ou ramifiée), aryle, aryloxycarbonyle, arylalkoxy (C₁-C₆) carbonyle linéaire ou ramifié, cycloalkyle éventuellement substitué, cycloalkylalkyle éventuellement substitué, hétérocycloalkyle éventuellement substitué, hétérocycloalkylalkyle éventuellement substitué, aminoalkyle (C₁-C₆) linéaire ou ramifié, la partie amino étant éventuellement substituée par un ou deux groupements identiques ou différents, alkyle (C₁-C₆) linéaire ou ramifié,
par aryle, on entend un groupement phényle ou naphtyle, les deux étant éventuellement substitués par un ou plusieurs atomes d'halogène, groupements nitro, amino, alkyle (C₁-C₆) linéaire ou ramifié, ou alkoxy (C₁-C₆) linéaire ou ramifié,
par cycloalkyle, on entend un groupement monocyclique saturé contenant de 4 à 8 chaînons,
par cycloalkylalkyle, on entend le groupement cycloalkyle-alkyle dans lequel le groupement alkyle désigne une chaîne de 1 à 6 atomes de carbone, linéaire ou ramifié et le groupement cycloalkyle désigne un groupement monocyclique saturé contenant de 4 à 8 chaînons,
par hétérocycloalkyle, on entend un groupement monocyclique saturé contenant de 4 à 8 chaînons et possédant 1 ou 2 hétéroatomes choisis parmi azote, oxygène et soufre,
par hétérocycloalkylalkyle, on entend le groupement hétérocycloalkyle-alkyle dans lequel le groupement alkyle désigne une chaîne de 1 à 6 atomes de carbone, linéaire ou ramifié et le groupement hétérocycloalkyle désigne un groupement monocyclique saturé contenant de 4 à 8 chaînons et possédant 1 ou 2 hétéroatomes choisis parmi azote, oxygène et soufre,
l'expression "éventuellement substitué" lorsqu'elle concerne les groupements cycloalkyle, cycloalkylalkyle, hétérocycloalkyle, hétérocycloalkylalkyle signifie que ces groupements peuvent être substitués par 1 ou plusieurs substituants, identiques ou différents, choisis parmi alkyle (C₁-C₆) linéaire ou ramifié, hydroxyalkyle (C₁-C₆) linéaire ou ramifié alkoxy (C₁-C₆)alkyle(C₁-C₆) linéaire ou ramifié, carboxy, alkoxycarbonyle (C₁-C₆) linéaire ou ramifié, aminoalkyle (C₁-C₆) linéaire ou ramifié, la partie amino étant éventuellement substituée par un ou deux groupements identiques ou différents, alkyle (C₁-C₆) linéaire ou ramifié,
par radical amino-acide, on entend les radicaux alanyl, arginyl, asparaginyl, α-aspartyl, cystéinyl, α-glutamyl, glutaminyl, glycyl, histidyl, isoleucyl, leucyl, lysyl, méthionyl, phénylalanyl, prolyl, seryl, thréonyl, thryptophyl, tyrosyl et valyl.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthane sulfonique, camphorique, la lysine, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont ceux pour lesquels A représente un radical bivalent : 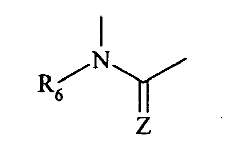 dans lequel R₆ est tel que défini dans la formule (I) et Z représente un atome d'oxygène.

Le substituant R₆ préféré selon l'invention est l'atome d'hydrogène.

Les substituants R₁, R₂, R₃ et R₄ préférés selon l'invention sont l'atome d'hydrogène, l'atome d'halogène ou le groupement alkoxy (C₁-C₆) linéaire ou ramifié.

Le substituant R₅ préféré selon l'invention sont l'atome d'hydrogène ou le groupement alkyle (C₁-C₆) linéaire ou ramifié.

Les substituants X et Y préférés selon l'invention sont l'atome d'hydrogène, ou le groupement alkyle (C₁-C₆) linéaire ou ramifié.

Les substituants Rₐ, R_{b}, R_{c} et R_{d} préférés selon l'invention sont l'atome d'hydrogène.

Le substituant Rₑ préféré selon l'invention sont l'atome d'hydrogène, ou le groupement alkyle (C₁-C₆) linéaire ou ramifié, ou alkényle (C₂-C₆) linéaire ou ramifié.

Selon une variante avantageuse de l'invention, les composés préférés sont les composés de formule (IA) : 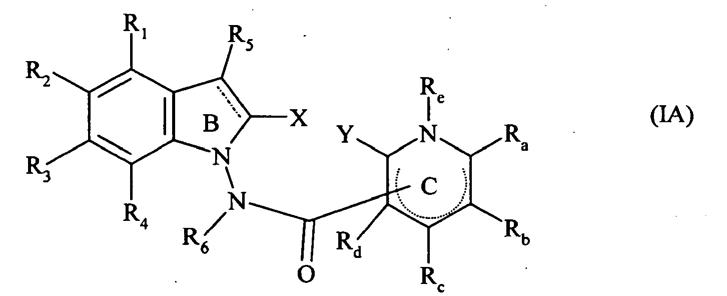 dans laquelle -----, X, Y, R₁, R₂, R₃, R₄, R₅, R₆, Rₐ, R_{b}, R_{c}, R_{d} et Rₑ sont tels que définis dans la formule (I).

Selon une deuxième variante avantageuse de l'invention, les composés préférés sont les composés de formule (IB) : 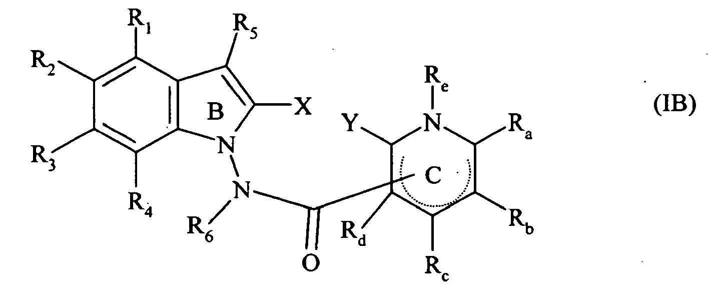 dans laquelle -----, X, Y, R₁, R₂, R₃, R₄, R₅, R₆, Rₐ, R_{b}, R_{c}, R_{d} et Rₑ sont tels que définis dans la formule (I).

Selon une troisième variante avantageuse de l'invention, les composés préférés sont les composés de formule (IC) : 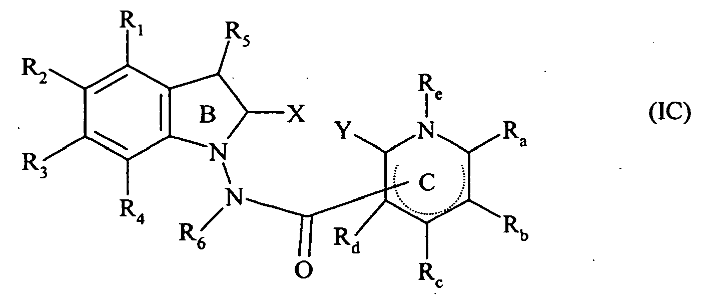 dans laquelle -----, X, Y, R₁, R₂, R₃, R₄, R₅, R₆, Rₐ, R_{b}, R_{c}, R_{d} et Rₑ sont tels que définis dans la formule (I).

Selon une quatrième variante avantageuse de l'invention, les composés préférés sont les composés de formule (ID) : 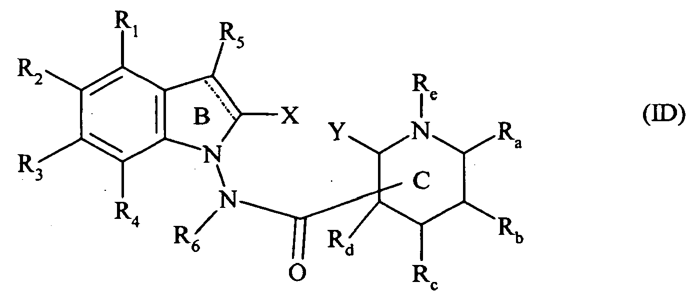 dans laquelle -----, X, Y, R₁, R₂, R₃, R₄, R₅, R₆, Rₐ, R_{b}, R_{c}, R_{d} et Rₑ sont tels que définis dans la formule (I).

Selon une cinquième variante avantageuse de l'invention, les composés préférés sont les composés de formule (IE) : 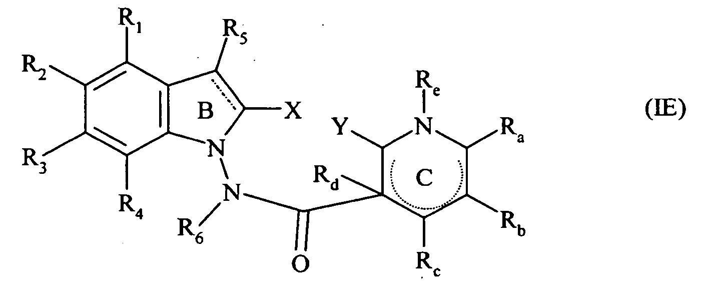 dans laquelle -----, X Y, R₁, R₂, R₃, R₄, R₅, R₆, Rₐ, R_{b}, R_{c}, R_{d} et Rₑ sont tels que définis dans la formule (I).

Selon une sixième variante avantageuse de l'invention, les composés préférés sont les composés de formule (IF) : 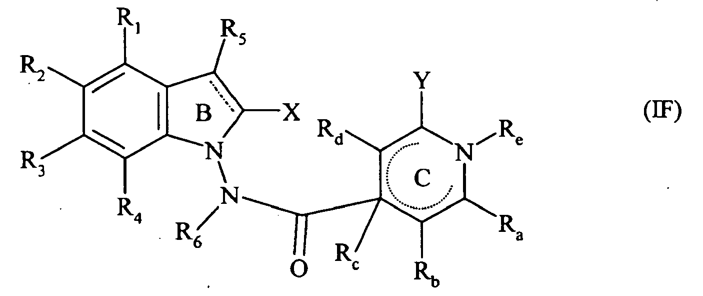 dans laquelle -----, X, Y, R₁, R₂, R₃, R₄, R₅, R₆, Rₐ, R_{b}, R_{c}, R_{d} et Rₑ sont tels que défmis dans la formule (I).

Selon une septième variante avantageuse de l'invention, les composés préférés sont les composés de formule (IG) : 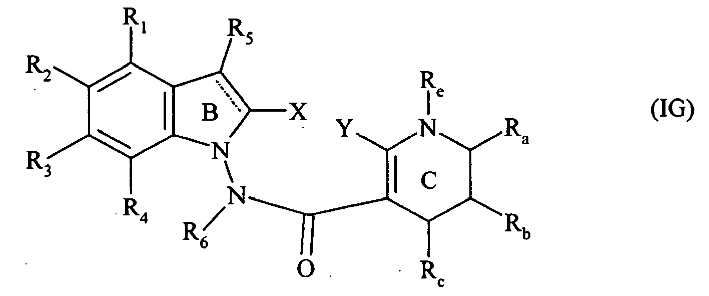 dans laquelle -----, X, Y, R₁, R₂, R₃, R₄, R₅, R₆, Rₐ, R_{b}, R_{c} et Rₑ sont tels que définis dans la formule (I).

Selon une huitième variante avantageuse de l'invention, les composés préférés sont les composés de formule (IH) : 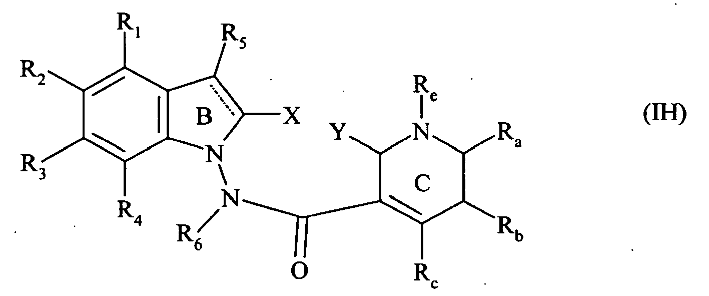 dans laquelle -----, X, Y, R₁, R₂, R₃, R₄, R₅, R₆, Rₐ, R_{b}, R_{c} et Rₑ sont tels que définis dans la formule (I).

Selon une neuvième variante avantageuse de l'invention, les composés préférés sont les composés de formule (IJ) : 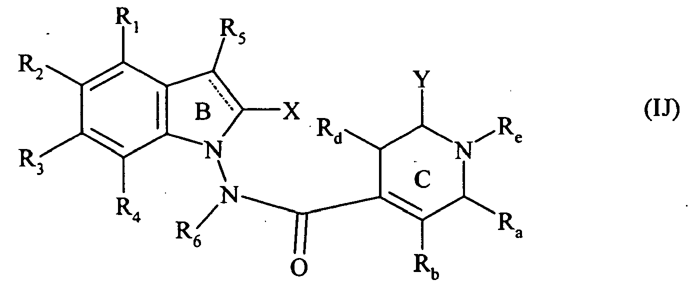 dans laquelle -----, X, Y, R₁, R₂, R₃, R₄, R₅, R₆, Rₐ, R_{b}, R_{d} et Rₑ sont tels que définis dans la formule (I).

Les composés préférés selon l'invention sont le :
- *N*-(1*H*-indol-1-yl)-1-méthyl-1,2,5,6-tétrahydropyridine-3-carboxamide,
- *N*-(2,3-dihydro-1*H*-indol-1-yl)-1-méthyl-1,4,5,6-tétrahydropyridine-3-carboxamide,
- *N*-(5-fluoro-1*H*-indol-1-yl)-1-méthyl-1,2,5,6-tétrahydropyridine-3-carboxamide,
- *N*-(2,3-dihydro-1*H*-indol-1-yl)-1-méthyl-1,4,5,6-tétrahydropyridine-3-carboxamide,
- 1-[2-(diméthylamino)éthyl]-*N*-(1*H*-indol-1-yl)-1,2,5,6-tétrahydropyridine-3-carboxamide,
- *N*-(1*H*-indol-1-yl)-1-[2-(4-méthyl-1-pipérazinyl)éthyl]-3-pipéridinecarboxamide,
- *N*-(5-chloro-1*H*-indol-1-yl)-1-(2-hydroxyéthyl)-1,4,5,6-tétrahydropyridine-3-carboxamide,
- 4-(2-{3-[(1*H*-indol-1-ylamino)carbonyl]-1-pipéridinyl}éthyl)-pipérazine-1-carboxylate de *tert*-butyle,
- 1-[3-(diméthylammonium)propyl]-3-[(1*H*-indol-1-ylamino)carbonyl]pipéridinium,
- *N*-(1*H*-indol-1-yl)-1-[3-(1-pipéridinyl)propyl]-3-pipéridinecarboxamide,
- *N*-(1*H*-indol-1-yl)-1-[3-(4-méthyl-1-pipérazinyl)propyl]-3-pipéridinecarboxamide,
- N*-*(Indol-1-yl)-1-(2-pipéridin-1-yl-éthyl)-1,2,5,6-tétrahydropyridine-3-carboxamide,
- (±)-N-(indol-1-yl)-1-[2-[4-(1-méthylpipéridin-4-yl)pipérazin-1-yl)]éthyl]pipéridine-3-carboxamide,
- (±)-N-(indol-1-yl)-1-[3-[4-(2-hydroxyéthyl) pipérazin-1-yl)]propyl]pipéridine-3-carboxamide,
- (±)-N-(indol-1-yl)1-[4-(4-méthylpipérazin-1-yl)butyl]pipéridine-3-carboxamide,
- (±)-N-(Indol-1-yl)-1-allylpipéridine-3-carboxamide,
- (±)-N-(Indol-1-yl)-1-[4-(pipéridin-1-yl)but-2-èn-1-yl]-pipéridine-3-carboxamirie,
- (R ou S) (-)-N-(Indol-1-yl)-1-[2-(pipéridin-1-yl)éthyl]pipéridine-3-carboxamide énantiomère 1,
- (R ou S) (+)-N-(Indol-1-yl)-1-[2-(pipéridin-1-yl)éthyl]pipéridine-3-carboxamide énantiomère 2.

Les énantiomères, diastéréoisomères, N-oxydes, ainsi que les sels d'addition à un acide ou à une base, pharmaceutiquement acceptables, des composés préférés font partie intégrante de l'invention.

La présente invention concerne aussi le procédé de préparation de composés de formule (I), **caractérisé en ce qu'**on utilise comme produit de départ un composé de formule (II) : 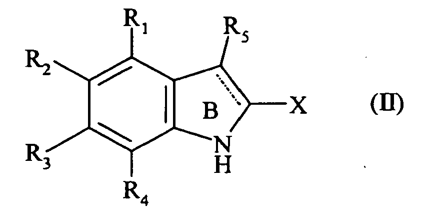 dans laquelle R₁, R₂, R₃, R₄, R₅ et X sont tels que définis dans la formule (I),
composé que l'on fait réagir avec la diphénylphosphinylhydroxylamine, pour conduire au composé de formule (III) : 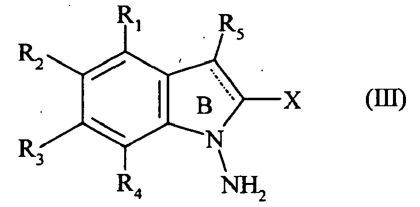 dans laquelle R₁, R₂, R₃, R₄, R₅ et X sont tels que définis précédemment,
composé de formule (III) qui est condensé avec un composé de formule (IV) : 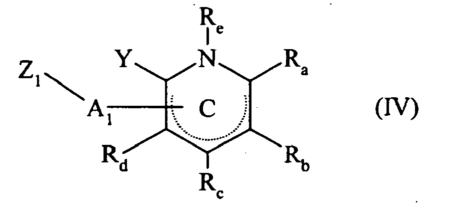 dans laquelle Rₐ, R_{b}, R_{c}, R_{d}, Rₑ et Y sont tels que définis dans la formule (I), A₁ représente un groupement de formule -C(=Z)-, -CH₂-, -SO₂- dans lequel Z est tel que défini dans la formule (I) et Z₁ représente un groupement choisi parmi hydroxy, éthoxy ou méthoxy,
pour conduire au composé de formule (I/a), cas particulier des composés de formule (I) : 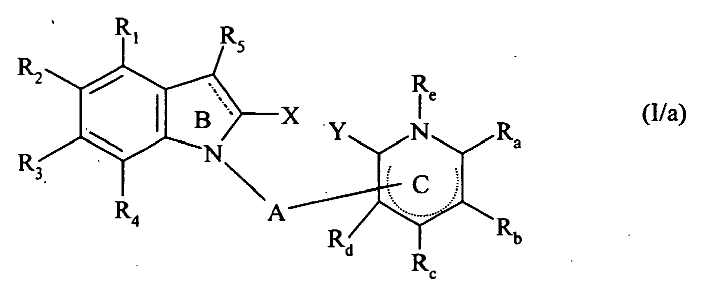 dans laquelle R₁, R₂, R₃, R₄, R₅, Rₐ, R_{b}, R_{c}, R_{d}, Rₑ, X et Y sont tels que définis précédemment et A est tel que défini dans la formule (I),
le composé de formule (I/a) formant l'ensemble des composés de l'invention qui sont purifiés, le cas échéant, selon une technique classique de purification, qui peuvent, si on le désire, être séparés en leurs différents isomères selon une technique classique de séparation et qui sont transformés, si on le souhaite, en leurs N-oxydes et, le cas échéant, en leurs sels d'addition à un acide ou à une base pharmaceutiquement acceptable.

Les composés de formules (II) et (IV) sont soit des produits commerciaux, soit obtenus selon des méthodes classiques de la synthèse organique, bien connues de l'homme de l'art.

Le composé de formule (IV) dans le cas spécifique où ----- représente une double liaison entre les atomes de carbone porteurs de substituants R_{c} et R_{d} respectivement de formule (IV/a) : 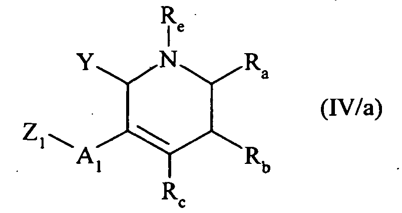 dans laquelle Rₐ, R_{b}, R_{c}, Rₑ, Y, A₁ et Z₁ sont tels que définis précédemment, peut notamment être obtenu à partir du composé de formule (V) : 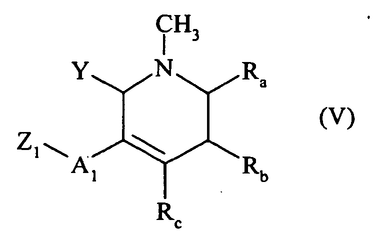 dans laquelle Rₐ, R_{b}, R_{c} Y, A₁ et Z₁ sont tels que définis précédemment, que l'on met en présence de carbonate de potassium et de chloroformiate de chlorométhyle,
pour conduire au composé de formule (VI) : 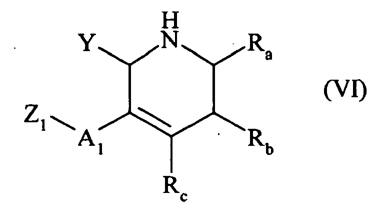 dans laquelle Rₐ, R_{b}, R_{c}, Y, A₁ et Z₁ sont tels que définis précédemment,
composé de formule (VI) qui est soumis à une réaction d'alkylation en présence d'un composé de formule (VII) :

Rₑ - Hal (VII)

dans laquelle Hal représente un atome d'halogène et Rₑ est tel que défini précédemment, pour conduire au composé de formule (IV/a) tel que défini précédemment.

Le composé de formule (IV) dans le cas spécifique où ----- représente une double liaison entre les atomes de carbone porteurs des substituants R_{d} et Y respectivement de formule (IV/b) : 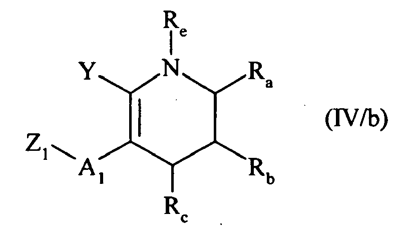 dans laquelle Rₐ, R_{b}, R_{c}, Rₑ, Y, A₁ et Z₁ sont tels que définis précédemment, peut notamment être obtenu à partir du composé de formule (VIII) : 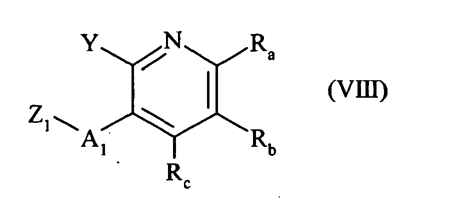 dans laquelle Rₐ, R_{b}, R_{c}, Y, A₁ et Z₁ sont tels que définis précédemment, qui est soumis à une réaction d'alkylation en présence d'un composé de formule (VII) tel que défini précédemment, pour conduire au composé de formule (IX) : 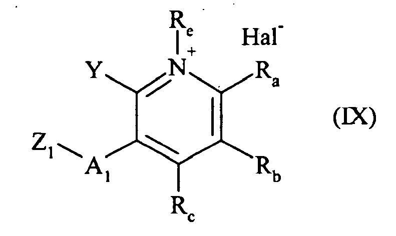 dans laquelle Rₐ, R_{b}, R_{c}, Rₑ, Y, A₁ et Z₁ sont tels que définis précédemment et Hal⁻ représente un anion halogènure,
composé de formule (IX) qui subit une réduction partielle en le faisant réagir avec de la triéthylamine et PtO₂,
pour conduire au composé de formule (IV/b) tel que défini précédemment.

Le composé de formule (IV) dans le cas spécifique où ----- représente une simple liaison de formule (IV/c) : 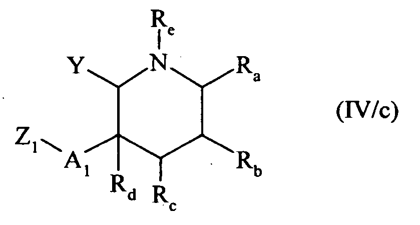 dans laquelle Rₐ, R_{b}, R_{c}, R_{d}, Rₑ, Y, A₁ et Y₁ sont tels que définis précédemment,
peut notamment être obtenu à partir de composé de formule (X) : 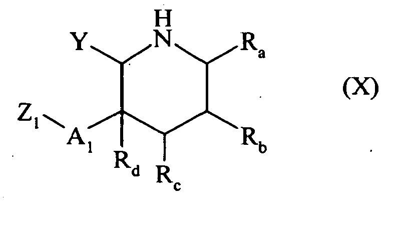 dans laquelle Rₐ, R_{b}, R_{c}, R_{d}, Y, A₁ et Z₁ sont tels que définis précédemment, qui est soumis à l'action d'un composé de formule (VII) tel que défini précédemment,
pour conduire au composé de formule (IV/c) tel que défini précédemment.

Les composés de formules (II), (IV), (V), (VII), (VIII) et (X) sont soit des produits commerciaux, soit obtenus selon des méthodes classiques de la synthèse organique bien connues de l'homme du métier.

Les composés de formule (I) possèdent d'intéressantes propriétés pharmacologiques et notamment celle d'être de puissants inducteurs de tyrosine hydroxylase (TH). On sait que la tyrosine hydroxylase est une enzyme limitante qui contrôle particulièrement la synthèse des neurotransmetteurs dans les neurones catécholaminergiques et dopaminergiques centraux. La vitesse de synthèse de ces neurotransmetteurs est notamment reliée à l'apparition de dysfonctions toniques du cerveau que sont de nombreuses pathologies comportementales chez l'homme, telles que l'anxiété, les psychoses, les dépressions, le stress, etc...

Grâce à leur capacité d'induction de tyrosine hydroxylase, les composés de l'invention trouvent donc leur utilisation thérapeutique dans le traitement des dépressions, de l'anxiété, des troubles de la mémoire au cours de la sénescence et/ou de maladies neurodégénératives, et dans le traitement palliatif de la maladie de Parkinson et pour l'adaptation au stress.

La présente invention a également pour objet les compositions pharmaceutiques renfermant comme principe actif au moins un composé de formule (I), ses énantiomères, diastéréoisomères, N-oxydes ou un de ses sels d'addition à une base ou un acide pharmaceutiquement acceptable, seul ou en combinaison avec un ou plusieurs excipients ou véhicules inertes, non toxiques pharmaceutiquement acceptables.

Les compositions pharmaceutiques ainsi obtenues se présentent généralement sous forme dosée. Elles peuvent par exemple revêtir la forme de comprimés, dragées, gélules, suppositoires, solutions injectables ou buvables et être administrées par voie orale, rectale, intramusculaire ou parentérale.

Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires ou ovules, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 0,1 mg à 100 mg en une ou plusieurs prises par jour.

Les exemples suivants illustrent l'invention mais ne la limitent en aucune façon.

Les produits de départ utilisés sont des produits connus ou préparés selon des modes opératoire connus. Les différentes préparations conduisent à des intermédiaires de synthèse utiles pour la préparation des composés de l'invention.

Les structures des composés décrits dans les exemples et dans les préparations ont été déterminées selon les techniques spectrométriques usuelles (infrarouge, résonance magnétique nucléaire, spectrométrie de masse, ...).

Les points de fusion ont été déterminés sur appareil TOTTOLI (sans correction de colonne émergente). Lorsque le composé existe sous forme de sel, le point de fusion correspond à celui du produit salifié.

### PRÉPARATION 1 : O-diphénylphosphinylhydroxylamine

À une solution aqueuse de 149,44 g de chlorhydrate d'hydroxylamine (340 ml d'eau) sont ajoutés une solution de 72,75 g de soude (290 ml d'eau) et 960 ml de 1,4-dioxane. Le mélange est refroidi à -15°C puis après 15 minutes, une solution de 180 g de chlorure de diphénylphosphinyle dans 725 ml de dioxane est ajoutée d'un trait sous agitation mécanique.
Après 5 minutes, 3 1 d'eau sont ajoutés d'un trait. Il se forme un précipité blanc qui est filtré puis repris dans une solution de soude 0,25 M à 0°C. Le mélange est agité mécaniquement à 0°C pendant 30 minutes avant d'être refiltré. Le précipité est séché sous vide (pentaoxyde de phosphore) pour donner 72,5 g du produit attendu.
*Point de fusion : 104°C*
*Microanalyses élémentaires :*

| | *C %* | *H %* | *N %* |
|---|---|---|---|
| *Calculé :* | *61,80* | *5,19* | *6,01* |
| *Trouvé :* | *61,20* | *5, 07* | *5,72* |

### PRÉPARATION 2 : N-aminoindole

À une suspension de 177,72 g de potasse pilée dans 1,3 1 de DMF sont ajoutés 21,85 g d'indole puis 72,5 g d'une suspension du composé de la préparation 1 dans 1,3 1 de DMF d'un trait. Le mélange épais est chauffé entre 60 et 70°C pendant 3h30 sous agitation mécanique puis coulé à chaud dans 3,5 1 d'eau glacée. La solution obtenue, après refroidissement, est extraite 3 fois par 1,5 1 d'éther éthylique. La phase organique est séchée sur sulfate de sodium, filtrée puis concentrée sous pression réduite. Une chromatographie sur gel de silice (cyclohexane/éther : 80/20 puis 50/50) permet d'isoler 16,5 g du produit attendu.
*Point de fusion : 35°C*
*Microanalyses élémentaires :*

| | *C %* | *H %* | *N %* |
|---|---|---|---|
| *Calculé :* | *72,70* | *6,10* | *21,20* |
| *Trouvé :* | *72, 68* | *6,14* | *21,17* |

### PRÉPARATION 3 : 5-Chloro-N-aminoindole

Le produit est obtenu selon le procédé de la préparation 2 en utilisant le 5-chloro-indole à la place de l'indole.
*Point de fusion : 46°C*
*Microanalyses élémentaires :*

| | *C%* | *H%* | *N%* |
|---|---|---|---|
| *Calculé :* | *57,61* | *4,23* | *16,81* |
| *Trouvé :* | *57,51* | *4,41* | *16, 68* |

### PRÉPARATION 4 : 3-Méthyl-N-aminoindole

Le produit est obtenu selon le procédé de la préparation 2 en utilisant le 3-méthyl-indole à la place de l'indole.

### PRÉPARATION 5 : 2,3-Diméthyl-N-aminoindole

Le produit est obtenu selon le procédé de la préparation 2 en utilisant le 2,3-diméthyl-indole à la place de l'indole.

### PRÉPARATION 6 : N-aminoindoline

### Stade A : 1-Nitrosoindoline

À une solution de 4 g d'indoline dans 100 ml d'acide acétique aqueux à 50% refroidie à 0°C est ajoutée goutte à goutte une solution de 2,32 g de NaNO₂ dans 50 ml d'eau, puis la solution est agitée 30 minutes à 0°C. La suspension est ensuite alcalinisée par l'ajout d'une solution de 190 ml de soude aqueuse à 20% puis extraite trois fois par de l'acétate d'éthyle. Les phases organiques réunies sont séchées sur sulfate de sodium, filtrées puis évaporées. 4,92 g du produit attendu sont obtenus.
*Point de fusion : 68°C*
*Microanalyses élémentaires :*

| | *C %* | *H %* | *N %* |
|---|---|---|---|
| *Calculé :* | *64,85* | *5,44* | *18,91* |
| *Trouvé :* | *64, 67* | *5,56* | *18, 69* |

### Stade B : N-aminoindoline

4 g du composé du stade A précédent sont dissous dans 50 ml d'un mélange éther diéthylique/dichlorométhane 85/15 puis versée goutte à goutte dans une solution de 1,4 g de LiAlH₄ 1M/Et₂O (36 ml) à 0°C. Après 30 minutes d'addition, le mélange réactionnel est agité 3 heures à température ambiante puis 1,5 ml d'eau, 4,5 ml de soude à 15% puis 1,5 ml d'eau sont ajoutés lentement. Les sels d'aluminium sont filtrés sur célite (élution au dichlorométhane) puis les solvants sont évaporés sous pression réduite. Une chromatographie sur gel de silice (cyclohexane/éther diéthylique : 7/3) permet d'isoler 2,9 g du produit attendu sous forme d'une huile orange.
*Infrarouge (*ν*_{cm-1}) :*
*3335 (νN-H); 3046; 3025 (*ν*=C-H); 2954; 2844 (*ν*C-H); 1605 (*ν*C=C); 1478 (*δ*C-H).*

### PRÉPARATION 7 : 1-Méthyl-1,4,5,6-tétrahydropyridine-3-carboxylate de méthyle

### Stade A : Iodure de 3-(méthoxycarbonyl)-1-méthylpyridinium

10 g de nicotinate de méthyle sont dissous dans 5,2 ml d'iodure de méthyle puis le mélange réactionnel est chauffé à 60°C pendant 24 heures à l'abri de la lumière. Le résidu est purifié par chromatographie éclair sur gel de silice ( CH₂Cl₂/CH₃OH : 9/1 puis 85/15) permettant d'isoler 13,46 g du produit attendu.

### Stade B : 1-Méthyl-1,4,5,6-tétrahydropyridine-3-carboxylate de méthyle

4 g du composé du stade A précédent dilué dans 100 ml de méthanol absolu sont hydrogénés à pression atmosphérique pendant 20 h à 20°C en présence de 4 ml de triéthylamine et de 800 mg de Pd/C à 10 %. Après filtration du catalyseur sur célite et lavage (deux fois avec du méthanol), le filtrat est évaporé sous vide et conduit à un résidu jaune. Celui-ci est lavé soigneusement trois fois avec de l'éther diéthylique distillé puis après évaporation sous vide du solvant, 2,15 g d'une huile jaune sont isolés.

### PRÉPARATION 8 : 2-Méthyl-1-indolinamine

Le produit est obtenu selon le procédé de la préparation 6 en utilisant la 2-méthylindoline à la place de l'indoline.

### PRÉPARATION 9 : 5-Fluoro-N-aminoindole

Le produit est obtenu selon le procédé de la préparation 2 en utilisant le 5-fluoro-indole à la place de l'indole.

### PRÉPARATION 10 : 5-Méthoxy-N-aminoindole

Le produit est obtenu selon le procédé de la préparation 2 en utilisant le 5-méthoxy-indole à la place de l'indole.

### PRÉPARATION 11 : 1-Allylpipéridine-3-carboxylate d'éthyle

2 g de pipéridine-3-carboxylate d'éthyle sont ajoutés à 1,6 g de bromure d'allyle puis sont additionnés 13 ml de benzène. 1,08 g de carbonate de sodium est ajouté et la réaction est agitée la nuit au reflux du benzène. Le mélange réactionnel est filtré puis évaporé sous pression réduite. Une purification par distillation dans le four à boules permet d'isoler 1,53 g du produit attendu.
*T_{éb}(8.10⁻² bar) : 80-85 °C*
*INFRAROUGE (ν_{cm-1}) :*
*3078 (*ν *=C-H) ; 2980 ; 2941 ; 2866 (*ν *C-H) ; 2791 (*ν *N-CH₂) ; 1733 (*ν *C=O) ; 1644 (*ν *C=C) ; 1468 ; 1453 (*δ*C-H) ; 1368 (*ν*C-N) ; 1223 ; 1182 (*ν*C-O).*

### PRÉPARATION 12 : 1-Méthylpipéridine-3-carboxylate d'éthyle

3 g de pipéridine-3-carboxylate d'éthyle, 4 ml de formaldéhyde aqueux, 300 mg de Pd/C à 10 % et 4 ml d'acide acétique glacial sont placés sous atmosphère d'hydrogène (1 atm) à 20°C pendant 17 heures. Après filtration du catalyseur sur célite et lavage avec 50 ml d'éthanol, la solution est évaporée sous pression réduite et conduit à un résidu huileux. Celui-ci est dilué dans un mélange toluène/eau (1/1) et le pH est ajusté à 9 par addition de 20 % de K₂CO₃. Après séparation des deux phases, la phase aqueuse est extraite deux fois avec du toluène. Les phases organiques sont lavées avec de l'eau, séchées sur Na₂SO₄ et conduisent à une huile légèrement colorée. La distillation sous pression réduite fournit 2,4 g du produit attendu.
*Point d'ébullition : 105-110°C (P = 20 mmHg)*

### PRÉPARATION 13 : 1-Propylpipéridine-3-carboxylate d'éthyle

2 g de pipéridine-3-carboxylate d'éthyle sont ajoutés à 1,6 g de 1-bromopropane puis sont additionnés 13 ml de benzène. 1,08 g de carbonate de sodium est ajouté et la réaction est agitée la nuit au reflux du benzène. Le mélange réactionnel est filtré puis évaporé sous pression réduite. Une purification par distillation dans le four à boules permet d'isoler 1,53 g du produit attendu.

### PRÉPARATION 14 : 1-(2-Hydroxyéthyl)-1,2,5,6-tétrahydropyridine-3-carboxylate de méthyle

### Stade A : 1,2,5,6-Tétrahydropyridine-3-carboxylate de méthyle

7 g de bromhydrate d'arécoline sont dissous dans 20 ml d'eau puis la solution est alcalinisée par l'ajout de 5,13 g de carbonate de potassium puis saturée en NaCl. La phase aqueuse est extraite trois fois par de l'éther. Les phases organiques réunies sont séchées sur sulfate de sodium, filtrées puis évaporées jusqu'à obtenir une masse de 4,7 g d'une huile incolore. Cette huile est diluée par 33 ml de toluène anhydre puis 3,92 ml de 1-chloroéthylchloroformiate sont ajoutés. Il se forme un précipité et le mélange est chauffé la nuit au reflux du toluène. Le précipité est filtré puis la phase organique est lavée par une solution d'acide chlorhydrique 0,1 M, la phase aqueuse est extraite une fois par de l'éther. Les phases organiques réunies sont séchées sur sulfate de sodium, filtrées puis évaporées sous pression réduite. Le résidu est repris par 25 ml de méthanol puis chauffé 2 heures au reflux. Le méthanol est évaporé sous pression réduite et 3,8 g de chlorhydrate de 1,2,5,6-tétrahydropyridine-3-carboxylate de méthyle sont obtenus. Le produit attendu est obtenu par dissolution du chlorhydrate de 1,2,5,6-tétrahydropyridine-3-carboxylate de méthyle dans l'eau puis alcalinisation par l'ajout de carbonate de potassium jusqu'à pH 10. On sature avec du chlorure de sodium puis la phase aqueuse est extraite trois fois par de l'éther et les phases organiques réunies sont séchées sur sulfate de sodium, filtrées puis évaporées jusqu'à obtention de 3 g du produit attendu.

### Stade B : 1-(2-Hydroxyéthyl)-1,2,5,6-tétrahydropyridine-3-èarboxylate de méthyle

À une solution de 1,77 g du composé du stade A précédent dans 17 ml de 1,4-dioxane anhydre sont ajoutés 5,22 g de carbonate de potassium, 24 mg d'iodure de sodium puis 900 µl de bromoéthanol. Le mélange réactionnel est agité la nuit au reflux puis concentré sous pression réduite. Le résidu est repris à l'eau, la phase aqueuse est extraite deux fois à l'acétate d'éthyle. Les phases organiques réunies sont séchées sur sulfate de sodium, filtrées puis évaporées, permettant d'isoler 1,75 g du produit attendu.
*Infrarouge (ν_{cm-1}) :*
*3398 (*ν *O-H); 2950; 2815 (*ν *C-H); 1710 (*ν *C=O); 1656 (*ν *C=C).*

### PRÉPARATION 15 : 1-(2-{[tert-Butyl(diméthyl)silyl]oxy}éthyl)-1,2,5,6-tétrahydro-pyridine-3-carboxylate de méthyle

À une solution de 1,5 g du composé de la préparation 14 dans 15 ml de pyridine sont ajoutés 2,08 g de chlorure de *tert*-butyldiméthylsilyle. La solution est agitée la nuit à température ambiante. Le solide obtenu est dissous dans du dichlorométhane puis lavé par une solution de carbonate de sodium à pH 11; la phase aqueuse est extraite par du dichlorométhane. Les phases organiques réunies sont séchées sur sulfate de sodium, filtrées puis évaporées sous pression réduite. Une chromatographie éclair sur gel de silice (cyclohexane/AcOEt : 3/1 puis 2/1) permet d'isoler 1,9 g du produit attendu.
*Infragrouge (ν_{cm-1})* :
*2951 ; 2929; 2856 (*ν *C-H); 1716 (*ν *C=O); 1658 (*ν *C=C).*

### PRÉPARATION 16 : 1-(2-Diméthylaminoéthyl)-1,2,5,6-tétrahydropyridine-3-carboxylate de méthyle.

À une solution de 1,94 g du composé du stade A de la préparation 14 dans 27 ml de 1,4-dioxane anhydre, 2,03 g d'iodure de sodium, 4,3 g de carbonate de sodium et 1,45 g de chlorure de 2-diméthylaminoéthyle sont ajoutés. La réaction est agitée la nuit au reflux du dioxane puis le mélange réactionnel est filtré et rincé avec du dichlorométhane. Le filtrat est évaporé sous pression réduite. Le résidu est repris par du dichlorométhane, lavé par une solution de carbonate de sodium à pH 10, séché sur Na₂SO₄, filtré puis évaporé sous pression réduite. Une purification dans le four à boules permet d'isoler 846 mg du produit attendu.
*Infrarouge (ν_{cm-1}) :*
*2948; 2816 (ν C-H); 2765 (ν N-CH₃); 1712 (v C=O); 1657 (ν C=C).*

### PRÉPARATION 17 : 1-(2-Hydroxyéthyl)-1,4,5,6-tétrahydropyridine-3-carboxylate de méthyle

### Stade A : Bromure de 1-(2-hydroxyéthyl)-3-(méthoxycarbonyl)-pyridinium

10 g de nicotinate de méthyle sont dissous dans 5,2 ml de 2-bromoéthanol puis le mélange réactionnel est chauffé à 60°C pendant 24 heures à l'abri de la lumière. Le résidu est purifié par chromatographie éclair sur gel de silice ( CH₂Cl₂/CH₃OH : 9/1 puis 85/15) permettant d'isoler 13,46 g du produit attendu.
*Point de fusion : 90°C*
*Microanalyses élémentaires :*

| | *C %* | *H %* | *N %* |
|---|---|---|---|
| *Calculé :* | *41,24* | *4,61* | *5,34* |
| *Calculé + H₂O :* | *38,59* | *5, 04* | *5, 00* |
| *Trouvé :* | *37,99* | *5,01* | *4,83* |

### Stade B : 1-(2-Hydroxyéthyl)-1,4,5,6-tétrahydropyridine-3-carboxylate de méthyle

À une solution de 4 g du composé du stade A précédent dans 100 ml de MeOH rectifié sont ajoutés 2,73 ml de triéthylamine et 400 mg de Pd/C à 10%. Le milieu est dégazé sous vide deux fois et mis sous hydrogène. Le mélange réactionnel est agité 18 heures puis filtré sur célite (élution par CH₂Cl₂), La solution est évaporée sous pression réduite. Le résidu est repris par 100 ml d'éther diéthylique et de l'eau; la phase aqueuse est extraite deux fois à l'éther et 2,41 g du produit attendu sont obtenus.
*Point de fusion : 38°C*
*Microanalyses élémentaires :*

| | *C %* | *H %* | *N %* |
|---|---|---|---|
| *Calculé :* | *58,36* | *8,16* | *7,56* |
| *Trouvé :* | *57,65* | *8,41* | *7,38* |

### PRÉPARATION 18 : 1-[(2-Diméthylamino)éthyl]-1,4,5,6-tétrahydropyridine-3-carboxylate de méthyle.

À une solution de 2 g du composé de la préparation 17 dans 20 ml de dichlorométhane anhydre sont ajoutés 2 ml de triéthylamine puis 1,1 ml de chlorure de mésyle à 0°C. Le mélange réactionnel est agité 30 minutes à température ambiante puis 20 ml d'eau sont ajoutés, la phase organique est diluée par du dichlorométhane. La phase aqueuse est extraite deux fois par du dichlorométhane. Les phases organiques réunies sont séchées sur Na₂SO₄, filtrées puis évaporées sous pression réduite. Le résidu est repris dans 20 ml d'acétonitrile puis 12,5 ml de diméthylamine 2M/MeOH sont ajoutés. La réaction est agitée 15 heures à température ambiante puis 2 heures à 50°C. Le mélange réactionnel est concentré sous pression réduite, repris par du dichlorométhane, lavé par une solution de K₂CO₃ pH 11 puis par une solution saturée en NaCl. La phase organique est séchée sur Na₂SO₄, filtrée puis évaporée sous pression réduite permettant d'isoler 1,42 g du produit attendu.
*Infrarouge (*ν*_{cm-1}) :*
*2961; 2853 (*ν *C-H); 2771 (*ν *N-CH₃); 1678 (*ν *C=O); 1617 (*ν *C=C)*.

### PRÉPARATION 19 : 1-[2-(Diméthylamino)éthyl]-pipéridine-3-carboxylate d'éthyle

3,51 g de chlorure de 2-diméthylaminoéthyle sont dissous dans 50 mL de 1,4-dioxane anhydre puis 9,58 g de carbonate de sodium, 4,52 g de d'iodure de sodium, suivi de 5,16 g de pipéridine-3-carboxylate d'éthyle sont ajoutés. Le mélange réactionnel est porté au reflux 20 heures puis le dioxane est évaporé sous pression réduite. Le résidu est repris par de l'acétate d'éthyle, lavé à l'eau puis par une solution saturée de chlorure de sodium. La phase organique est séchée sur sulfate de sodium puis évaporée sous pression réduite. Une purification par distillation dans le four à boules permet d'isoler 3,41 g du produit attendu.
*Température d'ébullition = 90°C (5.10⁻¹ bars)*
*Infrarouge (*ν*_{cm-1}) :*
*2941; 2857; 2815 (*ν *C-H); 2766 (*ν *N-CH₃); 1730 (*ν *C=O); 1464 (*δ *C-H)*.

### PRÉPARATION 20 : 1-[2-(4-Morpholinyl)éthyl]-pipéridine-3-carboxylate d'éthyle

2,0 g du composé de la préparation 36 et 2,3 ml de morpholine sont solubilisés dans 15 ml d'éthanol à 70%. Le milieu réactionnel est placé sous agitation à température ambiante pendant 96 heures. La solution est ensuite concentrée puis reprise par 30 ml de dichlorométhane. La phase organique est ensuite lavée plusieurs fois avec de l'eau, séchée sur Na₂SO₄, filtrée puis concentrée. Une chromatographie sur gel de silice (CH₂Cl₂/MeOH : 9/1) permet d'isoler 2,3 g du produit attendu.
*Infrarouge (*ν*_{cm-1}) :*
*2943, 2854, 2810 (*ν*C-H), 1729 (*ν*C=O ester), 1449 (*δ*C-H), 1372 (*ν*C-N), 1117 (*ν*C-O-C).*

### PRÉPARATION 21 : 1-[2-(1-Pipéridinyl)éthyl]-pipéridine-3-carboxylate d'éthyle

Le produit est obtenu selon le procédé de la préparation 20 en utilisant la pipéridine à la place de la morpholine.
*Infrarouge (*ν*ₘ₋₁) :*
*2939, 2859 (*ν*C-H), 1726 (*ν*C=O ester), 1453 (*δ*C-H), 1372 (*ν*C-N), 1183, 1154 (*ν*C-O).*

### PRÉPARATION 22 : 1-[2-(4-Méthyl-1-pipérazinyl)éthyl]-pipéridine-3-carboxylate d'éthyle

Le produit est obtenu selon le procédé de la préparation 20 en utilisant la N-méthylpipérazine à la place de la morpholine.
*Infrarouge (*ν_{c}*ₘ₋₁) :*
*2939, 2799 (*ν*C-H), 1731 (*ν*C=O ester), 1452 (*δ*C-H), 1373 (*ν*C-N).*

### PRÉPARATION 23 : 1-{2-[4-(2-Hydroxyéthyl)-1-pipérazinyl]éthyl}-pipéridine-3-carboxylate d'éthyle

Le produit est obtenu selon le procédé de la préparation 20 en utilisant la (2-hydroxy-éthyl)pipérazine à la place de la morpholine.
*Infrarouge (*ν*_{cm-1}) :*
*3235 (*ν*O-H), 2940, 2810 (*ν*C-H), 1730 (*ν*C=O ester), 1451 (*δ*C-H), 1371 (*ν*C-N), 1222 (*ν*C-O).*

### PRÉPARATION 24 : 1-[2-(1-Pyrrolidinyl)éthy]-pipéridine-3-carboxylate d'éthyle

Le produit est obtenu selon le procédé de la préparation 20 en utilisant la pyrrolidine à la place de la morpholine.
*Infrarouge (*ν*_{cm-1}) :*
*2942, 2782 (*ν*C-H), 1732 (*ν*C=O ester), 1452 (*δ*C-H), 1371 (*ν*C-N), 1226 (*ν*C-O)*.

### PRÉPARATION 25 : 1-[2-(1-Azépanyl)éthyl]-pipéridine-3-carboxylate d'éthyle

Le produit est obtenu selon le procédé de la préparation 20 en utilisant l'hexaméthyleneimine à la place de la morpholine.
*Infrarouge (*ν*_{cm-1}) :*
*2925, 2853, 2810 (*ν*C-H), 1733 (*ν*C=O ester), 1450 (*δ*c-H), 1370 (*ν*C-N), 1225 (*ν*C-O).*

### PRÉPARATION 26 : 1-[2-(4-Phényl-1-pipérazinyl)éthyl]-pipéridine-3-carboxylate d'éthyle

Le produit est obtenu selon le procédé de la préparation 20 en utilisant la 4-phényl-pipérazine à la place de la morpholine.
*Infrarouge (*ν*_{cm-1}) :*
*2942, 2817 (*ν*C-H), 1731 (*ν*C=O ester), 1600 ((*ν *C=C), 1502, 1451 (*ν*C-H).*

### PRÉPARATION 27 : 1-[2-(1-Pipéridinyl)éthyl]-pipéridine-3-carbaldéhyde

0,98 g du composé de la préparation 18 sont dissous dans 30 ml de THF anhydre puis la solution est refroidie à -80 °C. 2,5 ml d'une solution de DIBA1-H (1,5M dans le toluène) sont ensuite ajoutés lentement à la seringue. La solution est ensuite maintenue sous agitation à -80°C puis après 1,5 heure, 2,5ml supplémentaires de la solution DIBA1-H sont ajoutés dans le milieu. Après 3h de réaction à -80°C, 15 ml d'eau sont additionnés puis la température est laissée remonter jusqu'à température ambiante. La solution est ensuite extraite avec 2 fois 50 ml de CH₂Cl₂ puis lavée avec une solution saturée de NaCl. Après séchage sur Na₂SO₄ et concentration, le produit attendu est obtenu et est utilisée sans autre purification pour l'étape suivante.

### PRÉPARATION 28 : 5-Chloro-N-aminoindoline

### Stade A : 1-Acétylindoline

À 5 g d'indoline sont ajoutés 23 ml d'anhydride acétique, goutte à goutte, en maintenant la température du milieu à 0°. Le mélange est agité 4 heures au reflux puis, est évaporé sous pression réduite. Une chromatographie éclair sur gel de silice (cyclohexane/AcOEt : 6/4 puis AcOEt pur) permet d'isoler 6,4 g du produit attendu

### Stade B : 5-Chloroindoline

À une solution de 6,4 g du composé du stade A précédent dans 170 ml de tétrachlorure de carbone sont ajoutés à 0°C goutte à goutte 3,84 g de SO₂Cl₂. Il se forme un précipité blanc qui est agité à température ambiante pendant une heure. La suspension est diluée par du dichlorométhane et par de l'eau. La phase organique est extraite puis lavée par une solution de soude aqueuse à 20%. La phase organique est séchée sur sulfate de sodium, filtrée puis évaporée sous pression réduite. Le résidu est repris par 110 ml d'éthanol absolu et chauffé au reflux jusqu'à dissolution complète. 49 ml d'HCl à 37% sont ajoutés puis la solution est agitée au reflux pendant 3h30. L'éthanol est évaporé sous pression réduite puis la phase aqueuse est diluée par 50 ml d'eau, extraite à l'éther puis alcalinisée par une solution de soude aqueuse à 20%. La phase aqueuse est extraite trois fois par l'éther. Les phases organiques réunies sont séchées sur sulfate de sodium, filtrées puis évaporées sous pression réduite. Une chromatographie éclair sur gel de silice (cyclohexane/AcOEt : 7/3) permet d'isoler 3,53 g du produit attendu.

### Stade C: 5-Chloro-1-nitrosoindoline

À une solution de 3,53 g du composé du stade B précédent dans 70 ml d'acide acétique aqueux à 50% refroidie à 0°C, on ajoute goutte à goutte une solution de 1,59 g de NaNO₂ dans 35 ml d'eau; puis la solution est agitée 30 minutes à 0°C. Le milieu est ensuite alcalinisé par l'ajout de 130 ml d'une solution de soude aqueuse à 20% puis extrait trois fois par de l'acétate d'éthyle. Les phases organiques réunies sont séchées sur sulfate de sodium, filtrées puis évaporées sous pression réduite permettant d'isoler 4,15 g du produit attendu.
*Point de fusion : 89°C*
*Microanalyses élémentaires :*

| | *C %* | *H %* | *N %* |
|---|---|---|---|
| *Calculé :* | *52,62* | *3,86* | *15,34* |
| *Trouvé :* | *53,86* | *4,41* | *15,45* |

### Stade D : 5-Chloro-N-aminoindoline

Une suspension de 570 mg de LiAlH₄ dans 15 ml d'éther anhydre est agitée au reflux de l'éther 45 minutes, puis agitée la nuit à température ambiante. 2 g du composé du stade C précédent sont dissous dans 6 ml de THF anhydre puis on dilue par 14 ml d'éther anhydre. La solution ainsi obtenue est ajoutée goutte à goutte dans la solution de LiAlH₄ à 0°C. La solution est agitée trois heures à 0°C puis 0,5 ml d'eau, 1,5 ml de soude aqueuse à 15% et 0,5 ml d'eau sont'doucement ajoutés sous azote. Les sels d'aluminium sont filtrés sur célite (élution par le dichlorométhane). La solution est évaporée sous pression réduite. Une chromatographie éclair sur gel de silice (cyclohexane/AcOEt : 85/15 puis 8/2 à 7/3) permet d'isoler 1,7 g du produit attendu.
*Point de fusion : 30°C*
*Microanalyses élémentaires :*

| | *C %* | *H %* | *N %* |
|---|---|---|---|
| *Calculé :* | *56,98* | *5,38* | *16,61* |
| *Trouvé :* | *58,13* | *5,61* | *16,44* |

### PRÉPARATION 29 : 1-(2-{[tert-Butyl(diméthyl)silyl]oxy}éthyl)-1,4,5,6-tétrahydro-3-pyridine carboxylate de méthyle

Le produit est obtenu selon le procédé de la préparation 15 en utilisant le composé de la préparation 17 à la place du composé de la préparation 14.

### PRÉPARATION 30 : 1-(2-Hydroxyéthyl)-pipéridine-3-carboxylate d'éthyle

2 g de pipéridine-3-carboxylate d'éthyle sont ajoutés à 1,6 g de 2-bromoéthanol puis on dilue par 13 ml de benzène. 1,08 g de carbonate de sodium sont ajoutés et la réaction est agitée la nuit au reflux du benzène. Le mélange réactionnel est filtré puis évaporé sous pression réduite. Une purification par distillation dans le four à boules permet d'isoler 1,53 g du produit attendu.
*Température d'ébullition : 100°C (3.10⁻² bars)*
*INFRAROUGE (*ν_{c}*ₘ₋₁) :*
*3412 (*ν *O-H); 2941; 2808 (*ν *C-H); 1730 (*ν *C=D); 1468 (*δ *C-H).*

### PRÉPARATION 31 : 1-Benzylpipéridine-3-carboxylate d'éthyle

Le produit est obtenu selon le procédé de la préparation 30 en utilisant le bromure de benzyle à la place du 2-bromoéthanol.

### PRÉPARATION 32 : 4-(4-Fluorophényle)-1-méthylpipéridine-3-carboxylate de méthyle

2,5 g d'arécoline sont dissous dans 20 ml d'éther diéthylique anhydre et 25 ml de dichlorométhane anhydre. La solution est refroidie à -30°C et 32,2 ml d'une solution 1 M de bromure de (4-fluorophényl)magnésium sont ajoutés goutte à goutte. Le mélange est agité 3 heures à une température de -30°C à -35°C puis celui-ci est refroidi à -78°C pendant 30 minutes. 10 ml de mélange 1/1 d'acide trifluoroacétique et d'éther sont ensuite ajoutés goutte à goutte puis le mélange est placé à 0°C et 15 ml d'une solution d'acide chlorhydrique 1M sont ajoutés suivi d'une solution à 28 % d'ammoniaque aqueux jusqu'à pH 12. La phase organique est récupérée, la phase aqueuse est extraite trois fois par de l'acétate d'éthyle. Les phases organiques réunies sont séchées sur sulfate de sodium, filtrées puis évaporées sous pression réduite. Une chromatographie éclair sur gel de silice (cyclohexane/triéthylamine : 3/1) permet d'isoler le produit attendu.

### PRÉPARATION 33: 4-{-[3-(Éthoxycarbonyl)-1-pipéridinyl]éthyl}-pipérazine-1-carboxylate de tert-butyle

Le produit attendu est obtenu selon le procédé de la préparation 20 en utilisant la pipérazine-1-carboxylate de *tert*-butyle à la place de la morpholine.
*Infrarouge (*ν*_{cm-1}) :*
*2938, 2811 (*ν *C-H); 1731 (vC=O ester); 1698 (*ν *C=ONBOc) ; 1455, 1421, 1373 (*ν*C-N)*

### PRÉPARATION 34 : 1-[3-(Diméthylamino)propyl]pipéridine-3-carboxylate d'éthyle

1,5 g du composé de la préparation 24 et 4,8 ml de diméthylamine sont solubilisés dans 10 ml d'éthanol à 70 %. Le milieu réactionnel est placé sous agitation à température ambiante pendant 48 heures. 4,8 ml de diméthylamine supplémentaires sont ajoutés puis la réaction est portée à 50°C pendant 24 heures. La solution est ensuite concentrée dans un évaporateur rotatif puis reprise par 50 ml de dichlorométhane. La phase organique est ensuite lavée plusieurs fois à l'eau, séchée sur Na₂SO₄, filtrée puis concentrée. Une chromatographie sur gel de silice (CH₂Cl₂/MeOH : 9/1) permet d'isoler 0,81 g du produit attendu.
*Infrarouge (*ν*_{cm-1}) :*
*2942, 2813, 2761 (*ν *C-H); 1730 (*ν*C=O ester); 1466 (*δ *C=H) ; 1373 (*ν *C-N) ; 1235, 1179 (*ν*C=O), 1104*

### PRÉPARATION 35 : 1-(3-Hydroxypropyl)-pipéridine-3-carboxylate d'éthyle

Le produit est obtenu selon le procédé de la préparation 30 en utilisant le 3-bromopropanol à la place du 2-bromoéthanol.
*Température d'ébullition : 125°C (3.10⁻² bars)*
*Infrarouge (*ν*_{cm-1})* :
*3388 (*ν *O-H); 2941; 2861; 2811 (*ν *C-H) 1729 (*ν *C=O); 1470 (*δ *C-H).*

### PRÉPARATION 36 : 1-(2-Chloroéthyl)-pipéridine-3-carboxylate d'éthyle

10,0 g de 3-pipéridine-3-carboxylate d'éthyle sont dissous dans 80 ml d'acétone puis 11 ml de 1-bromo-2-chloroéthane et 14 g de carbonate de potassium sont ajoutés à température ambiante. Après 40 heures sous agitation, le solvant est évaporé puis 50 ml d'eau ainsi que 100 ml d'éther diéthylique sont ajoutés. La phase organique est séparée, lavée avec de l'eau, séchée sur Na₂SO₄ puis concentrée. Une chromatographie sur gel se silice (éther de pétrole/éther éthylique : 6/4) permet d'isoler 9.24 g du produit attendu.
*Infrarouge (*ν*_{cm-1}) :*
*2943, 2808 (*ν*C-H), 1729 (*ν*C=O ester), 1468, 1450 (*δ*C-H), 1370 (*ν*C-N), 1209, 1179 (νC-O).*

### PRÉPARATION 37 : 1-(3-Chloropropyl)-pipéridine-3-carboxylate d'éthyle

Le produit est obtenu selon le procédé de la préparation 36 en utilisant le 1-bromo-3-chloropropane à la place du 1-bromo-2-chloropropane.
*Infrarouge (*ν_{c}*ₘ₋₁) :*
*2945, 2808 (*ν*C*-*H), 1730 (*ν*C*=*O ester), 1469, 1446 ((*ν*C-H), 1371 (*ν*C-N), 1179, 1153* (ν*C-O*).

### PRÉPARATION 38 : 1-[3-(1-Pipéridinyl)propyl]-pipéridine-3-carboxylate d'éthyle

Le produit est obtenu selon le procédé de la préparation 20 en utilisant la pipéridine à la place de la morpholine et le composé de la préparation 37 à la place du composé de la préparation 36.
*Infrarouge (*ν*_{cm-1}) :*
*2933, 2854, 2802, 2761 (*ν*C-H), 1731 (*ν*C=O ester), 1469, 1463 (*δ*C-H), 1373 (*ν*C-N), 1271, 1178 (*ν*C-O).*

### PRÉPARATION 39 : 1-[3-(4-Méthyl-1-pipérazinyl)propyl]-pipéridine-3-carboxylate d'éthyle

Le produit est obtenu selon le procédé de la préparation 20 en utilisant la N-méthylpipérazine à la place de la morpholine et le composé de la préparation 37 à la place du composé de la préparation 36.
*Infrarouge (*ν*_{cm-1}) :*
*2939, 2794 (*ν*C-H), 1730 (*ν*C=O ester), 1448 (*δ*C-H), 1372 (*ν*C-N), 1283, 1150 (*ν*C-O)*.

### PRÉPARATION 40 : 1-(2-{[tert-Butyl(diméthyl)silyl]oxy}éthyl)-pipéridine-3-carboxylate d'éthyle

À une solution de 1 g du composé de la préparation 30 dans 16 ml de pyridine est ajouté 1,276 g de chlorure de terbutyldiméthylsilyl. La réaction est agitée la nuit à température ambiante puis la pyridine est évaporée sous pression réduite (coévaporation avec le toluène). Le résidu est repris par du dichlorométhane, lavé deux fois à l'eau puis par une solution saturée en NaCl. La phase organique est séchée sur sulfate de sodium, filtrée puis évaporée. Une chromatographie éclair sur gel de silice (cyclohexane/AcOEt : 3/1) permet d'isoler 1,1 g du produit attendu.
*Infrarouge (*ν*_{cm-1}) :*
*2930; 2857 (*ν*C-H); 1733 (*ν*C=O); 1470 (*δ*C-H).*

### PRÉPARATION 41 : 1-(3-{[tert-Butyl(diméthyl)silyl]oxy}propyl)-pipéridine-3-carboxylate d'éthyle

Le produit est obtenu selon le procédé de la préparation 40 en utilisant le composé de la préparation 35 à la place du composé de la préparation 30.
*Infrarouge (*ν*_{cm-1}) :*
*2951; 2930; 2857 (*ν*C-H); 1734 (*ν*C=O); 1471 (*δ*C-H).*

### PRÉPARATION 42 : 1-(2-Chloroéthyl)-1,2,5,6-tétrahydropyridine-3-carboxylate de méthyle.

### Stade A : Chlorhydrate de guvacine

7 g de bromhydrate d'arécoline sont dissous dans 20 ml d'eau ; la solution est alcalinisée par l'ajout de 5,13 g de carbonate de potassium, puis saturée en NaCl. La phase aqueuse est extraite trois fois à l'éther diéthylique. Les phases organiques réunies sont séchées sur sulfate de sodium, filtrées, puis évaporées jusqu'à l'obtention d'une masse de 4,7 g d'huile incolore. Cette huile est reprise par 20 ml de toluène ; la solution se trouble. Après addition de sulfate de sodium et filtration, l'insoluble est lavé par 13 ml de toluène. À la solution organique, sont ajoutés 3,92 ml de chloroformiate de 1-chloroéthyle. Il se forme un précipité et le milieu réactionnel est chauffé 12 heures au reflux du toluène. Le précipité est éliminé par filtration, puis la phase organique est lavée par une solution aqueuse d'acide chlorhydrique 0,1M ; la phase aqueuse est extraite une fois à l'éther diéthylique. Les phases organiques réunies sont séchées sur sulfate de sodium, filtrées, puis évaporées sous pression réduite. Le résidu est repris par 25 ml de méthanol et chauffé 2 heures au reflux. Le méthanol est éliminé par évaporation sous pression réduite et 3,8 g du produit attendu sont obtenus avec un rendement de 73 %. La guvacine base est obtenue par dissolution du chlorhydrate dans l'eau ; la phase aqueuse est alcalinisée par l'ajout de carbonate de potassium jusqu'à pH 10 et saturée en NaCl. La phase aqueuse est extraite trois fois par l'éther diéthylique et les phases organiques réunies sont séchées sur sulfate de sodium, filtrées, puis évaporées jusqu'à obtention de 3 g d'une huile incolore.
*Microanalyses élémentaires :*

| | *C %* | *H %* | *N %* |
|---|---|---|---|
| *Calculé :* | *47,33* | *6,81* | *7,89* |
| *Trouvé :* | *47,38* | *6,93* | *7,83* |

### Stade B : 1-(2-Chloroéthyl)-1,2,5,6-tétrahydropyridine-3-carboxylate de méthyle.

À une suspension de 530 mg du composé du stade A précédent dans 12 ml d'acétone, sont ajoutés 2,53 ml de triéthylamine et 1,1 ml de 1-bromo-2-chloroéthane. Le mélange est agité à température ambiante 18 heures, puis chauffé au reflux 8 heures. Il est évaporé sous pression réduite, repris par du dichlorométhane et lavé par une solution aqueuse de carbonate de potassium. La phase aqueuse est extraite par du dichlorométhane. Les phases organiques réunies sont séchées sur sulfate de sodium, filtrées, puis évaporées sous pression réduite. Le résidu est purifié par chromatographie éclair sur gel de silice (cyclohexane/AcOEt : 7/3) permettant d'obtenir 430 mg du composé attendu.
*Microanalyses élémentaires :*

| | *C %* | *H %* | *N %* |
|---|---|---|---|
| *Calculé :* | *53,08* | *6,93* | *6,88* |
| *Trouvé :* | *53,74* | *7,22* | *6,72* |

*INFRAROUGE (*ν*_{cm-1}) :*
*2951 ; 2917 ; 2811 (*ν *C-H) ; 2767 (v N-CH₂) ; 1709 (*ν *C=O) ; 1657 (*ν *C=C) ; 1462 ; 1436 (*δ *C-H) ; 1375 (*ν *C-N) ; 1261 (*ν *C-O).*

### PRÉPARATION 43 : 1-[2-(4-Méthylpipérazin-1-yl)éthyl]-1,2,5,6-tétrahydroxyridine-3-carboxylate de méthyle.

À une solution de 320 mg du composé de la préparation 42 dans 5 ml d'éthanol aqueux à 70 %, sont ajoutés 540 µl de 1-méthylpipérazine. La solution est agitée 72 heures à température ambiante, puis évaporée sous pression réduite. Le résidu est repris par du dichlorométhane et lavé deux fois par une solution aqueuse de carbonate de sodium. La phase organique est séchée sur sulfate de sodium, filtrée et évaporée sous pression réduite pour obtenir 320 mg du composé attendu.
*INFRAROUGE (*ν*_{cm-1}) :*
*2938 (*ν *C-H) ; 2793 (*ν *N-CH₃) ; 1712 (*ν *C=O) ; 1657 (*ν *C=C) ; 1438 (*δ *C-H) ; 1373 (v C-N) ; 1262 (*ν *C-O).*

### PRÉPARATION 44: 5-Méthyl-N-aminoindole

Le produit est obtenu selon le procédé de la préparation 2 en utilisant la 5-méthyl-indole à la place de l'indole.

### PRÉPARATION 45 : 1-[2-[4-[2-(tert-Butyldiméthylsilanoxy)éthyl]pipérazin-1-yl] éthyl]-1,2,5,6-tétrahydropyridine-3-carboxylate de méthyle

### StadeA : 1-[2-[4-(2-Hydroxyéthyl)pipérazin-1-yl]éthyl]-1,2,5,6-tétrahydropyridine-3-carboxylate de méthyle.

Le produit est obtenu selon le procédé de la préparation 43 en utilisant la 4-(2-hydroxyéthyl)pipérazine à la place de la 1-méthylpipérazine.
*INFRAROUGE (*ν*_{cm-1}) :*
*3350 (*ν *O-H) ; 2946 (*ν *C-H) ; 2812 (*ν *N-CH₂) ; 1710 (v C=O) ; 1656 (*ν *C=C) ; 1437 (*δ *C-H) ; 1351 (*ν *C-N) ; 1262 (*ν *C-O).*

### Stade B : 1-[2-[4-[2-(tert-Butyldiméthylsilanoxy)éthyl]pipérazin-1-yl]éthyl]-1,2,5,6-tétrahydropyridine-3-carboxylate de méthyle

À une solution de 340 mg du composé du stade A précédent dans 5 ml de pyridine, sont ajoutés 260 mg de chlorure de tributyldiméthylsilyle. La solution est agitée 16 heures à température ambiante, puis la pyridine évaporée sous pression réduite. Après dissolution du résidu dans le dichlorométhane, la phase organique est extraite par une solution aqueuse de carbonate de potassium. La phase aqueuse est extraite par du dichlorométhane. Les phases organiques réunies sont séchées sur sulfate de sodium, filtrées et évaporées sous pression réduite. Une chromatographie éclair sur gel de silice (CH₂Cl₂/CH₃OH : 9/1 puis 85/15) permet d'obtenir 360 mg du produit attendu.

### PRÉPARATION 46 : 1-[2-(Pipéridin-1-yl)-éthyl]-1,2,5,6-tétrahydropyridine-3-carboxylate de méthyle

Le produit est obtenu selon le procédé de la préparation 43 en utilisant la pipéridine à la place de la 1-méthylpipérazine.
*INFRAROUGE (*ν*_{cm-1}) :*
*2955 ; 2932 ; 2872 (*ν *C-H) ; 1736 (*ν *C=O) ; 1661 (*ν *C=C) ; 1434 (*δ *C-H) ; 1368 ; 1341 (*ν *C-N) ; 1236 ; 1194 (*ν *C-O).*

### PRÉPARATION 47 : 1,2-Bis[3-(éthoxycarbonyl)-1,2,5,6-tétrahydropyridin-1-yl] éthane

À une solution de 900 mg du composé du stade A de la préparation 42 dans 10 ml de méthanol, sont ajoutés 0,32 ml de 2-bromochloroéthane, puis 1,6 ml de triéthylamine. Le mélange est chauffé au reflux 20 heures et évaporé sous pression réduite. Le résidu est dissous dans le dichlorométhane et extrait par une solution aqueuse saturée de carbonate de potassium. La phase aqueuse est extraite par le dichlorométhane. Les phases organiques réunies sont séchées sur sulfate de sodium, filtrées et évaporées sous pression réduite. Une chromatographie éclair sur gel de silice (AcOEt, puis AcOEt/MeOH : 95/5 à 90/10) permet d'obtenir 500 mg du produit attendu.
*Point de fusion : 77°C*
*Microanalyses élémentaires :*

| | *C %* | *H %* | *N %* |
|---|---|---|---|
| *Calculé :* | *61,13* | *7,91* | *8,91* |
| *Trouvé :* | *61,17* | *7,76* | *8,80* |

*INFRAROUGE (*ν*_{cm-1}) :*
*2950 ; 2908 (v C-H) ; 2807 (*ν *N-CH₂) ; 1708 (*ν *C=O) ; 1656 (*ν *C=C) ; 1435 (*δ *C-H) ; 1351 (*ν *C-N) ; 1258 (*ν *C-O).*

### PRÉPARATION 48 : (±)-1-[2-[4-[(Tétrahydrofuran-2-yl)méthyl]pipérazin-1-yl)] éthyl]pipéridine-3-carboxylate d'éthyle

Le produit est obtenu selon le procédé de la préparation 20 en utilisant la 4-[(tétrahydrofuran-2-yl)méthyl]pipéridine à la place de la morpholine.

Une chromatographie sur gel de silice (CH₂Cl₂/CH₃OH : 95/5) permet d'isoler 1,2 g du produit attendu.
*INFRAROUGE (*ν*_{cm-1}) :*
*2939 ; 2871 ; 2810 (*ν*C-H) ; 1730 (*ν*C=O ester) ; 1451 (*δ*C-H) ; 1371 (*ν*C-N) ; 1300 ; 1154 (*ν*C-O).*

### PRÉPARATION 49 : 1-[2-[4-[2-(Diméthylamino)éthyl]pipérazin-1-yl)]éthyl] pipéridine-3-carboxylate d'éthyle

Le produit est obtenu selon le procédé de la préparation 20 en utilisant la N-[2-(diméthylamino)éthyl]pipéridine à la place de la morpholine.
*INFRAROUGE (*ν*_{cm-1}) :*
*2941 ; 2810 (*ν*C-H) ; 1730 (*ν*C=O ester) ; 1466 (*δ*C-H) ; 1304 ; 1154 (*ν*C-O).*

### PRÉPARATION 50 : (±)-1-[2-(4-(2-Méthoxyéthyl)pipérazin-1-yl)éthyl]pipéridine-3-carboxylate de méthyle

Le produit est obtenu selon le procédé de la préparation 20 en utilisant la (2-méthoxyéthyl)pipéridine à la place de la morpholine.
*INFRAROUGE (*ν*_{cm-1}) :*
*2941 ; 2809 (*ν*C-H) ; 1734 (*ν*C=O ester) ; 1451 (*δ*C-H) ; 1305 (*ν*C-N) ; 1155 (*ν*C-O) ; 1014.*

### PRÉPARATION 51 : 1-[2-[4-(1-Méthylpipéridin-4-yl)pipérazin-1-yl]éthyl]pipéridine-3-carboxylate d'éthyle

Le produit est obtenu selon le procédé de la préparation 20 en utilisant la (1-méthylpipéridin-4-yl)pipérazine à la place de la morpholine.
*INFRAROUGE (*ν*_{cm-1}) :*
*2936 ; 2807 (*ν*C-H) ; 1731 (*ν*C=O ester) ; 1449 (*δ*C-H) ; 1375 (*ν*C-N) ; 1152 (*ν*C-O).*

### PRÉPARATION 52 : (±)-1-[3-[4-[2-(tert-Butyldiméthylsilyloxy)éthyl]pipérazin-1-yl] propyl]pipéridine-3-carboxylate d'éthyle

### Stade A : (±)-1-[3-[4-(2-Hydroxyéthyl)pipérazin-1-yl]propyl]pipéridine-3-carboxylate d'éthyle

Le produit est obtenu selon le procédé de la préparation 20 en utilisant la (2-hydroxyéthyl)pipérazine à la place de la morpholine.
*INFRAROUGE (*ν*_{cm-1}) :*
*2940 ; 2810 (*ν*C-H) ; 1731 (*ν*C=O ester) ; 1573 ; 1372 ; 1154 (*ν*C-O).*

### Stade B : (±)-1-[3-[4-[2-(tert-Butyldiméthylsilyloxy)éthyl]pipérazin-1-yl]propyl] pipéridine-3-carboxylate d'éthyle

1,0 g du composé du stade A précédent est dissous dans 10 ml de pyridine et 0,7 g de TBDMSCI sont ajoutés. Le milieu réactionnel est placé sous agitation à température ambiante pendant 12 heures ; le solvant est éliminé, en présence de toluène, par distillation à l'aide de l'évaporateur rotatif. Une chromatographie sur gel de silice (CH₂Cl₂/MeOH : 95/5) permet d'obtenir 1,15 g du produit attendu.
*INFRAROUGE (*ν*_{cm-1}) :*
*2935 ; 2858 ; 2807 (*ν*C-H) ; 2477 ; 1731 (*ν*C=O ester) ; 1460 (*δ*C-H) ; 1409 ; 1372 ; 1337 ; 1314 ; 1278 ; 1217 ; 1181.*

### PRÉPARATION 53 : Bromure de 3-[N-(indol-1-yl)aminocarbonyl]-1-(4-chlorobutyl)pyridinium

### Stade A : Bromure de 1-(4-chlorobutyl)-3-(méthoxycarbonyl)pyridinium

3,0 g de nicotinate de méthyle sont dissous dans 9 ml de méthanol, puis 5 ml de 1-bromo-4-chlorobutane sont additionnés. Le milieu réactionnel est porté au reflux pendant 24 heures. Après retour à température ambiante, 25 ml d'éther diéthylique sont ajoutés. L'huile formée est isolée par soutirage du surnageant et lavée deux fois avec 20 ml d'éther diéthylique. Après séchage sous pression réduite, 4,2 g du produit attendu sont obtenus.

### Stade B : Bromure de 3-[N-(indoi-1-yl)aminocarbonyl]-1-(4-chlorobutyl)pyridinium

0,28 g du composé de la préparation 2 est dissous dans 4 ml de dichlorométhane anhydre. La solution est refroidie à -20 °C et 2,1 ml d'une solution de triméthylaluminium 2M dans l'hexane sont ajoutés sous argon. Après remontée de la température du milieu réactionnel à 0 °C en 1 heure 30, une solution de 0,6 g du composé du stade A précédent dans 4 ml de dichlorométhane anhydre, est ajoutée. Le milieu réactionnel est ensuite porté au reflux pendant 18 heures. Après retour à température ambiante, 40 ml de dichlorométhane sont additionnés et une solution aqueuse d'hydroxyde de sodium 20 % (p/v) est ajoutée goutte à goutte jusqu'à fin du dégagement de méthane. La phase organique est séparée, lavée successivement avec une solution aqueuse d'hydroxyde de sodium 20 % (p/v) et une solution saturée en NaCl, séchée sur Na₂SO₄ et concentrée jusqu'à un volume d'environ 10 ml. 50 ml d'éther diéthylique sont additionnés. Le solide obtenu est séparé par filtration, puis rincé plusieurs fois à l'éther diéthylique. Après séchage, 0,53 g du composé attendu sont obtenus.
*Point de fusion : 164°C*
*INFRAROUGE (*ν*_{cm-1}) :*
*3028 ; 1625 ; 1589 ; 1556 ; 1443 ; 1286 ; 1210 ; 1174.*

### PRÉPARATION 54 : (±)-1-[2-(4-Butylpipérazin-1-yl)éthyl]pipéridine-3-carboxylate d'éthyle

Le produit est obtenu selon le procédé de la préparation 20 en utilisant la 1-butylpipérazine à la place de la morpholine.

Une chromatographie sur gel de silice (CH₂Cl₂/CH₃OH : 9/1 + 0,5 % NEt₃) permet d'isoler 1,2 g du produit attendu.
*INFRAROUGE (*ν*_{cm-1}) :*
*2935 ; 2806 (*ν*C-H) ; 1732 (*ν*C=O ester) ; 1450 (*δ*C-H) ; 1373* (ν*C-N) ; 1155 (*ν*C-O).*

### PRÉPARATION 55 : (±)-1-(Prop-2-ynyl)pipéridine-3-carboxylate d'éthyle

Le produit est obtenu selon le procédé de la préparation 11 en utilisant le bromure de propargyle à la place du bromure d'allyle.

Une chromatographie éclair sur gel de silice (cyclohexane/AcOEt : 85/15 puis 8/2) permet d'obtenir 500 mg du produit attendu.
*Microanalyses élémentaires :*

| | *C %* | *H %* | *N %* |
|---|---|---|---|
| *Calculé :* | *67, 66* | *8,78* | *7,17* |
| *Trouvé :* | *67,44* | *8,98* | *7,30* |

*INFRAROUGE (*ν*_{cm-1}) :*
*3291 (*ν *≡C-H) ; 2940 ; 2858 ; 2806 (*ν *C-H) ; 1727 (*ν *C=O) ; 1629 (*ν *C=C) ; 1468 ; 1450 (*δ *C-H) ; 1368 ; 1310 (*ν *N-C) ; 1223 ; 1181 (*ν *C-O).*

### PRÉPARATION 56 : (±)-1-[4-(Pipéridin-1-yl)but-2-èn-1-yl]pipéridine-3-carboxylate d'éthyle

Sous une *hotte bien ventilée, à* une solution de 680 mg de 1,4-dibromobutène dans 5 ml de benzène, sont ajoutés, successivement et à température ambiante, goutte à goutte une solution de 500 mg de nipécotate d'éthyle dans 5 ml de benzène et 340 mg de carbonate de sodium. Le milieu réactionnel est agité 18 heures à température ambiante, puis 1,26 ml de pipéridine est ajouté. La suspension est agitée une heure à température ambiante et le milieu réactionnel est évaporé sous pression réduite. Le résidu est dissous dans le dichlorométhane et la phase organique extraite par une solution aqueuse de carbonate de sodium. La phase aqueuse est extraite par du dichlorométhane. Les phases organiques réunies sont séchées sur sulfate de sodium, filtrées et évaporées sous pression réduite. Une chromatographie éclair sur gel de silice (CH₂Cl₂CH₃OH : 95/5 puis 9/1) permet d'obtenir 260 mg du produit attendu.
*INFRAROUGE (*ν*_{cm-1}) :*
*2934 ; 2854 (*ν *C-H) ; 2796 ; 2757 (*ν *N-CH₂) ; 1731 (*ν *C=O) ; 1467 ; 1442 (δ C-H) ; 1368 ; 1352 (*ν *C-N) ; 1218 ; 1180 (*ν *C-O).*

### EXEMPLE 1 : N-(1H-indol-1-yl)-1-méthyl-1,2,5,6-tétrahydropyridine-3-carboxamide

1,94 g de chlorhydrate de 1-méthyl-1,2,5,6-tétrahydropyridine-3-carboxylate de méthyle est solubilisé dans 5 ml d'eau puis la solution est alcalinisée par du carbonate de potassium jusque pH 10 et alors saturée en NaCl. La phase aqueuse est extraite trois fois à l'éther diéthylique. Les phases organiques réunies sont séchées sur Na₂SO₄, filtrées puis évaporées. 1,3 g du composé de la préparation 2 est dissout dans 26 ml de dichlorométhane anhydre puis après refroidissement à -25°C, 9 ml d'une solution de triméthylaluminium 2M dans l'hexane sont ajoutés. Après 1h30, une solution de 1,27 g d'arécoline dans 6,5 ml de dichlorométhane anhydre est ajoutée à température ambiante. Le mélange réactionnel est chauffé la nuit au reflux puis dilué alors par du dichlorométhane et versé dans 50 ml d'une solution de soude aqueuse à 20%. La phase organique est isolée et la phase aqueuse extraite par du dichlorométhane. Les phases organiques réunies sont lavées par une solution saturée en NaCl, séchées sur sulfate de sodium, filtrées puis évaporées sous pression réduite. Une chromatographie éclair sur gel de silice (CH₂Cl₂/MeOH : 95/5 puis 9/1) permet d'isoler 470 mg du produit attendu.
*Point de fusion : 194°C*
*Microanalyses élémentaires :*

| | *C %* | *H %* | *N %* |
|---|---|---|---|
| *Calculé :* | *70,56* | *6,71* | *16,46* |
| *Trouvé :* | *70,35* | *6,80* | *16,36* |

*Spectrométrie de masse (ESI* +, *m*/*z) : 256,1 (M*+*H*⁺*); 278,1 (M*+*Na*⁺*).*

### EXEMPLE 2 : N-(1H-indol-1-yl)-1-méthyl-1,2,3,6-tétrahydropyridine-3-carboxamide

Le produit attendu est obtenu au cours de la purification du composé de l'exemple 1. 180 mg du composé sont obtenus après reprise du résidu par l'éther diéthylique, trituation et filtration sur verre fritté.
*Point de fusion : 117°C*
*Microanalyses élémentaires :*

| | *C %* | *H %* | *N %* |
|---|---|---|---|
| *Calculé :* | *70,56* | *6,71* | *16,46* |
| *Trouvé :* | *70,08* | *6,81* | *16,24* |

### EXEMPLE 3 : N-(1H-indol-1-yl)-1-méthyl-1,4,5,6-tétrahydropyridine-3-carboxamide

1 g du composé de la préparation 2 est solubilisé dans 10 ml de dichlorométhane anhydre puis le mélange réactionnel est refroidi à -20°C. 5,5 ml d'une solution de triméthylaluminium 2M dans l'hexane sont ajoutés et la réaction est agitée 1h30 en laissant évoluer la température. Une solution de 1,5 g du composé de la préparation 7 dans 5 ml de dichlorométhane est ajoutée et la réaction est chauffée 12 heures au reflux. Le mélange réactionnel est dilué par du dichlorométhane puis versé dans une solution de soude aqueuse à 20%. La phase organique est extraite, d'abord lavée par une solution de soude à 20%, puis par une solution saturée en NaCl. La phase organique est séchée sur sulfate de sodium, filtrée puis évaporée sous pression réduite. Une chromatographie éclair sur gel de silice (dichlorométhane) permet d'isoler 0,640 g du produit attendu.
*Point de fusion : 207°C*
*Microanalyses élémentaires :*

| | *C %* | *H %* | *N %* |
|---|---|---|---|
| *Calculé :* | *70,56* | *6,71* | *16,46* |
| *Trouvé :* | *70,40* | *6,70* | *16,35* |

*Spectrométrie de masse (ESI +, m*/*z): 256 (M*+*H*⁺*).*

### EXEMPLE 4 : N-(2,3-dihydro-1H-indol-1-yl)-1-méthyl-1,4,5,6-tétrahydro pyridine-3-carboxamide

### StadeA : N-(2,3-dihydro-1H-indol-1-yl)nicotinamide

À 600 mg du composé de la préparation 2 dissous dans 18 ml de dichlorométhane anhydre sont ajoutés à 0°C 108 mg de DMAP, 1,9 ml de triéthylamine suivi de l'addition de 955 mg de chlorhydrate de chlorure de nicotinoyle. La réaction est agitée la nuit à température ambiante puis le milieu réactionnel est dilué par du dichlorométhane puis lavé par une solution de carbonate de potassium jusqu'à pH 11. La phase aqueuse est extraite par du dichlorométhane. Les phases organiques réunies sont lavées par une solution saturée en NaCl, séchées sur Na₂SO₄, filtrées puis évaporées sous pression réduite. Une chromatographie éclair sur gel de silice (CH₂Cl₂ puis CH₂Cl₂/CH₃OH : 96/4 et 95/5) permet d'isoler 600 mg du produit attendu.
*Infrarouge* (ν*_{cm-1}) :*
*3224 (*ν *N-H); 3048 (*ν *=C-H); 2845 (*ν *C-H); 1656 (*ν *C=O); 1590; 1532 (*ν *C=C).*

### Stade B : Iodure de 3-[(2,3-dihydro-1H-indol-1-ylamino)carbonyl]-1-méthylpyridinium

420 mg du composé du stade A précédent sont dissous dans 1,56 ml d'iodométhane. Il se forme une huile brune qui est agitée 24 heures à température ambiante à l'abri de la lumière. L'excès d'iodométhane est éliminé sous pression réduite. 670 mg du produit attendu sont ainsi obtenus .
*Infrarouge (*ν*_{cm-1}) :*
*3224 (*ν *N-H); 3048 (*ν *=C-H); 2845 (*ν *C-H); 1656 (*ν *C=O); 1590; 1532 (*ν *C=C).*

### Stade C: N-(2,3-dihydro-1H-indol-1-yl)-1-méthyl-1,4,5,6-tétrahydropyridine-3-carboxamide

1,1 g du composé du stade B précédent est dissous dans 15 ml de méthanol puis 450 µl de triéthylamine et 100 mg de PtO₂ sont ajoutés. Le mélange réactionnel est dégazé puis placé sous atmosphère d'hydrogène (opération renouvelée 2 fois). Le mélange réactionnel est agité une heure à température ambiante puis purgé à l'azote. Le mélange est filtré sur célite (élution : méthanol) puis les solvants sont évaporés sous pression réduite. Une chromatographie éclair sur gel de silice (AcOEt) permet d'isoler 320 mg du produit attendu.
*Point de fusion : 127°C*
*Microanalyses élémentaires :*

| | *C %* | *H %* | *N %* |
|---|---|---|---|
| *Calculé :* | *70,01* | *7,44* | *16,33* |
| *Trouvé :* | *69,80* | *7,55* | *16,24* |

### EXEMPLE 5 : 1-Méthyl-N-(2-méthyl-2,3-dihydro-1H-indol-1-yl)-1,2,5,6-tétrahydropyridine-3-carboxamide

Le produit est obtenu selon le procédé de l'exemple 3 en utilisant le composé de la préparation 8 à la place du composé de la préparation 2 et le chlorhydrate d'arécoline à la place du composé de la préparation 7.
*Point de fusion : 178°C*
*Microanalyses élémentaires :*

| | *C %* | *H %* | *N %* |
|---|---|---|---|
| *Calculé :* | *70,82* | *7,80* | *15,49* |
| *Trouvé :* | *69,97* | *7,89* | *15,11* |

*Spectrométrie de masse (ESI* +, *m*/*z): 272 (M*+*H*⁺*)*

### EXEMPLE 6 : N-(5-chloro-1H-indol-1-yl)-1-méthyl-1,2,5,6-tétrahydropyridine-3-carboxamide

Le produit est obtenu selon le procédé de l'exemple 3 en utilisant le composé de la préparation 3 à la place du composé de la préparation 2 et l'arécoline à la place du composé de la préparation 7.
*Point de fusion : 155°C*
*Microanalyses élémentaires :*

| | *C %* | *H %* | *N %* |
|---|---|---|---|
| *Calculé :* | *62,18* | *5,57* | *14,50* |
| *Trouvé :* | *62,09* | *5,59* | *14,44* |

### EXEMPLE 7 : N-(5-fluoro-1H-indol-1-yl)-1-méthyl-1,2,5,6-tétrahydropyridine-3-carboxamide

Le produit est obtenu selon le procédé de l'exemple 3 en utilisant le composé de la préparation 9 à la place du composé de la préparation 2 et l'arécoline à la place du composé de la préparation 7.
*Point de fusion : 105°C*
*Microanalyses élémentaires :*

| | *C %* | *H %* | *N %* |
|---|---|---|---|
| *Calculé :* | *65,92* | *5,90* | *15,37* |
| *Trouvé :* | *65,75* | *5,96* | *15,19* |

### EXEMPLE 8 : N-(5-méthoxy-1H-indol-1-yl)-1-méthyl-1,2,5,6-tétrahydropyridine-3-carboxamide

Le produit est obtenu selon le procédé de l'exemple 3 en utilisant le composé de la préparation 10 à la place du composé de la préparation 2 et l'arécoline à la place du composé de la préparation 7.
*Point de fusion : 162°C*
*Microanalyses élémentaires :*

| | *C %* | *H %* | *N %* |
|---|---|---|---|
| *Calculé :* | *67,35* | *6,71* | *14, 72* |
| *Trouvé :* | *66,76* | *6,85* | *14,73* |

### EXEMPLE 9 : N-(2,3-diméthyl-1H-indol-1-yl)-1-méthyl-1,2,5,6-tétrahydro pyridine-3-carboxamide

Le produit est obtenu selon le procédé de l'exemple 3 en utilisant le composé de la préparation 5 à la place du composé de la préparation 2 et l'arécoline à la place du composé de la préparation 7.
*Point de fusion : 168°C*
*Microanalyses élémentaires :*

| | *C %* | *H %* | *N %* |
|---|---|---|---|
| *Calculé :* | *72,06* | *7,47* | *14,83* |
| *Trouvé :* | *71,59* | *7,74* | *14,75* |

### EXEMPLE 10 : 1-Méthyl-N-(3-méthyl-1H-indol-1-yl)-1,2,5,6-tétrahydro pyridine-3-carboxamide

Le produit est obtenu selon le procédé de l'exemple 3 en utilisant le composé de la préparation 4 à la place du composé de la préparation 2 et l'arécoline à la place du composé de la préparation 7.
*Point de fusion : 152°C*
*Microanalyses élémentaires :*

| | *C %* | *H %* | *N %* |
|---|---|---|---|
| *Calculé :* | *71,35* | *7,11* | *15,60* |
| *Trouvé :* | *71,36* | *7,17* | *15,59* |

### EXEMPLE 11 : N-(1H-indol-1-yl)-1-méthyl-1,2,5,6-tétrahydropyridine-4-carboxamide

Le produit est obtenu selon le procédé de l'exemple 3 en utilisant le 1-méthyl-1,2,5,6-tétrahydropyridine-4-carboxylate de méthyle à la place du composé de la préparation 7.
*Point de fusion : 112°C*
*Microanalyses élémentaires :*

| | *C %* | *H %* | *N %* |
|---|---|---|---|
| *Calculé :* | *70,56* | *6,71* | *16,46* |
| *Trouvé :* | *70,23* | *6,79* | *16,17* |

### EXEMPLE 12 : 1-Allyl-N-(5-chloro-1H-indol-1-yl)-pipéridine-3-carboxamide

Le produit est obtenu selon le procédé de l'exemple 3 en utilisant le composé de la préparation 3 à la place du composé de la préparation 2 et le composé de la préparation 11 à la place du composé de la préparation 7.
*Point de fusion : 130°C*
*Microanalyses élémentaires :*

| | *C %* | *H %* | *N %* |
|---|---|---|---|
| *Calculé :* | *64,25* | *6,34* | *13,22* |
| *Trouvé :* | *64,18* | *6,47* | *13,20* |

### EXEMPLE 13 : N-(5-chloro-1H-indol-1-yl)-pipéridine-3-carboxamide

Le produit est obtenu selon le procédé de l'exemple 3 en utilisant le composé de la préparation 3 à la place du composé de la préparation 2 et le nipécotate d'éthyle à la place du composé de la préparation 7.
*Point de fusion : 167°C*
*Microanalyses élémentaires :*

| | *C%* | *H %* | *N %* |
|---|---|---|---|
| *Calculé :* | *60,54* | *5,81* | *15,13* |
| *Trouvé :* | *60,36* | *5,93* | *15,12* |

*Spectrométrie de masse (IE): 277 (M*+*H*⁺*)*

### EXEMPLE 14 : Chlorure de 3-[(1H-indol-1-ylamino)carbonyl]-1-méthylpipéridinium

### Stade A : N-(1H-indol-1-yl)-1-méthyl-pipéridine-3-carboxamide

Le produit est obtenu selon le procédé de l'exemple 3 en utilisant le composé de la préparation 12 à la place du composé de la préparation 7.
*Spectrométrie de masse (ESI* +, *m*/*z): 258 (M*+*H*⁺*)*

### Stade B : Chlorure de 3-[(1H-indol-1-ylamino)carbonyl]-1-méthylpipéridinium

À une solution de 228 mg du composé du stade A précédent dans 6 ml de THF anhydre, sont ajoutées 6 gouttes de HCl concentré. Le solvant est évaporé puis on lave le résidu avec de l'éther diéthylique permettant d'obtenir 256 mg du produit attendu.
*Point de fusion :* > *250°C*
*Microanalyses élémentaires :*

| | *C %* | *H %* | *N %* |
|---|---|---|---|
| *Calculé :* | *61,32* | *6,86* | *14,30* |
| *Trouvé :* | *61,14* | *6,99* | *14,16* |

### EXEMPLE 15 : N-(2,3-dihydro-1H-indol-1-yl)-1-méthyl-1,2,5,6-tétrahydro-pyridine-3-carboxamide

Le produit est obtenu selon le procédé de l'exemple 3 en utilisant le composé de la préparation 6 à la place du composé de la préparation 2 et l'arécoline à la place du composé de la préparation 7.
*Point de fusion : 152°C*
*Microanalyses élémentaires :*

| | *C %* | *H %* | *N %* |
|---|---|---|---|
| *Calculé :* | *70,01* | *7,44* | *16,33* |
| *Trouvé :* | *69,99* | *7,47* | *16,28* |

### EXEMPLE 16 : 1-Méthyl-N-(2-méthyl-2,3-dihydro-1H-indol-1-yl)-pipéridine-3-carboxamide

Le produit est obtenu selon le procédé de l'exemple 3 en utilisant le composé de la préparation 8 à la place du composé de la préparation 2 et le composé de la préparation 12 à la place du composé de la préparation 7.
*Point de fusion : 133°C*
*Microanalyses élémentaires :*

| | *C %* | *H %* | *N %* |
|---|---|---|---|
| *Calculé :* | *70,30* | *8,48* | *15,37* |
| *Trouvé :* | *70,19* | *8,56* | *15,23* |

*Spectrométrie de masse (IE): 273 (M*+*H*⁺*)*

### EXEMPLE 17 : N-(5-chloro-1H-indol-1-yl)-1-propyl-pipéridine-3-carboxamide

Le produit est obtenu selon le procédé de l'exemple 3 en utilisant le composé de la préparation 13 à la place du composé de la préparation 7.
*Point de fusion : 157°C*
*Microanalyses élémentaires :*

| | *C %* | *H %* | *N %* |
|---|---|---|---|
| *Calculé :* | *63,84* | *6,94* | *13,14* |
| *Trouvé :* | *63,30* | *7,09* | *13,12* |

*Spectrométrie de masse (ESI* +,*m*/*z): 320, 322 (M*+*H*⁺*)*

### EXEMPLE 18 : N-(5-chloro-1H-indol-1-yl)-1-(2-hydroxyéthyl)-1,2,5,6-tétrahydro-3-pyridinecarboxamide

### Stade A : 1-(2-{[tert-Butyl(diméthyl)silyl]oxy}éthyl)-N-(5-chloro-1H-indol-1-yl)-1,2,5,6-tétrahydro-3-pyridinecarboxamide

1 g du composé de la préparation 3 est solubilisé dans 10 ml de dichlorométhane anhydre puis le mélange réactionnel est refroidi à -20°C. 5,5 ml de triméthylaluminium 2M/hexane sont ajoutés et la réaction est agitée 1h30 en laissant évoluer la température. Une solution de 1,5 g du composé de la préparation 15 dans 5 ml de dichlorométhane est ajoutée et la réaction est chauffée la nuit au reflux du dichlorométhane. Le mélange réactionnel est dilué par du dichlorométhane puis versé dans une solution de soude aqueuse à 20%. La phase organique est extraite puis lavée par une solution de soude à 20%, puis par une solution saturée en NaCl. La phase organique est séchée sur sulfate de sodium, filtrée puis évaporée sous pression réduite. Une chromatographie éclair sur gel de silice (AcOEt/Cyclohexane : 1/1 puis 2/1) permet d'isoler 1,5 g du produit attendu.
*Infrarouge (*ν*_{cm-1}) :*
*3244 (*ν *N-H); 2953; 2928; 2856 (*ν *C-H); 1672 (*ν *C=O); 1642; 1520 (*ν *C=C).*

### Stade B : N-(5-chloro-1H-indol-1-yl)-1-(2-hydroxyéthyl)-1,2,5,6-tétrahydro-3-pyridinecarboxamide

À une solution de 1,46 g du composé du stade A précédent dans 12 ml de THF anhydre sont ajoutés 6,72 ml de Bu₄NF 1M/THF puis le mélange réactionnel est agité la nuit à température ambiante. Le mélange est concentré sous pression réduite puis repris par du dichlorométhane, lavé avec de l'eau puis par une solution de carbonate de sodium à pH 11. Les phases aqueuses sont extraites par du dichlorométhane. Les phases organiques réunies sont séchées sur sulfate de sodium, filtrées puis évaporées sous pression réduite. Une chromatographie éclair sur gel de silice (AcOEt/MeOH : 95/5 puis 9/1) permet d'isoler 920 mg du produit attendu.
*Point de fusion : 84°C*
*Microanalyses élémentaires :*

| | *C %* | *H %* | *N %* |
|---|---|---|---|
| *Calculé :* | *60, 09* | *5,67* | *13,14* |
| *Trouvé :* | *59,94* | *5,78* | *12,98* |

### EXEMPLE 19 : Dichlorhydrate de 1-[2-(diméthylamino)éthyl]-N-(1H-indol-1-yl)-1,2,5,6-tétrahydro-3-pyridinecarboxamide

### Stade A : 1-[2-(Diméthylamino)éthyl]-N-(1H-indol-1-yl)-1,2,5,6-tétrahydro-3-pyridinecarboxamide

Le produit est obtenu selon le procédé du stade A de l'exemple 18 en utilisant le composé de la préparation 2 à la place du composé de la préparation 3 et le composé de la préparation 16 à la place du composé de la préparation 15.

### Stade B : Dichlorhydrate de 1-[2-(diméthylamino)éthyl]-N-(1H-indol-1-yl)-1,2,5,6-tétrahydro-3-pyridinecarboxamide

200 mg du composé du stade A précédent sont dissous dans 0,5 ml de méthanol puis 285 µl d'acide chlorhydrique méthanolique 4,53M sont ajoutés. La solution est noyée par de l'éther et le précipité formé est filtré puis rincé avec de l'éther. 210 mg du produit attendu sont obtenus par séchage sous pression réduite.
*Point de fusion : 210°C*
*Microanalyses élémentaires :*

| | *C %* | *H %* | *N %* |
|---|---|---|---|
| *Calculé :* | *53,60* | *7,00* | *13,89* |
| *Trouvé :* | *53,63* | *7,41* | *13,42* |

### EXEMPLE 20 : N-(5-chloro-1H-indol-1-yl)-1-[2-(diméthylamino)éthyl]-1,4,5,6-tétrahydro-3-pyridinecarboxamide

Le produit est obtenu selon le procédé du stade A de l'exemple 18 en utilisant le composé de la préparation 18 à la place du composé de la préparation 15.
*Point de fusion : 166°C*
*Microanalyses élémentaires :*

| | *C %* | *H %* | *N %* |
|---|---|---|---|
| *Calculé :* | *62,33* | *6,68* | *16,15* |
| *Trouvé :* | *62,17* | *6,73* | *16,04* |

*Spectrométrie de masse (ESI* +, *m*/*z): 347,1; 349,1 (M*+*H*⁺*); 369,1; 371,1 (M*+*Na*⁺*).*

### EXEMPLE 21 : 1-[2-(Diméthylamino)éthyl]-N-(1H-indol-1-yl)-1,4,5,6-tétrahydro-3-pyridinecarboxamide

Le produit est obtenu selon le procédé du stade A de l'exemple 18 en utilisant le composé de la préparation 2 à la place du composé de la préparation 3 et le composé de la préparation 18 à la place du composé de la préparation 15.
*Point de fusion : 106°C*
*Microanalyses élémentaires :*

| | *C %* | *H %* | *N %* |
|---|---|---|---|
| *Calculé :* | *69,20* | *7,74* | *17,93* |
| *Trouvé :* | *68,98* | *7,79* | *17,84* |

*Spectrométrie de masse (ESI* +, *m*/*z): 313,1 (M*+*H*⁺*); 335,2 (M*+*Na*⁺*).*

### EXEMPLE 22 : 1-[2-(Diméthylamino)éthyl]-N-(1H-indol-1-yl)-3-pipéridine carboxamide

Le produit est obtenu selon le procédé du stade A de l'exemple 18 en utilisant le composé de la préparation 2 à la place du composé de la préparation 3 et le composé de la préparation 19 à la place du composé de la préparation 15.
*Point de fusion : 79°C*
*Microanalyses élémentaires :*

| | *C %* | *H %* | *N %* |
|---|---|---|---|
| *Calculé :* | *68,76* | *8,33* | *17,82* |
| *Trouvé :* | *68,70* | *8,41* | *17,79* |

*Spectrométrie de masse (ESI* +, *m*/*z): 315,2 (M*+*H*⁺*); 337,2 (M*+*Na*⁺*).*

### EXEMPLE 23 : N-(1H-indol-1-yl)-1-[2-(4-morpholinyl)éthyl]-3-pipéridine carboxamide

Le produit est obtenu selon le procédé du stade A de l'exemple 18 en utilisant le composé de la préparation 2 à la place du composé de la préparation 3 et le composé de la préparation 20 à la place du composé de la préparation 15.
*Point de fusion : 131°C*
*Microanalyses élémentaires :*

| | *C %* | *H %* | *N %* |
|---|---|---|---|
| *Calculé :* | *67,39* | *7,92* | *15,72* |
| *Trouvé :* | *67,44* | *8,04* | *15,78* |

### EXEMPLE 24 : N-(1H-indol-1-yl)-1-[2-(1-pipéridinyl)éthyl]-3-pipéridinecarboxamide

Le produit est obtenu selon le procédé du stade A de l'exemple 18 en utilisant le composé de la préparation 2 à la place du composé de la préparation 3 et le composé de la préparation 21 à la place du composé de la préparation 15.
*Point de fusion : 113°C*
*Microanalyses élémentaires :*

| | *C %* | *H %* | *N %* |
|---|---|---|---|
| *Calculé :* | *71,15* | *8,53* | *15,80* |
| *Trouvé :* | *71,01* | *8,54* | *15,75* |

*Infrarouge (*ν*_{cm-1}) :*
*3108 (*ν*N-H amide +* ν*=C-H), 2921, 2853 (*ν*C-H), 1690 (*ν*C=O ester).*

### EXEMPLE 25 : N-(1H-indol-1-yl)-1-[2-(4-méthyl-1-pipérazinyl)éthyl]-3-pipéridinecarboxamide

Le produit est obtenu selon le procédé du stade A de l'exemple 18 en utilisant le composé de la préparation 2 à la place du composé de la préparation 3 et le composé de la préparation 22 à la place du composé de la préparation 15.
*Point de fusion : 117°C*
*Microanalyses élémentaires :*

| | *C %* | *H %* | *N %* |
|---|---|---|---|
| *Calculé :* | *68,26* | *8,46* | *18,95* |
| *Trouvé:* | *68,14* | *8,49* | *18,88* |

### EXEMPLE 26 : 1-{2-[4-(2-Hydroxyéthyl)-1-pipérazinyl]ethyl}-N-(1H-indol-1-yl)-3-pipéridinecarboxamide

Le produit est obtenu selon le procédé du stade A de l'exemple 18 en utilisant le composé de la préparation 2 à la place du composé de la préparation 3 et le composé de la préparation 23 à la place du composé de la préparation 15.
*Point de fusion : 118°C*
*Microanalyses élémentaires :*

| | *C %* | *H %* | *N %* |
|---|---|---|---|
| *Calculé :* | *66,14* | *8,32* | *17,53* |
| *Trouvé :* | *65,96* | *8,33* | *17,49* |

### EXEMPLE 27 : N-(1H-indol-1-yl)-1-[2-(1-pyrrolidinyl)éthyl]-3-pipéridine carboxamide

Le produit est obtenu selon le procédé du stade A de l'exemple 18 en utilisant le composé de la préparation 2 à la place du composé de la préparation 3 et le composé de la préparation 24 à la place du composé de la préparation 15.
*Point de fusion : 117°C*
*Microanalyses élémentaires :*

| | *C %* | *H %* | *N %* |
|---|---|---|---|
| *Calculé :* | *70,56* | *8,29* | *16,46* |
| *Trouvé :* | *70,54* | *8,21* | *16,44* |

### EXEMPLE 28 : 1-[2-(1-Azépanyl)éthyl]-N-(1H-indol-1-yl)-3-pipéridinecarboxamide

Le produit est obtenu selon le procédé du stade A de l'exemple 18 en utilisant le composé de la préparation 2 à la place du composé de la préparation 3 et le composé de la préparation 25 à la place du composé de la préparation 15.
*Point de fusion :*> *250°C*
*Microanalyses élémentaires :*

| | *C %* | *H %* | *N %* |
|---|---|---|---|
| *Calculé :* | *58,90* | *7,82* | *12,48* |
| *Trouvé :* | *58,88* | *7,84* | *12,38* |

### EXEMPLE 29 : Trichlorhydrate de N-(1H-indol-1-yl)-1-[2-(4-phényl-1-pipérazinyl)-éthyl]-3-pipéridinecarboxamide

Le produit est obtenu selon le procédé du stade A de l'exemple 18 en utilisant le composé de la préparation 2 à la place du composé de la préparation 3 et le composé de la préparation 26 à la place du composé de la préparation 15.

430 mg du résidu obtenu sont dissout dans 1,5 ml de MeOH anhydre puis 1,65 ml d'une solution HCl méthanolique 2M est ajouté. Le trichlorhydrate est obtenu par précipitation par ajout de 5 ml d'éther, filtration puis séchage sous vide.
*Point de fusion :* > *250°C*
*Microanalyses élémentaires :*

| | *C %* | *H %* | *N %* |
|---|---|---|---|
| *Calculé :* | *55,87* | *6,85* | *12,52* |
| *Trouvé :* | *55,84* | *6,83* | *12,45* |

### EXEMPLE 30 : Trichlorhydrate de N-(1H-indol-1-yl)-N-({1-[2-(1-pipéridinyl)éthyl]-3-pipéridinyl}méthyl)amine

Le produit est obtenu selon le procédé du stade A de l'exemple 18 en utilisant le composé de la préparation 2 à la place du composé de la préparation 3 et le composé de la préparation 27 à la place du composé de la préparation 15.

310 mg du résidu obtenu sont dissout dans 1,5 ml de MeOH anhydre puis 1,5 ml d'une solution HCl méthanolique 2M est ajouté. Le trichlorhydrate est obtenu par précipitation par ajout de 5 ml d'éther, filtration puis séchage sous vide.
*Point de fusion :* > *250°C*
*Microanalyses élémentaires :*

| | *C %* | *H %* | *N %* |
|---|---|---|---|
| *Calculé :* | *56, 06* | *7,84* | *12,45* |
| *Trouvé :* | *55,88* | *8,02* | *12,29* |

### EXEMPLE 31 : N-(5-chloro-2,3-dihydro-1H-indol-1-yl)-1-(2-hydroxyéthyl)-1,4,5,6-tétrahydro-3-pyridinecarboxamide

### StadeA : N-(5-chloro-2,3-dihydro-1H-indol-1-yl)nicotinamide

Le produit est obtenu selon le procédé du stade A de l'exemple 4 en utilisant le composé de la préparation 28 à la place du composé de la préparation 2 .
*Infrarouge (*ν*_{cm-1}) :*
*3265 (*ν *N-H); 3025 (*ν *=C-H); 2975; 2883 (*ν *C-H); 1652 (*ν *C=O); 1605; 1591; 1578; 1537 (*ν *C=C).*

### Stade B : Bromure de 3-{[(5-chloro-2,3-dihydro-1H-indol-1-yl)amino]carbonyl}-1-(2-hydroxyéthyl)pyridinium

Le produit est obtenu selon le procédé du stade B de l'exemple 4 en utilisant le composé du stade A précédent et en utilisant du 2-bromoéthanol à la place du iodométhane.
*Infrarouge (*ν*_{cm-1}) :*
*3349 (*ν *N-H,* ν *O-H); 3052 (*ν *=C-H); 2854 (*ν *C-H); 1675 (*ν *C=O);*
*1634; 1606; 1543 (*ν *C=C).*

### Stade C: N-(5-chloro-2,3-dihydro-1H-indol-1-yl)-1-(2-hydroxyéthyl)-1,4,5,6-tétrahydro-3-pyridinecarboxamide

Le produit est obtenu selon le procédé du stade C de l'exemple 4 en utilisant le composé du stade B précédent.
*Point de fusion : 161°C*
*Microanalyses élémentaires :*

| | *C %* | *H %* | *N %* |
|---|---|---|---|
| *Calculé :* | *59,72* | *6,26* | *13,06* |
| *Trouvé :* | *59,86* | *6,35* | *13,16* |

### EXEMPLE 32 : Dichlorhydrate de N-(2,3-dihydro-1H-indol-1-yl)-1-[2-(diméthylamino)éthyl]-3-pipéridinecarboxamide

### Stade A : N-(2,3-dihydro-1H-indol-1-yl)-1-[2-(diméthylamino)éthyl]-3-pipéridine carboxamide

À 500 mg du composé de l'exemple 22 dissous dans 3 ml de THF anhydre, sont ajoutés 3 ml d'acide trifluoroacétique. La réaction est placée sous courant d'azote puis une solution de 1 g de cyanoborohydrure de sodium dans 10 ml de THF sont ajoutés goutte à goutte pendant 2 heures. Les solvants sont évaporés sous pression réduite, le résidu est repris par du dichlorométhane et par de l'eau, le milieu est alcalinisé par une solution de carbonate de potassium jusque pH 11, la phase aqueuse est ensuite extraite deux fois par du dichlorométhane. Les phases organiques réunies sont séchées sur sulfate de sodium, filtrées puis évaporées sous pression réduite. Le résidu est repris dans 13 ml d'éthanol absolu puis 1,5 ml d'acide chlorhydrique 2N sont ajoutés. Le mélange réactionnel est agité 5 heures au reflux puis évaporé sous pression réduite. Le résidu est repris par 20 ml d'un mélange 1/1 de dichlorométhane et d'eau, la phase aqueuse étant alcalinisée jusqu'à pH 11 par l'ajout de carbonate de sodium. La phase organique est extraite puis la phase aqueuse est extraite deux fois par du dichlorométhane. Les phases organiques réunies sont séchées sur sulfate de sodium, filtrées puis évaporées. Une chromatographie éclair sur gel de silice (AcOEt/MeOH : 9/1 puis 8/2 à 6/4) permet d'isoler 255 mg du produit attendu.

### Stade B : Dichlorhydrate de N-(2,3-dihydro-1H-indol-1-yl)-1-[2-(diméthylamino) éthyl]-3-pipéridinecarboxamide

255 mg du composé du stade A précédent sont dissous dans 4 ml de dichlorométhane puis 360 µl d'acide chlorhydrique méthanolique 4,53 M sont ajoutés. La solution est agitée 5 minutes puis un large excès d'éther est ajouté : il se forme un précipité qui est filtré sur verre fritté permettant d'isoler 270 mg du produit attendu.
*Point de fusion : 108°C*
*Microanalyses élémentaires :*

| | *C %* | *H %* | *N %* |
|---|---|---|---|
| *Calculé :* | *55,52* | *7,77* | *14,39* |
| *Trouvé :* | *55,63* | *7,91* | *14,23* |

### EXEMPLE 33 : N-(5-chloro-1H-indol-1-yl)-1-(2-hydroxyéthyl)-1,4,5,6-tétrahydro-3-pyridinecarboxamide

Le produit est obtenu selon le procédé du stade A de l'exemple 18 en utilisant le composé de la préparation 17 à la place du composé de la préparation 15.
*Point de fusion* : *195°C*
*Microanalyses élémentaires :*

| | *C %* | *H %* | *N %* |
|---|---|---|---|
| *Calculé :* | *60,69* | *5,67* | *13,14* |
| *Trouvé :* | *60,20* | *5,86* | *12,90* |

### EXEMPLE 34 : 1-(2-Hydroxyéthyl)-N-(1H-indol-1-yl)-1,4,5,6-tétrahydro-3-pyridinecarboxamide

### StadeA : 1-(2-{[tert-Butyl(diméthyl)silyl]oxy}méthyl)-N-(1H-indol-1-yl)-1,4,5,6-tétrahydro-3-pyridine carboxamide

Le produit est obtenu selon le procédé du stade A de l'exemple 18 en utilisant le composé de la préparation 2 à la place du composé de la préparation 3 et le composé de la préparation 29 à la place du composé de la préparation 15.

### Stade B : 1-(2-Hydroxyéthyl)-N-(1H-indol-1-yl)-1,4,5,6-tétrahydro-3-pyridinecarboxamide

Le produit est obtenu selon le procédé du stade B de l'exemple 18 en utilisant le composé du stade A précédent.
*Point de fusion : 152°C*
*Microanalyses élémentaires :*

| | *C %* | *H %* | *N %* |
|---|---|---|---|
| *Calculé :* | *67,35* | *6,71* | *14,73* |
| *Trouvé :* | *67,37* | *6,81* | *14,70* |

*Spectrométrie de masse (ESI* +, *m*/*z):* 286 *(M*+*H*⁺*)*

### EXEMPLE 35 : Dichlorhydrate de N-(indol-1-yl)-N-[(1-méthyl-1,2,5,6-tétrahydropyridin-3-yl)méthyl]amine

### StadeA : (Z,E)-(Indol-1-yl)[(pyridin-3-yl)méthylène]amine

2,0 g de pyridine-3-carboxaldéhyde sont dissous avec 100 ml de toluène, puis 3,7 g du composé de la préparation 2 sont ajoutés. Le mélange est placé dans un ballon équipé d'un Dean-Stark et porté au reflux sous agitation jusqu'à obtention du volume théorique d'eau (18 heures). Le milieu réactionnel est concentré à l'évaporateur rotatif, puis le résidu est purifié par chromatographie sur gel de silice (CH₂Cl₂ / MeOH : 98/2) permettant d'obtenir 3,8 g du composé attendu.
*Point de fusion : 112°C*

### Stade B : Iodure de 3-[(Z,E)-N-(indol-1-yl)iminométhyl]-1-méthylpyridinium

À une solution de 2,0 g du composé du stade A précédent dans 10 ml de méthanol, sont ajoutés 5 ml de iodométhane. Le mélange réactionnel est agité 24 heures à température ambiante. 50 ml d'éther diéthylique sont additionnés ; le précipité est séparé par filtration et séché pour donner 3,05 g du composé attendu.
*Point de fusion : 218°C*
*Microanalyses élémentaires :*

| | *C %* | *H %* | *N %* |
|---|---|---|---|
| *Calculé :* | *49,60* | *3,89* | *11,57* |
| *Trouvé :* | *49,62* | *3,98* | *11,48* |

*INFRAROUGE (*ν*_{cm-1}) :*
*3120 ; 3038 ; 1633 ; 1593 ; 1475 ; 1450 ; 1297 ; 1251 ; 1158.*

### Stade C : (Z,E)-(Indol-1-yl)-[(1-méthyl-1,2,5,6-tétrahydropyridin-3-yl]méthylène)]amine

À une solution de 1,0 g du composé du stade B précédent dans 20 ml de méthanol, refroidie à 0°C, sont ajoutés 522 mg de NaBH₄ par petites portions. Après l'addition, la température est remontée à température ambiante, puis le mélange réactionnel est agité pendant une heure. 50 ml d'une solution saturée d'hydrogénocarbonate de sodium sont ensuite additionnés et le milieu est extrait par 3 x 30 ml de dichlorométhane. Les phases organiques réunies sont extraites successivement par une solution d'hydrogénocarbonate de sodium et une solution saturée en NaCl. Après séchage sur Na₂SO₄, les solvants sont éliminés sous vide à l'évaporateur rotatif. Une chromatographie sur gel de silice (CH₂Cl₂/MeOH : 95/5) permet d'isoler 0,51 g du produit attendu.
*Microanalyses élémentaires :*

| | *C %* | *H %* | *N %* |
|---|---|---|---|
| *Calculé :* | *75,28* | *7,16* | *17,56* |
| *Trouvé :* | *72,02* | *7,35* | *17,75* |

*Infrarouge (*ν*_{cm-1}) :*
*3104 ; 3053 ; 2964 ; 2960 ; 2930 ; 2895 ; 2846 ; 2762 ; 1642 ; 1614 ; 1454 ; 1292 ; 1255.*

### Stade D : Dichlorhydrate de (indol-1-yl)[(1-méthyl-1,2,5,6-tétrahydropyridin-3-yl) méthyl]amine

À une solution de 0,65 g du composé du stade C précédent dans 10 ml d'éther diéthylique, est ajouté 0,4 g de LiAlH₄ par petites portions à température ambiante. La réaction est agitée 2 heures à température ambiante, puis 10 ml d'eau sont ajoutés. Le milieu réactionnel est extrait par 3 x 20 ml de dichlorométhane ; la phase organique est lavée avec une solution saturée d'hydrogénocarbonate de sodium, séchée sur sulfate de sodium et filtrée, puis évaporée sous pression réduite. Le résidu qui est purifié par chromatographie sur gel de silice (CH₂Cl₂/MeOH : 95/5 + 0,5% NEt₃) permet d'obtenir 0,56 g de la base. 100 mg de la base sont dissous dans 0,5 ml de MeOH anhydre, puis 0,45 ml d'une solution d'HCl méthanolique 2M est ajouté. Le dichlorhydrate précipite par ajout de 5 ml d'éther diéthylique ; il est séparé par filtration, puis séché sous vide.
*Point de fusion : 152°C*
*Microanalyses élémentaires :*

| | *C %* | *H %* | *N %* |
|---|---|---|---|
| *Calculé :* | *54,22* | *6,98* | *12,65* |
| *Trouvé :* | *54,15* | *7,02* | *12,48* |

*Infrarouge (*ν*_{cm-1}) :*
*2956 ; 2437 ; 2024 ; 1437 ; 1376 ; 1258.*

### EXEMPLE 36 : 1-Benzyl-N-(5-chloro-1H-indol-1-yl)-3-pipéridinecarboxamide

Le produit est obtenu selon le procédé du stade A de l'exemple 18 en utilisant le composé de la préparation 31 à la place du composé de la préparation 15.
*Point de fusion : 197°C*
*Microanalyses élémentaires :*

| | *C %* | *H %* | *N %* |
|---|---|---|---|
| *Calculé :* | *68,56* | *6,03* | *11,42* |
| *Trouvé :* | *68,45* | *6, 25* | *11,31* |

### EXEMPLE 37 : Chlorhydrate de 3-{[(5-chloro-1H-indol-1-yl)amino]carbonyl}-1-méthylpipéridinium

### Stade A : N-(5-chloro-1H-indol-1-yl)-1-méthyl-3-pipéridine carboxamide

Le produit est obtenu selon le procédé du stade A de l'exemple 18 en utilisant le composé de la préparation 12 à la place du composé de la préparation 15.
*Infrarouge (*ν*_{cm-1}) :*
*3117, 3081, 2937, 2464, 1697*

### Stade B : Chlorhydrate de 3-{[(5-chloro-1H-indol-1-yl)amino]carbonyl}-1-méthylpipéridinium

A une solution de 228 mg du composé du stade A précédent dans 6 ml de THF anhydre, on ajoute 6 gouttes de HCl concentré. Le solvant est évaporé puis on lave le résidu avec de l'éther éthylique permettant d'obtenir 256 mg du produit attendu.
*Point de fusion :* > *250°C*
*Microanalyses élémentaires :*

| | *C %* | *H %* | *N %* |
|---|---|---|---|
| *Calculé :* | *54,89* | *5,83* | *12,80* |
| *Trouvé :* | *54,87* | *5,98* | *12,83* |

*Spectrométrie de masse (ESI* +, *m*/*z):* 292 *(M*+*H*⁺*)*

### EXEMPLE 38 : (3R,4S)-3-(4-Fluorophényl)-N-(1H-indol-1-yl)-1-méthylpipéridine-4-carboxamide

590 mg du composé de la préparation 2 sont dissous dans 6 ml de dichlorométhane anhydre puis après refroidissement à -20°C, 4,1 ml de triméthylaluminium 2M/hexane sont ajoutés. Le mélange réactionnel est agité 1h30 sans contrôle de la température. 930 mg du composé de la préparation 32 dans 4 ml de dichlorométhane anhydre sont ajoutés puis la solution est agitée 18h au reflux du dichlorométhane. La solution est diluée par du dichlorométhane puis versée dans une solution aqueuse de soude à 20%. La phase organique est extraite puis lavée par une solution aqueuse de soude à 20% et par une solution saturée en NaCl. La phase organique est séchée sur sulfate de sodium, filtrée puis évaporée sous pression réduite. Le résidu est purifié par chromatographie éclair sur gel de silice (CH₂Cl₂/MeOH : 97/3 puis 95/5 et 9/1 ). Le produit attendu est obtenu par précipitation en présence d'éther.
*Point de fusion : 134°C*
*Spectrométrie de masse (ESI* +, *m*/*z): 352,1 (M*+*H*⁺*)*

### EXEMPLE 39 : (3R,4R)-3-(4-Fluorophényl)-N-(1H-indol-1-yl)-1-méthylpipéridine-4-carboxamide

Le produit attendu est obtenu lors de la purification du composé de l'exemple 38.
*Point de fusion : 205°C*
*Microanalyses élémentaires :*

| | *C %* | *H %* | *N %* |
|---|---|---|---|
| *Calculé :* | *71,77* | *6,31* | *11,96* |
| *Trouvé :* | *71,05* | *6,37* | *11,86* |

*Spectrométrie de masse (ESI* +, *m*/*z): 352,1 (M*+*H*⁺*); 374,2 (M*+*Na*⁺*)*

### EXEMPLE 40 : 4-(2-{3-[(1H-Indol-1-ylamino)carbonyl]-1-pipéridinyl}éthyl)-pipérazine-1-carboxylate de tert-butyle

Le produit est obtenu selon le procédé du stade A de l'exemple 18 en utilisant le composé de la préparation 2 à la place du composé de la préparation 3 et le composé de la préparation 33 à la place du composé de la préparation 15.
*Point de fusion : 48°C*
*Microanalyses élémentaires :*

| | *C %* | *H %* | *N %* |
|---|---|---|---|
| *Calculé :* | *65,91* | *8,19* | *15,37* |
| *Trouvé :* | *65,34* | *8,21* | *15,00* |

### EXEMPLE 41 : Dichlorhydrate de 1-[3-(diméthylammonium)propyl]-3-[(1H-indol-1-ylamino)carbonyl]pipéridinium

Le produit est obtenu selon le procédé de l'exemple 37 en utilisant le composé de la préparation 2 à la place du composé de la préparation 3 et le composé de la préparation 34 à la place du composé de la préparation 12.
*Point de fusion : > 250°C*
*Microanalyses élémentaires :*

| | *C %* | *H %* | *N %* |
|---|---|---|---|
| *Calculé :* | *54,41* | *7,69* | *13,36* |
| *Trouvé :* | *54,45* | *7,71* | *13,41* |

### EXEMPLE 42 : N-(1H-indol-1-yl)-1-[3-(1-pipéridinyl)propyl]-3-pipéridine carboxamide

Le produit est obtenu selon le procédé du stade A de l'exemple 18 en utilisant le composé de la préparation 2 à la place du composé de la préparation 3 et le composé de la préparation 38 à la place du composé de la préparation 15.
*Point de fusion : 95°C*
*Microanalyses élémentaires :*

| | *C %* | *H %* | *N %* |
|---|---|---|---|
| *Calculé _{:}* | *68,36* | *8,86* | *14,44* |
| *Trouvé :* | *68,30* | *8,94* | *14,24* |

### EXEMPLE 43 : N-(1H-indol-1-yl)-1-[3-(4-méthyl-1-pipérazinyl)propyl]-3-pipéridine carboxamide

Le produit est obtenu selon le procédé du stade A de l'exemple 18 en utilisant le composé de la préparation 2 à la place du composé de la préparation 3 et le composé de la préparation 39 à la place du composé de la préparation 15.
*Microanalyses élémentaires :*

| | *C %* | *H %* | *N %* |
|---|---|---|---|
| *Calculé :* | *68,90* | *8,67* | *18,00* |
| *Trouvé :* | *68,60* | *8,71* | *18,15* |

### EXEMPLE 44 : N-(5-chloro-1H-indol-1-yl)-1-(2-hydroxyéthyl)-3-pipéridine carboxamide

### Stade A : 1-(2-{[tert-Butyl(diméthyl)silyl]oxy}éthyl)-N-(5-chloro-1H-indol-1-yl)-3-pipéridinecarboxamide

Le produit est obtenu selon le procédé du stade A de l'exemple 18 en utilisant le composé de la préparation 40 à la place du composé de la préparation 15.
*Infrarouge (*ν*_{cm-1}) :*
*3225 (*ν *N-H); 2930; 2856 (*ν *C-H); 1677 (*ν *C=O); 1517 (*ν *C=C).*

### Stade B : N-(5-chloro-1H-indol-1-yl)-1-(2-hydroxyéthyl)-3-pipéridinecarboxamide

Le produit est obtenu selon le procédé du stade B de l'exemple 18 en utilisant le composé du stade A précédent.
*Point de fusion : 125°C*
*Microanalyses élémentaires :*

| | *C %* | *H %* | *N %* |
|---|---|---|---|
| *Calculé :* | *59,72* | *6,55* | *13,06* |
| *Trouvé :* | *59, 02* | *6,26* | *12,79* |

*Spectrométrie de masse (ESI* +*, m*/*z): 322,1; 324,1(M*+*H*⁺*); 344,1; 346,1 (M*+*Na*⁺*).*

### EXEMPLE 45 : 1-(3-Hydroxypropyl)-N-(1H-indol-1-yl)-3-pipéridinecarboxamide

### Stade A : 1-(3-{[tert-Butyl(diméthyl)silyl]oxy}propyl)-N-(1H-indol-1-yl)-3-pipéridine carboxamide

Le produit est obtenu selon le procédé du stade A de l'exemple 18 en utilisant le composé de la préparation 2 à la place du composé de la préparation 3 et le composé de la préparation 41 à la place du composé de la préparation 15.
*Point de fusion : 94°C*
*Infrarouge (*ν*_{cm-1}) :*
2951; 2927; *2855 (*ν *C-H); 1706 (*ν *C=O); 1614; 1584 (*ν *C=C).*

### Stade B : 1-(3-Hydroxypropyl)-N-(1H-indol-1-yl)-3-pipéridinecarboxamide

Le produit est obtenu selon le procédé du stade B de l'exemple 18 en utilisant le composé du stade A précédent.
*Point de fusion : 51°C*
*Microanalyses élémentaires :*

| | *C %* | *H %* | *N %* |
|---|---|---|---|
| *Calculé :* | *67,75* | *7,69* | *13,94* |
| *Trouvé :* | *67,18* | *7,82* | *13,49* |

### EXEMPLE 46 : N-(Indol-1-yl)-1-[2-(4-méthylpipérazin-1-yl)éthyl]-1,2,5,6-tétrahydropyridine-3- carboxamide

Le produit est obtenu selon le procédé de l'exemple 3 en utilisant le composé de la préparation 43 à la place du composé de la préparation 7.

Une chromatographie éclair sur gel de silice (CH₂Cl₂/CH₃OH : 95/5, puis 9/1 et 8/2) permet d'isoler 380 mg du produit attendu.
*Point de fusion : 130°C*
*Microanalyses élémentaires :*

| | *C %* | *H %* | *N %* |
|---|---|---|---|
| *Calculé :* | *68,63* | *7,95* | *19,06* |
| *Trouvé :* | *68,49* | *8,09* | *18,93* |

*Infrarouge (*ν*_{cm-1}) :*
*3054 (*ν *=C-H) ; 2935 (*ν *C-H) ; 2795 (*ν *N-CH₃) ; 1681 (*ν *C=O) ; 1646 ; 1614 (*ν *C=C) ; 1521 (*δ *N-H) ; 1458 (*δ *C-H) ; 1372 (*ν *C-N).*

### EXEMPLE 47 : N-(5-Chloroindol-1-yl)-1-[2-(4-méthylpipérazin-1-yl)éthyl]-1,2,5,6-tétrahydropyridine-3-carboxamide.

À une solution de 782 mg du composé de la préparation 3 dans 12 ml de dichlorométhane anhydre, préalablement refroidie à -20 °C, sont ajoutés 4,30 ml d'une solution 2M de triméthylaluminium dans l'hexane. La solution est agitée pendant 1 h 30 sous azote en laissant remonter progressivement la température vers la température ambiante. 1,05 g d'une solution du composé de la préparation 43 dans 8 ml de dichlorométhane anhydre, est alors ajouté. Le mélange est chauffé à reflux sous azote pendant 16 heures. Au mélange réactionnel refroidi à 0 °C, sont ajoutés 100 ml d'une solution aqueuse de HCl 1M (addition lente au début), puis 250 ml d'eau. Une première extraction par 3 x 100 ml de dichlorométhane élimine l'excès de N-amino-5-chloroindole. La phase aqueuse est alcalinisée avec une solution saturée de carbonate de sodium jusqu'à pH 10 et extraite avec 3 x 100 ml de dichlorométhane. La phase organique est séchée sur sulfate de sodium, filtrée et concentrée sous pression réduite. Une chromatographie sur colonne de silice (NH₄OH/MeOH/CH₂Cl₂ : 1/1/98 jusqu'à 2/18/80) suivie d'une recristallisation dans 15 ml d'un mélange *i*PrOH/AcOEt (1/2) permet d'obtenir 470 mg du produit attendu.
*Point de fusion : 157°C*
*Microanalyses élémentaires :*

| | *C %* | *H %* | *N %* |
|---|---|---|---|
| *Calculé :* | *62,75* | *7,02* | *17,42* |
| *Trouvé :* | *62,50* | *6,92* | *17,23* |

*Spectrométrie de masse (ESI* +*, mlz) : 402 (M*+*1).*
*Infrarouge (*ν*_{cm-1}) :*
*2946 à 2814 (*ν *CH) ; 1681 (*ν *CO) ; 1650 ; 1531 ; 1465.*

### EXEMPLE 48 : N-(5-Méthoxyindol-1-yl)-1-[2-(4-méthylpipérazin-1-yl)éthyl]-1,2,5,6-tétrahydropyridine-3-carboxamide

Le produit est obtenu selon le procédé de l'exemple 47 en utilisant le composé de la préparation 10 à la place du composé de la préparation 3.

Une chromatographie sur colonne de silice (NH₄OH/MeOH/CH₂Cl₂ : 1/1/98 jusqu'à 2/18/80) suivie d'une trituration pendant 15 minutes dans 15 ml d'éther diéthylique et suivie d'une cristallisation dans 10 ml d'un mélange AcOEt/Et₂O (1/2) permet d'obtenir 820 mg du produit attendu.
*Point de fusion : 121 °C*
*Microanalyses élémentaires :*

| | *C %* | *H %* | *N %* |
|---|---|---|---|
| *Calculé :* | *66,47* | *7,86* | *17,62* |
| *Trouvé :* | *65,98* | *8,07* | *17,33* |

*Spectrométrie de masse (ESI +, m*/*z) : 398 (M*+*1).*
*Infrarouge (*ν*_{cm-1}) :*
*3216 (*ν *NH); 2938 à 2788 (*ν *CH); 1669 (*ν *CO); 1636; 1514; 1477.*

### EXEMPLE 49 : N-(5-Méthoxyindol-1-yl)-1-[2-(4-méthylpipérazin-1-yl)éthyl]-1,2,5,6-tétrahydropyridine-5-carboxamide

Le produit est obtenu lors de la purification du composé de l'exemple 48.
Une recristallisation dans 5 ml d'éther diisopropylique permet d'obtenir 138 mg du produit attendu.
*Point de fusion : 127°C*
*Microanalyses élémentaires :*

| | *C %* | *H %* | *N %* |
|---|---|---|---|
| *Calculé :* | *66,47* | *7,86* | *17,62* |
| *Trouvé :* | *66,41* | *8,02* | *17,41* |

*Spectrmétrie de masse (ESI* +, *mlz) : 398 (M*+*1).*
*Infrarouge (*ν*_{cm-1}) :*
*3102 (*ν *NH);* 2932 *et 2799 (*ν *CH); 1684 (*ν *CO); 1480; 1445.*

### EXEMPLE 50 : N-(5-Méthylindol-1-yl)-1-[2-(4-méthylpipérazin-1-yl)éthyl]-1,2,5,6-tétrahydropyridine-3-carboxamide

Le produit est obtenu selon le procédé de l'exemple 47 en utilisant le composé de la préparation 44 à la place du composé de la préparation 3.

Une chromatographie sur colonne de silice (NH₄OH/MeOH/CH₂Cl₂ : 1/1/98 jusqu'à 2/18/80) suivie d'une trituration pendant 15 minutes dans 15 ml d'éther diéthylique permet d'obtenir 1,16 g du produit attendu.
*Point de fusion : 140°C*
*Microanalyses élémentaires :*

| | *C %* | *H %* | *N %* |
|---|---|---|---|
| *Calculé :* | *69, 26* | *8,19* | *18,36* |
| *Trouvé :* | *68,85* | *8,41* | *17,96* |

*Spectrométrie de masse (ESI* +*, mlz) : 382 (M*+*1).*
*Infrarouge (*ν*_{cm-1}) :*
*2945 à 2816 (*ν *CH) ; 1681 (*ν *CO) ; 1651 ; 1533 ; 1466.*

### EXEMPLE 51 : N-(5-Méthylindol-1-yl)-1-[2-(4-méthylpipérazin-1-yl)éthyl]-1,2,5,6-tétrahydropyridine-5-carboxamide

Le produit est obtenu lors de la purification du composé de l'exemple 50.

Une recristallisation dans 5 ml d'éther diisopropylique permet d'obtenir 120 mg du produit attendu.
*Point de fusion : 131 °C*
*Microanalyses élémentaires :*

| | *C %* | *H %* | *N %* |
|---|---|---|---|
| *Calculé :* | *69,26* | *8,19* | *18,36* |
| *Trouvé :* | *69,12* | *8,41* | *18,18* |

*Spectrométrie de masse (ESI* +*, mlz) : 382 (M*+*1).*
*Infrarouge (*ν*_{cm-1}) :*
*3141 (*ν *NH); 2934 à 2 766 (*ν *CH); 1694 (*ν *CO); 1515; 1457.*

### EXEMPLE 52 : N-(Indol-1-yl)-1-[2-(4-(2-hydroxyéthyl)pipérazin-1-yl)éthyl]-1,2,5,6-tétrahydropyridine-3-carboxamide

### Stade A : N-(Indol-1-yl)-1-[2-[4-[2-(tertbutyldiméthylsilanoxy)éthyl]pipérazin-1-yl] éthyl]-1,2,5,6-tétrahydropyridine-3-carboxamide

À une solution de 200 mg du composé de la préapration 2 dans 5 ml de dichlorométhane anhydre, sont ajoutés, après refroidissement à -20 °C, 1,35 ml de triméthylaluminium en solution 2M dans l'hexane. La solution est agitée 1 h 30 sans contrôle de la température, puis 500 mg du composé de la préparation 45 dans 3 ml de dichlorométhane anhydre sont ajoutés à température ambiante. Le mélange est porté au reflux 16 heures et dilué, après refroidissement, par du dichlorométhane et versé sur une solution aqueuse d'hydroxyde de sodium à 20 %. La phase organique est récupérée et la phase aqueuse, extraite par du dichlorométhane. Les phases organiques réunies sont séchées sur sulfate de sodium, filtrées et évaporées sous pression réduite. Une chromatographie éclair sur gel de silice (CH₂Cl₂/CH₃OH : 9/1 puis 8/2) permet d'obtenir 400 mg du produit attendu.
*Inflarouge (*ν*_{cm-1}) :*
*3244 (*ν *N-H) ; 2949 ; 2929 ; 2855 (*ν *C-H) ; 2817 (*ν *N-CH₂) ; 1674 (*ν *C=O) ; 1644 (*ν *C=C) ; 1521 ; 1505 (*δ *N-H) ; 1460 (*δ *C-H) ; 1360 (*ν *C-N) ; 1256 ; 1223 (*ν *C-O).*

### Stade B : N-(Indol-1-yl)-1-[2-[4-(2-hydroxyéthyl)pipérazin-1-yl]éthyl]-1,2,5,6-tétra-hydropyridine-3-carboxamide

À une solution de 300 mg du composé du stade A précédent dans 4 ml de THF anhydre, est ajouté 1,17 ml de fluorure de tétrabutylammonium en solution 1M dans le THF. Le mélange est agité 12 heures à température ambiante, puis la solution est concentrée sous pression réduite. Après reprise du résidu dans le dichlorométhane, la phase organique est extraite par une solution aqueuse d'hydroxyde de sodium ; la phase aqueuse est extraite par du dichlorométhane. Les phases organiques réunies sont séchées sur sulfate de sodium, filtrées et évaporées sous pression réduite. Une chromatographie éclair sur gel de silice (AcOEt/MeOH : 7/3 + 0, 1 % de triéthylamine) permet d'obtenir 180 mg du produit attendu.
*Point de fusion : 113°C*
*Microanalyses élémentaires :*

| | *C %* | *H %* | *N %* |
|---|---|---|---|
| *Calculé :* | *63,59* | *8,00* | *16,85* |
| *Trouvé :* | *64,12* | *8,08* | *16,49* |

*Infrarouge (*ν*_{cm-1}) :*
*3192 (*ν *N-H) ; 2939 (*ν *C-H) ; 2815 (*ν *N-CH₂) ; 1674 (*ν *C=O) ; 1644 (*ν *C=C) ; 1523 (*δ *N-H) ; 1459 (*δ *C-H) 1353 (*ν *C-N) ; 1270 ; 1223 (*ν *C-O).*

### EXEMPLE 53 : N-(Indol-1-yl)-1-(2-pipéridin-1-yl-éthyl)-1,2,5,6-tétrahydropyridine-3-carboxamide

Le produit est obtenu selon le procédé de l'exemple 3 en utilisant le composé de la préparation 46 à la place du composé de la préparation 7.

Une chromatographie éclair sur gel de silice (CH₂Cl₂/CH₃OH : 9/1 puis 8/2) permet d'isoler 240 mg du produit attendu.
*Point de fusion : 65°C*
*Microanalyses élémentaires :*

| | *C %* | *H %* | *N %* |
|---|---|---|---|
| *Calculé :* | *69,78* | *8,09* | *15,50* |
| *Trouvé :* | *69,98* | *8,17* | *15,40* |

*Infrarouge (*ν*_{cm-1}) :*
3238 *(*ν *N-H) ; 2931 (*ν *C-H) ; 2788 (*ν *-CH₂); 1672 (*ν *C=O) ; 1643 (*ν *C=C) ; 1521 (*δ *N-H) ; 1459 (*δ *C-H) ; 1370 (*ν *C-N).*

### EXEMPLE 54 : N-(Indol-1-yl)-1-[2-[3-(éthoxycarbonyl)-1,2,5,6-tétrahydropyridin-1-yl]éthyl]-1,2,5,6-tétrahydropyridine-3-carboxamide

Le produit est obtenu selon le procédé de l'exemple 3 en utilisant le composé de la préparation 47 à la place du composé de la préparation 7.

Une chromatographie éclair sur gel de silice (AcOEt puis AcOEt/CH₃OH : 95/5) permet d'isoler 180 mg du produit attendu.
*Point de fusion : 82°C*
*Microanalyses élémentaires :*

| | *C %* | *H %* | *N %* |
|---|---|---|---|
| *Calculé :* | *67,63* | *6,91* | *13,72* |
| *Trouvé :* | *67,41* | *7,09* | *13,63* |

*Infrarouge (*ν*_{cm-1}) :*
*3265 (*ν *N-H) ; 2948 ; 2915 ; 2802 (*ν *C-H) ; 1708 ; 1673 (*ν *C=O) ; 1646 ; 1614 ; 1519 (*ν *C=C) ; 1459 ; 1436 (*δ *C-H); 1371 ; 1351 (*ν *C-N) ; 1261 ; 1223 ; 1194 (*ν *C-O).*

### EXEMPLE 55 : Trichlorhydrate de (±)-N-(indol-1-yl)-1-[2-[4-[(tétrahydrofuran-2-yl)méthyl]pipérazin-1-yl)]éthyl] pipéridine-3-carboxamidyle

0,49 g du composé de la préparation 48 est dissous dans 9 ml de dichlorométhane anhydre. La solution est refroidie à -20 °C, puis 3,5 ml d'une solution de triméthylaluminium 2M dans l'hexane sont ajoutés sous argon. Après remontée de la température du milieu réactionnel à 0°C en 1 h 30, une solution de 1,1 g du composé de la préparation 48 dans 6 ml de dichlorométhane anhydre, est ajoutée. Le milieu réactionnel est ensuite porté au reflux pendant 18 heures. Après refroidissement, 50 ml de dichlorométhane sont additionnés, puis une solution aqueuse d'hydroxyde de sodium 20 % (p/v) est ajoutée goutte à goutte jusqu'à fin du dégagement de méthane. La phase organique est séparée, lavée successivement avec une solution aqueuse d'hydroxyde de sodium 20 % (p/v), une solution saturée en NaCl, puis séchée sur Na₂SO₄ et concentrée. Une chromatographie sur gel de silice (CH₂Cl₂/MeOH : 9/1 + 0,5 % NEt₃) permet d'obtenir 0,46 g de l'amide attendu. 150 mg de l'amide sont dissous dans 1,0 ml de MeOH anhydre ; à la solution est additionnée 0,6 ml d'une solution HCl méthanolique 2M. Le produit attendu est précipité par ajout de 5 ml d'éther diéthylique, puis séparation par filtration et séchage sous vide.
*Point de fusion : >250°C*
*Microanalyses élémentaires :*

| | *C %* | *H %* | *N %* |
|---|---|---|---|
| *Calculé :* | *54,70* | *7,34* | *12,76* |
| *Trouvé :* | *55,02* | *7,48* | *12,70* |

*Infrarouge (*ν*_{cm-1}) :*
*3385 ; 2975 ; 2411 ; 1694 ; 1531 ; 1458 ; 1325.*

### EXEMPLE 56 : Tétrachlorhydrate de (±)-N-(indol-1-yl)-1-[2-[4-[2-(diméthylamino)éthyl]pipérazin-1-yl)]éthyl]pipéridine-3-carboxylate d'éthyle

Le produit est obtenu selon le procédé de l'exemple 55 en utilisant le composé de la préparation 49 à la place du composé de la préparation 48.

Une chromatographie sur gel de silice (CH₂Cl₂%MeOH : 9/1 à 7/3 + 0,5 % NEt₃) permet d'obtenir 0,59 g de l'amide attendu. 130 mg de l'amide sont dissous dans 0,5 ml de MeOH anhydre ; à la solution est ajouté 0,7 ml d'une solution HCl méthanolique 2M. Le produit attendu est obtenu par précipitation par ajout de 5 ml d'éther diéthylique, séparation par filtration puis séchage sous vide.
*Point de fusion : >250°C*
*Microanalyses élémentaires :*

| | *C %* | *H %* | *N %* |
|---|---|---|---|
| *Calculé :* | *50,36* | *7,40* | *14,68* |
| *Trouvé :* | *50,15* | *7,48* | *14,40* |

*Infrarouge (*ν*_{cm-1}) :*
*2938 ; 2815 ; 1689 ; 1460 ; 1396 ; 1315.*

### EXEMPLE 57 : Trichlorhydrate de (±)-N-(indol-1-yl)-1-[2-[4-(2-méthoxyéthyl) pipérazin-1-yl)]éthyl]pipéridine-3-carboxamide

Le produit est obtenu selon le procédé de l'exemple 55 en utilisant le composé de la préparation 50 à la place du composé de la préparation 48.

Une chromatographie sur gel de silice (CH₂Cl₂/MeOH : 90/10) permet d'obtenir 0,22 g de l'amide attendu. 200 mg de l'amide sont dissous dans 1,0 ml de MeOH anhydre ; à la solution est ajouté 0,8 ml d'une solution HCl méthanolique 2M. Le produit attendu est obtenu par précipitation par ajout de 5 ml d'éther diéthylique, séparation par filtration puis séchage sous vide.
*Point de fusion : >250°C*
*Microanalyses élémentaires :*

| | *C %* | *H %* | *N %* |
|---|---|---|---|
| *Calculé :* | *52,83* | *7,32* | *13,39* |
| *Trouvé :* | *52,74* | *7,39* | *13,32* |

*Infrarouge (*ν*_{cm-1}) :*
*3381 ; 2981 ; 2503 ; 2299 ; 1689 ; 1656 ; 1449 ; 1325.*

### EXEMPLE 58 : Tétrachlorhydrate de (±)-N-(indol-1-yl)-1-[2-[4-(1-méthylpipéridin-4-yl)pipérazin-1-yl)]éthyl]pipéridine-3-carboxamide

Le produit est obtenu selon le procédé de l'exemple 55 en utilisant le composé de la préparation 51 à la place du composé de la préparation 48.

Une chromatographie sur gel de silice (CH₂Cl₂/MeOH : 8/2 + 0,5 % de NEt₃) permet d'obtenir 0,51 g de l'amide attendu. 227 mg de l'amide sont dissous dans 1,0 ml de MeOH anhydre ; à la solution est ajouté 1,1 ml d'une solution HCl méthanolique 2M. Le produit attendu est obtenu par précipitation par ajout de 5 ml d'éther diéthylique, séparation par filtration puis séchage sous vide.
*Point de fusion : >250°C*
*Microanalyses élémentaires :*

| | *C %* | *H %* | *N %* |
|---|---|---|---|
| *Calculé :* | *52,18* | *7,41* | *14,04* |
| *Trouvé :* | *52,03* | *7,41* | *13,94* |

*Infrarouge (*ν*_{cm-1}) :*
*3502 ; 3126 ; 2980 ; 2410 ; 1696 (*ν*C=O amide) ; 1543 ; 1473 ; 1458.*

### EXEMPLE 59 : Trichlorhydrate de (±)-N-(indol-1-yl)-1-[3-[4-(2-hydroxyéthyl) pipérazin-1-yl)]propyl]pipéridine-3-carboxamide

### Stade A : (±)-N-(Indol-1-yl)-1-[3-[4-(2-tert-butyldiméthylsilyloxyéthyl)pipérazin-1-yl)] propyl]pipéridine-3-carboxamide

Le produit est obtenu selon le procédé de l'exemple 55 en utilisant le composé de la préparation 52 à la place du composé de la préparation 48.

Une chromatographie sur gel de silice (CH₂Cl₂/MeOH : 9/1 à 8/2 + 0,5 % de NEt₃) permet d'obtenir 0,89 g du produit attendu.
*Infrarouge (*ν*_{cm-1}) :*
*2934 ; 2856 ; 2810 ; 1682 ; 1460 ; 1361 ; 1253, 1155 ; 1099.*

### Stade B : Trichlorhydrate de (±)-N-(indol-1-yl)-1-[3-[4-(2-hydroxyéthyl)pipérazin-1-yl)]propyl]pipéridine-3-carboxamide

0,35 g du produit du stade A précédent est dissous dans 5 ml d'éthanol à 95 %. 500 µl d'une solution d'HCl 12 M sont ajoutés à température ambiante. La solution est ensuite chauffée à 60 °C pendant 2 heures. Après refroidissement à température ambiante, la suspension est filtrée sur verre fritté ; le solide obtenu est lavé plusieurs fois avec l'éthanol et séché permettant d'obtenir 0,29 g du produit attendu.
*Point de fusion : >250°C*
*Microanalyses élémentaires :*

| | *C %* | *H %* | *N %* |
|---|---|---|---|
| *Calculé :* | *53,83* | *7,32* | *13,39* |
| *Trouvé :* | *53,37* | *7,41* | *13,20* |

*Infrarouge (*ν*_{cm-1}) :*
*3266 ; 3155 ; 2978 ; 2688 ; 2503 ; 2436 ; 1713 ; 1515 ; 1499 ; 1460.*

### EXEMPLE 60 : Trichlorhydrate de (±)-N-(indol-1-yl)1-[4-(4-méthylpipérazin-1-yl) butyl]pipéridine-3-carboxamide

### Stade A : Bromure de 3-[N-(indol-1-yl)aminocarbonyl]1-[4-(4-méthylpipérazin-1-yl) butyl]pyridinium

0,6 g du composé de la préparation 53 et 500 µl de N-méthylpipérazine sont dissous dans 8 ml d'éthanol à 70 %. Le milieu réactionnel est agité à 50 °C pendant 18 heures. 1 ml supplémentaire de N-méthylpipérazine est ajouté en 2 fois au cours des 24 heures suivantes dans les mêmes conditions. Les solvants et l'excès de N-méthylpipérazine sont éliminés par distillation à l'évaporateur rotatif. Le résidu brut est engagé dans l'étape suivante sans autre purification.

### Stade B : N-(Indol-1-yl)1-[4-(4-méthylpipérazin-1-yl)butyl]-1,4,5,6-tétrahydropyridine - 3-carboxamide

Le composé du stade A précédent est dissous dans 9 ml d'un mélange acétate d'éthyle/éthanol (6:3); 100 mg de palladium sur charbon (10 %) sont ajoutés. La suspension, sous agitation, est dégazée sous vide, puis placée sous atmosphère d'hydrogène. Le milieu réactionnel est maintenu 18 heures sous atmosphère d'hydrogène, ensuite purgé et placé sous atmosphère d'azote. Le mélange réactionnel est filtré sur cellite et le filtrat concentré. Une chromatographie sur gel de silice (CH₂Cl₂MeOH : 9/1 à 8/2 + 0,5 % NEt₃) permet d'obtenir 0,29 g du produit attendu.
*Infrarouge (*ν*_{cm-1}) :*
*2933 ; 1615 ; 1574 ; 1497 ; 1355 ; 1293 ; 1181.*

### Stade C: Trichlorhydrate de (±)-N-(indo/-1-y/)1-[4-(4-méthy/pipérazin-1-y/) butyl]pipéridine-3-carboxamide

À une solution de 295 mg du composé du stade B précédent dans 8 ml d'un mélange acide acétique/méthanol (6:2), sont ajoutés par petites portions et à température ambiante, 200 mg de cyanoborohydrure de sodium. La réaction est agitée 3 heures à température ambiante. 20 ml d'une solution d'hydroxyde de sodium 10 % et 20 ml de dichlorométhane sont ensuite ajoutés. La phase organique est lavée 3 fois par une solution d'hydroxyde de sodium 10 %, séchée sur sulfate de sodium, filtrée et évaporée sous pression réduite. Une chromatographie sur gel de silice (CH₂Cl₂/MeOH : 8/2 + 0,5 % NEt₃)permet d'obtenir 0,14 g de l'amide. 100 mg de l'amide sont dissous dans 0,5 ml de MeOH anhydre ; à la solution, est ajouté 0,75 ml d'une solution HCl méthanolique 2M. Le produit attendu est obtenu par précipitation par ajout de 5 ml d'éther diéthylique, séparation par filtration puis séchage sous vide.
*Point de fusion : >250°C*
*Microanalyses élémentaires :*

| | *C %* | *H %* | *N %* |
|---|---|---|---|
| *Calculé :* | *54,49* | *7,56* | *13,82* |
| *Trouvé :* | *54,60* | *7,80* | *13,61* |

*Infrarouge (*ν*_{cm-1}) :*
*3382 ; 2948 ; 2401* ; *1687 ; 1528 ; 1458 ; 1315.*

### EXEMPLE 61 : Trichlorhydrate de (±)-N-(indol-1-yl)-1-[4-[4-(2-hydroxyéthyl) pipérazin-1-yl)]butyl]pipéridine-3-carboxamide

### Stade A : Bromure de 3-[N-(indol-1-yl)aminocarbonyl]1-[4-([4-(2-hydroxyéthyl) pipérazin-1-yl)butyl]pyridinium

Le produit est obtenu selon le procédé du stade A de l'exemple 60 en utilisant le composé de la 1-(2-hydroxyéthyl)pipérazine à la place de la N-méthylpipérazine.

### Stade B : N-(Indol-1-yl)-1-[4-[4-(2-hydroxyéthyl)pipérazin-1-yl)]butyl]-1,4,5,6-tétrahydropyridine-3-carboxamide

Le produit est obtenu selon le procédé du stade B de l'exemple 60 en utilisant le composé du stade A précédent.

Une chromatographie sur gel de silice (CH₂Cl₂MeOH : 8/2 à 7/3 + 0,5 % NEt₃) permet d'obtenir 0,12 g du produit attendu.

### Stade C: Trichlorhydrate de (±)-N-(indol-1-yl)-1-[4-[4-(2-hydroxyéthyl)pipérazin-1-yl)]butyl]pipéridine-3-carboxamide

Le produit est obtenu selon le procédé du stade C de l'exemple 60 en utilisant le composé du stade B précédent.
*Point de fusion : >250°C*
*Microanalyses élémentaires :*

| | *C %* | *H %* | *N %* |
|---|---|---|---|
| *Calculé :* | *53,68* | *7,51* | *13,04* |
| *Trouvé :* | *53,42* | *7,61* | *12,89* |

### EXEMPLE 62 : Trichlorhydrate de (±)-N-(indol-1-yl)-1-[2-(4-butylpipérazin-1-yl)] éthyl]pipéridine-3-carboxamide

Le produit est obtenu selon le procédé de l'exemple 55 en utilisant le composé de la préparation 54 à la place du composé de la préparation 48.

Une chromatographie sur gel de silice (CH₂Cl₂/MeOH : 9/1 + 0,5 % NEt₃) permet d'obtenir 0,82 g de l'amide attendu. 600 mg de l'amide sont dissous dans 2,0 ml de MeOH anhydre ; à la solution est ajouté 2,3 ml d'une solution HCl méthanolique 2M. Le produit attendu est obtenu par précipitation par ajout de 10 ml d'éther diéthylique, séparation par filtration puis séchage sous vide.
*Point de fusion : >250°C*
*Microanalyses élémentaires :*

| | *C %* | *H %* | *N %* |
|---|---|---|---|
| *Calculé :* | *55,33* | *7,74* | *13,44* |
| *Trouvé :* | *55,12* | *7,91* | *13,26* |

*Infrarouge* (ν*_{cm-1}*) :
*3371 ; 2959 ; 1686 ; 1535 ; 1459 ; 1372 ; 1314.*

### EXEMPLE 63 : (±)-N-(Indol-1-yl)-1-allylpipéridine-3-carboxamide

Le produit est obtenu selon le procédé de l'exemple 3 en utilisant le compose de la préparation 11 à la place du compose de la préparation 7.

Une chromatographie éclair sur gel de silice (AcOEt) permet d'obtenir 930 mg du produit attendu.
*Point de fusion : 92°C*
*Microanalyses élémentaires :*

| | *C %* | *H %* | *N %* |
|---|---|---|---|
| *Calculé :* | *72,06* | *7,47* | *14,83* |
| *Trouvé :* | *71,70* | *7,73* | *14,61* |

*Infrarouge (*ν*_{cm-1}) :*
*3198 (*ν *N-H) ; 3032 (*ν *=C-H) 2940 (*ν *C-H) ;* 2792 *(*ν *N-CH₂) ; 1687 (*ν *C=C) ; 1667 (*ν *C=O) ; 1615 (*ν *C=C) ; 1542 (*δ*N-H) 1462 ; 1421 (*δ*C-H) ; 1378 ; 1362 (*ν*C-N).*

### EXEMPLE 64 : (±)-N-(Indol-1-yl)-1-(prop-2-ynyl)pipéridine-3-carboxamide

Le produit est obtenu selon le procédé de l'exemple 3 en utilisant le compose de la préparation 55 à la place du compose de la préparation 7.

Une chromatographie éclair sur gel de silice (cyclohexane/AcOEt : 2/8 puis AcOEt) permet d'obtenir 550 mg du produit attendu.
*Point de fusion : 154°C*
*Microanalyses élémentaires :*

| | *C %* | *H %* | *N %* |
|---|---|---|---|
| *Calculé :* | *72,57* | *6,81* | *14,94* |
| *Trouvé :* | *72,38* | *6,97* | *14,78* |

*Infrarouge (*ν*_{cm-1}) :*
3232 *(*ν *≡C-H) 3050 (*ν *=C-H) ; 2946 ; 2913 (*ν *C-H) ; 2118 (*ν *C≡C) ; 1698 (*ν *C=O) ; 1614 (*ν *C=C) ; 1569 (*δ *N-H); 1475 ; 1458 (*δ *C-H) ; 1368 ; 1340 (*ν *C-N).*

### EXEMPLE 65 : (±)-N-(Indol-1-yl)-1-[4-(pipéridin-1-l)but-2-èn-1-yl]-pipéridine-3-carboxamide

À une solution de 140 mg du composé de la préparation 2 dans 4 ml de dichlorométhane anhydre, refroidie à -20 °C, est ajouté 0,97 ml de triméthylaluminium 2M dans l'hexane. Après 1 heure 30 sans contrôle de la température, une solution de 260 mg du composé de la préparation 56 dans 2 ml de dichlorométhane anhydre est ajoutée à température ambiante. Le milieu réactionnel est porté 12 heures au reflux, additionné de dichlorométhane, puis versé sur une solution aqueuse d'hydroxyde de sodium à 20 % (p/v). La phase organique est séparée et la phase aqueuse extraite par du dichlorométhane. Les phases organiques réunies sont séchées sur sulfate de sodium, filtrées, puis évaporées sous pression réduite. Une chromatographie éclair sur gel de silice (CH₂Cl₂/CH₃OH : 9:1 puis 8/2) permet d'obtenir 310 mg d'une fraction impure du composé attendu. Le solide est dissous dans 6 ml d'un mélange THF/H₂O (2:1). 50 mg de LiOH.H₂O sont ajoutés à température ambiante ; la suspension est agitée 12 heures. Le milieu réactionnel est additionné de dichlorométhane et d'eau. La phase organique est séparée et la phase aqueuse extraite par du dichlorométhane. Les phases organiques réunies sont séchées sur sulfate de sodium, filtrées et évaporées sous pression réduite. Une chromatographie éclair sur gel de silice (CH₂Cl₂CH₃OH :9/1 puis 8/2) permet d'obtenir 180 mg du produit attendu.
*Point de fusion : 116°C*
*Microanalyses élémentaires :*

| | *C %* | *H %* | *N %* |
|---|---|---|---|
| *Calculé :* | *70,92* | *8,54* | *14,38* |
| *Trouvé :* | *71,15* | *8,50* | *14,30* |

*Infrarouge (*ν*_{cm-1}) :*
*3166 (*ν *N-H) ;* 2999 ; *2934 ; 2854 (*ν *C-H) ; 2798 ; 2754 (*ν *N-CH₂) ; 1667 (*ν *C=O) ; 1539 (*δ *N-H) ; 1459 (*δ *C-H) ; 1367 (*ν *C-N).*

### EXEMPLE 66 : (R ou S) (-)-N-(Indol-1-yl)-1-[2-(pipéridin-1-yl)éthyl]pipéridine-3-carboxamide énantiomère 1

Ce composé est obtenu par séparation des deux énantiomères du composé de l'exemple 24 réalisée par CLHP chirale semi-préparative (Daicel Chiralpak AD, 1x25 cm) dans les conditions suivantes : éluant : hexane (+ 0,1% TEA)/EtOH (+ 0, 1 % TEA): 96/4 ; débit: 4,7 ml/min.
*Temps de rétention : 17 min ; pureté 100 % ;*
*[*α*]_{D}²⁹ = - 15° (c = 1,0 ; MeOH).*

### EXEMPLE 67 : (R ou S) (+)-N-(Indol-1-yl)-1-[2-(pipéridin-1-yl)éthyl]pipéridine-3-carboxamide énantiomère 2

Ce composé est obtenu par séparation des deux énantiomères du composé de l'exemple 24 réalisée par CLHP chirale semi-préparative (Daicel Chiralpak AD, 1x25 cm) dans les conditions suivantes : éluant : hexane (+ 0,1% TEA)/EtOH (+ 0,1% TEA): 96/4 ; débit: 4,7 ml/min.
*Temps de rétention : 13 min ; pureté 100 % ;*
*[*α*]_{D}²⁹* = *13° (c = 1,0 ; MeOH).*

### ETUDE PHARMACOLOGIQUE DES DERIVES DE L'INVENTION

### EXEMPLE A : Induction de tyrosine hydroxylase

Il est recherché, parmi les dérivés, ceux qui sont capables d'induire une augmentation de la protéine tyrosine hydroxylase (TH) dans le cerveau de la souris Balb/C, au niveau du locus coeruleus (LC), de la substance noire (SN) et de l'aire tegmentale ventrale (ATV).
Les animaux utilisés sont des souris consanguines mâles de la lignée pure Balb/C (Charles River Laboratories), âgées de 6 semaines au moment du traitement.

Les souris reçoivent une injection unique par voie intra péritonéale du composé à tester, dissous dans une solution d'HCl 0.04 M (contrôle correspondant : HCl 0.04 M) si le composé est suffisamment soluble, ou dans l'huile d'olive 90 % / DMSO 10 % (contrôle correspondant : huile d'olive 90 % / DMSO 10 %) pour les composés insolubles en milieu aqueux. Le volume injecté est de 100 µl. Les groupes expérimentaux comprennent 10 animaux.

Trois jours après l'injection de chaque molécule, tous les animaux sont sacrifiés par décapitation. Les cerveaux extraits sont ensuite congelés dans une solution d'isopentane à -30° C pendant 45 secondes, puis stockés à -80° C.

Des coupes coronales sériées de 20 microns d'épaisseur sont ensuite pratiquées sur un cryomicrotome.. Chaque structure d'intérêt est ainsi échantillonnée le long de l'axe postéro-antérieur du cerveau en prélevant des coupes espacées de 80 microns dans le cas du LC et de 80 ou 160 microns dans le cas de l'ATV et de la SN. Les limites de ces structures sont celles décrites dans l'Atlas de Franklin et Paxinos. Les protéines totales de chacune de ces coupes sont transférées par recueil direct des coupes sur un filtre de nitrocellulose (Millipore). Les quantités de TH présentes dans chaque échantillon sont mesurées par immunochimie, détection fluorimétrique et analyse d'image.

### Résultats :

Les résultats d'induction de la TH au niveau du LC sont présentés dans le tableau I suivant.

**TABLEAU I**

| **Mesure de la quantité de TH au niveau des différentes coupes du LC numérotées de 1 à 11 dans le sens postérieur vers antérieur, après administration i.p. (20 mg/kg)** *Les résultats sont exprimés en % par rapport à la valeur moyenne du groupe témoin¹* (6 ≤ n ≤ 9) | | | | | | | | | | | | |
|---|---|---|---|---|---|---|---|---|---|---|---|---|
| % | 1 | 2 | 3 | 4 | 5 | 6 | 7 | 8 | 9 | 10 | 11 | LC total |
| Ex. 1 | 57 | 72 | 128 | 128 | 158* | 152* | 131 | 107 | 106 | 95 | 55 | 121 |
| Ex. 7 | 113 | 139* | 112 | 119 | 138** | 122 | 141** | 131* | 96 | 125 | 106 | 123** |
| Ex. 12 | 57 | 70 | 80 | 110 | 114 | 92 | 95 | 82 | 98 | 94 | 26 | 90 |
| Ex. 22 | 79 | 101 | 112 | 98 | 108 | 113 | 146** | 120 | 135* | 179** | 145* | 119** |
| Ex. 24 | 165* | 157* | 120 | 127 | 145* | 131** | 139* | 116 | 139 | 96 | 94 | 129** |
| Ex. 26 | 152* | 98 | 136** | 107 | 154** | 133* | 127* | 96 | 132 | 110 | 89 | 121** |
| Ex.47 | 232** | 138* | 176** | 179** | 172** | 169** | 153** | 140** | 84* | 88 | 127 | 147** |

| | | | | | | | | | | | | |
|---|---|---|---|---|---|---|---|---|---|---|---|---|
| * 0,01 < p < 0,05 - ** p < 0,01 ¹animaux traités par le même véhicule | | | | | | | | | | | | |

Les résultats d'induction de la TH au niveau de la SN et de l'ATV sont présentés dans le tableau II. Ils sont exprimés en % par rapport à la valeur moyenne du groupe témoin (6 ≤ n ≤ 9).

**TABLEAU II**

| | SN | ATV |
|---|---|---|
| Ex. 1 | 155** | 125 |
| Ex. 12 | 122* | 95 |
| Ex. 15 | 91 | 88* |
| Ex. 7 | 88* | 94 |

| | | |
|---|---|---|
| * 0,01.< p < 0,05 - ** p < 0,01 | | |

Le composé de l'exemple 1 induit de manière importante la TH dans le LC médian (coupes 4, 5 et 6) mais également dans l'ATV et la SN.
D'autres composés induisent la TH dans la SN sans augmenter l'expression de la protéine dans les LC et L'ATV (exemple 12).
D'autres n'induisent la TH que dans le LC (exemple 7).

### EXEMPLE B : Affinité pour les récepteurs

L'affinité pour les récepteurs est déterminée selon les méthodes habituelles de relation entre le ligand spécifique et le récepteur, qui peut être d'origine animale ou un recombinant humain. Elle a été déterminée par la méthode du déplacement du ligand spécifique marquée par le composé étudié et exprimé par la constante de dissociation KI.
L'affinité réceptorielle pour 28 récepteurs classiques a ainsi été étudiée. L'étude montre que l'induction de TH observée ne passe pas par une affinité vis-à-vis des récepteurs habituellement touchés par les produits psychotropes, comme les récepteurs adrénergiques alpha (de type α₂), 5HT (de type 5HT2A), dopaminergiques (de type D₁ et D₂).
Quelques dérivés montrent une affinité non négligeable pour les récepteurs sigma (σ) (ligand halopéridol) ou muscarinique (M).

**TABLEAU III**

| % d'inhibition concentration (M) | α₂ | | 5HT2A | | D₁ | | D₂ | | M | | σ | |
|---|---|---|---|---|---|---|---|---|---|---|---|---|
| | 10⁻⁷ | 10⁻⁵ | 10⁻⁷ | 10⁻⁵ | 10⁻⁷ | 10⁻⁵ | 10⁻⁷ | 10⁻⁵ | 10⁻⁷ | 10⁻⁵ | 10⁻⁷ | 10⁻⁵ |
| Exemple 1 | - | - | - | - | - | - | - | - | - | 23 | - | 44 |
| Exemple 7 | - | - | - | - | - | - | - | - | - | - | - | - |
| Exemple 12 | - | - | - | - | - | - | - | - | - | 51 | 18 | 85 |
| Exemple 25 | - | - | 12 | 14 | - | - | - | - | - | 27 | 17 | 100 |
| Exemple 47 | - | - | 10 | 15 | - | - | - | - | 19 | 10 | - | 67 |

### EXEMPLE C : Prédiction de passage de la barrière hémato-encéphalique (BHE)

Les substances sont incubées à 20 µM dans le compartiment supérieur d'une double cuve séparé du compartiment inférieur soit par un filtre de polycarbonate seul, soit par le même filtre recouvert de cellules endotahéliales confluentes de capillaires bovins. L'évaluation de la cinétique de perméabilité est réalisée par quantification LC-MS-MS de la substance inchangée dans le compartiment inférieur après 10, 20, 30, 40 et 60 minutes.
Les dérivés testés présentent un passage généralement élevé de la BHE favorisant la cible neurologique. Les résultats sont rendus sous forme de classes : élevé, intermédiaire, faible. Ainsi, les exemples 1, 7 et 24 présentent un passage élevé de la BHE.

### EXEMPLE D : Prédiction de stabilité métabolique

La prédiction de stabilité métabolique est testée par incubation des dérivés à la concentration de 10⁻⁷ M en présence de microsomes hépatiques de souris, rat ou d'homme (0,33 mg prot/ml). Après addition de NADPH (nicotinamide adénine dinucléotide phosphate, forme réduite), des prélèvements sont réalisés à 0, 5, 15, 30 et 60 minutes. La réaction enzymatique est arrêtée avec le méthanol (V/V). La protéine est précipitée par centrifugation et le surnageant analysé par LC-MS-MS.
La bonne stabilité métabolique de ces dérivés permet d'envisager un traitement per os.

**TABLEAU IV**

| **% de prédiction de stabilité métabolique sur microsomes hépatiques** | | | |
|---|---|---|---|
| | Souris | Rat | Homme |
| Exemple 1 | 40 | - | 70 |
| Exemple 25 | 85 | - | 63 |
| Exemple 47 | 53 | 87 | 78 |
| Exemple X (41319) | 17 | - | 85 |

### EXEMPLE E : Composition pharmaceutique

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3g |

## Revendications

1. Composés de formule (I) : 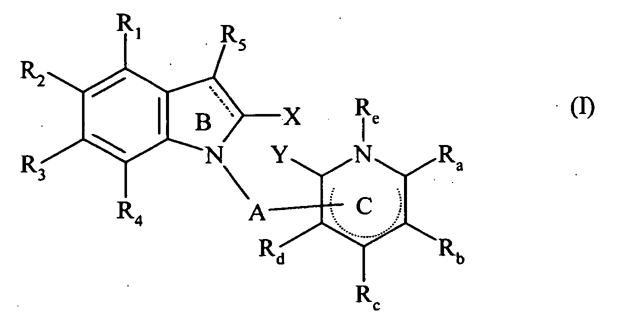 dans laquelle :
• A représente un radical bivalent : 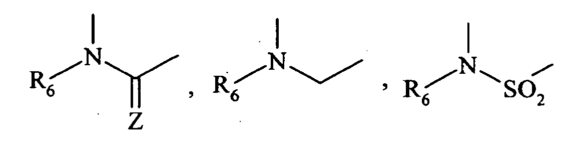 dans lesquels :
Z représente un atome d'oxygène ou un atome de soufre,
R₆ représente :
un atome d'hydrogène,
un groupement alkyle (C₁-C₆) linéaire ou ramifié, C(O)-AA dans lequel AA représente un radical amino-acide, alkoxycarbonyle (C₁-C₆) linéaire ou ramifié, CHR'-O-C(O)-R" dans lequel R' représente un atome d'hydrogène ou un groupement alkyle (C₁-C₆) linéaire ou ramifié et R" représente un alkyle (C₁-C₆) linéaire ou ramifié,
alkényle (C₂-C₆) linéaire ou ramifié, aryle, arylalkyle (C₁-C₆) linéaire ou ramifié, polyhalogénoalkyle (C₁-C₆) linéaire ou ramifié,
ou une chaîne alkyle (C₁-C₆) linéaire ou ramifié substituée par un ou plusieurs atomes d'halogène, un ou plusieurs groupements hydroxy, alkoxy (C₁-C₆) linéaire ou ramifié, ou amino éventuellement substitué par un ou deux groupements identiques ou différents, alkyle (C₁-C₆) linéaire ou ramifié,
• dans le cycle B,
----- représente une liaison simple ou une liaison double,
• dans le cycle C,
----- représente une liaison simple ou une liaison double, le cycle C ne contenant tout au plus qu'une seule liaison double,
• R₁, R₂, R₃, R₄, identiques ou différents, indépendamment les uns des autres, représentent :
un atome d'hydrogène ou halogène,
un groupement alkyle (C₁-C₆) linéaire ou ramifié, alkoxy (C₁-C₆) linéaire ou ramifié, hydroxy, cyano, nitro, polyhalogénoalkyle (C₁-C₆) linéaire ou ramifié, amino (éventuellement substitué par un ou deux groupements alkyle (C₁-C₆) linéaire ou ramifié, et/ou alkényle (C₂-C₆) linéaire ou ramifié, les groupements alkyle et alkényle pouvant être identiques ou différents),
ou une chaîne alkyle (C₁-C₆) linéaire ou ramifiée substituée par un ou plusieurs atomes d'halogène, un ou plusieurs groupements hydroxy, alkoxy (C₁-C₆) linéaire ou ramifié, ou amino éventuellement substitué par un ou deux groupements, alkyle (C₁-C₆) linéaire ou ramifié, identiques ou différents,
• R₅ représente :
un atome d'hydrogène,
un groupement alkyle (C₁-C₆) linéaire ou ramifié, un aminoalkyle (C₁-C₆) linéaire ou ramifié, ou un groupement hydroxyalkyle (C₁-C₆) linéaire ou ramifié,
• X, Y, identiques ou différents, indépendamment les uns des autres, représentent : un atome d'hydrogène, ou un groupement alkyle (C₁-C₆) linéaire ou ramifié,
• Rₐ, R_{b}, R_{c}, R_{d}, identiques ou différents, indépendamment les uns des autres, représentent :
un atome d'hydrogène, halogène,
un groupement alkyle (C₁-C₆) linéaire ou ramifié, hydroxy, alkoxy (C₁-C₆) linéaire ou ramifié, cyano, nitro, polyhalogénoalkyle (C₁-C₆) linéaire ou ramifié, amino (éventuellement substitué par un ou deux groupements identiques ou différents, alkyle (C₁-C₆) linéaire ou ramifié),
une chaîne alkyle (C₁-C₆) linéaire ou ramifiée substituée par un ou plusieurs groupements choisis parmi halogène, hydroxy, alkoxy (C₁-C₆) linéaire ou ramifié, ou amino éventuellement substitué par un ou deux groupements identiques ou différents, alkyle (C₁-C₆) linéaire ou ramifié,
étant entendu que lorsque A est lié au cycle C par un des atomes de carbone qui porte l'un des substituants Rₐ, R_{b}, R_{c}, R_{d} ou Y, et que ledit atome de carbone de liaison porte aussi une double liaison, le substituant Rₐ, R_{b}, Rₑ, R_{d} ou Y correspondant est absent,
• Rₑ représente :
un atome d'hydrogène,
un groupement alkyle (C₁-C₆) linéaire ou ramifié, arylalkyle (C₁-C₆) linéaire ou ramifié, alkényle (C₂-C₆) linéaire ou ramifié, alkynyle (C₂-C₆) linéaire ou ramifié, une chaîne alkyle (C₁-C₆) linéaire ou ramifiée substituée par un ou plusieurs groupements choisis parmi hydroxy, amino (éventuellement substitué par un ou deux groupements identiques ou différents, alkyle (C₁-C₆) linéaire ou ramifié), alkoxy (C₁-C₆) linéaire ou ramifié, ou NR₇R₈ dans lequel R₇ et R₈ forment ensemble avec l'atome d'azote qui les porte, un hétérocycle de 4 à 8 chaînons éventuellement substitué, contenant éventuellement une ou plusieurs doubles liaisons au sein de l'hétérocycle et contenant éventuellement au sein du système cyclique un second hétéroatome choisi parmi l'atome d'oxygène et l'atome d'azote, une chaîne alkényle (C₂-C₆)linéaire ou ramifiée substituée par les mêmes groupements que la chaîne alkyle et une chaîne alkynyle (C₂-C₆) linéaire ou ramifiée substituée par les mêmes groupements que la chaîne alkyle,
leurs énantiomères, diastéréoisomères, N-oxydes, ainsi que leurs sels d'addition à un acide ou à une base pharmaceutiquement acceptable,
étant entendu que :
par hétérocycle de 4 à 8 chaînons éventuellement substitué, contenant éventuellement une ou plusieurs doubles liaisons au sein de l'hétérocycle et contenant éventuellement au sein du système cyclique, un second hétéroatome choisi parmi l'atome d'oxygène ou l'atome d'azote, on peut nommer à titre non limitatif la pyrrolidine, la pipéridine, l'azépane, la pipérazine, la morpholine, ces hétérocycles pouvant éventuellement être substitués, y compris sur le deuxième atome d'azote de la pipérazine par un ou plusieurs groupements, identiques ou différents, choisis parmi alkyle (C₁-C₆) linéaire ou ramifié, hydroxyalkyle (C₁-C₆) linéaire ou ramifié, alkoxy (C₁-C₆)alkyle(C₁-C₆) linéaire ou ramifié, CO₂Rᵥ, CO₂-R_{w}-NRᵥR'ᵥ, CO₂-R_{w}-ORᵥ dans lesquels Rᵥ représente un atome d'hydrogène ou un groupement alkyle (C₁-C₆) linéaire ou ramifié, R'ᵥ a les mêmes définitions que Rᵥ et R_{w} représente une chaîne alkylène (C₁-C₆) linéaire ou ramifiée, aryle, aryloxycarbonyle, arylalkoxy (C₁-C₆) carbonyle linéaire ou ramifié, cycloalkyle éventuellement substitué, cycloalkylalkyle éventuellement substitué, hétérocycloalkyle éventuellement substitué, hétérocycloalkylalkyle éventuellement substitué, aminoalkyle (C₁-C₆) linéaire ou ramifié, la partie amino étant éventuellement substituée par un ou deux groupements identiques ou différents, alkyle (C₁-C₆) linéaire ou ramifié,
par aryle, on entend un groupement phényle ou naphtyle, les deux étant éventuellement substitués par un où plusieurs atomes d'halogène, groupements nitro, amino, alkyle (C₁-C₆) linéaire ou ramifié, ou alkoxy (C₁-C₆) linéaire ou ramifié,
par cycloalkyle, on entend un groupement monocyclique saturé contenant de 4 à 8 chaînons,
par cycloalkylalkyle, on entend le groupement cycloalkyle-alkyle dans lequel le groupement alkyle désigne une chaîne de 1 à 6 atomes de carbone, linéaire ou ramifié et le groupement cycloalkyle désigne un groupement monocyclique saturé contenant de 4 à 8 chaînons,
par hétérocycloalkyle, on entend un groupement monocyclique saturé contenant de 4 à 8 chaînons et possédant 1 ou 2 hétéroatomes choisis parmi azote, oxygène et soufre,
par hétérocycloalkylalkyle, on entend le groupement hétérocycloalkyle-alkyle dans lequel le groupement alkyle désigne une chaîne de 1 à 6 atomes de carbone, linéaire ou ramifié et le groupement hétérocycloalkyle désigne un groupement monocyclique saturé contenant de 4 à 8 chaînons et possédant 1 ou 2 hétéroatomes choisis parmi azote, oxygène et soufre,
l'expression "éventuellement substitué" lorsqu'elle concerne les groupements cycloalkyle, cycloalkylalkyle, hétérocycloalkyle, hétérocycloalkylalkyle signifie que ces groupements peuvent être substitués par 1 ou plusieurs substituants, identiques ou différents, choisis parmi alkyle (C₁-C₆) linéaire ou ramifié, hydroxyalkyle (C₁-C₆) linéaire ou ramifié alkoxy (C₁-C₆)alkyle(C₁-C₆) linéaire ou ramifié, carboxy, alkoxycarbonyle (C₁-C₆) linéaire ou ramifié, aminoalkyle (C₁-C₆) linéaire ou ramifié, la partie amino étant éventuellement substituée par un ou deux groupements identiques ou différents, alkyle (C₁-C₆) linéaire ou ramifié,
par radical amino-acide, on entend les radicaux alanyl, arginyl, asparaginyl, α-aspartyl, cystéinyl, α-glutamyl, glutaminyl, glycyl, histidyl, isoleucyl, leucyl, lysyl, méthionyl, phénylalanyl, prolyl, seryl, thréonyl, thryptophyl, tyrosyl et valyl.

2. Composés de formule (I) selon la revendication 1; **caractérisés en ce que** A représente un radical divalent : 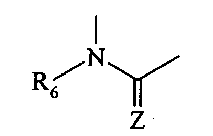 dans lequel R₆ est tel que défini dans la formule (I) et Z représente un atome d'oxygène, leurs énantiomères, diastéréoisomères, ainsi que leurs sels d'addition à un acide ou à une base pharmaceutiquement acceptable.

3. Composés de formule (I) selon l'une quelconque des revendications 1 à 2, **caractérisés en ce que** R₆ représente un atome d'hydrogène, leurs énantiomères, diastéréoisomères, N-oxydes, ainsi que leurs sels d'addition à un acide ou à une base pharmaceutiquement acceptable.

4. Composés de formule (I) selon l'une quelconque des revendications 1 à 3, **caractérisés en ce que** R₁, R₂, R₃ et R₄ représentent un atome d'hydrogène, un atome d'halogène ou le groupement alkoxy (C₁-C₆) linéaire ou ramifiée, leurs énantiomères, diastéréoisomères, N-oxydes, ainsi que leurs sels d'addition à un acide ou à une base pharmaceutiquement acceptable.

5. Composés de formule (I) selon l'une quelconque des revendications 1 à 4, **caractérisés en ce que** R₅ représente un atome d'hydrogène ou un groupement alkyle (C₁-C₆) linéaire ou ramifié, leurs énantiomères, diastéréoisomères, N-oxydes, ainsi que leurs sels d'addition à un acide ou à une base pharmaceutiquement acceptable.

6. Composés de formule (I) selon l'une quelconque des revendications 1 à 5, **caractérisés en ce que** X et Y représentent un atome d'hydrogène, ou un groupement alkyle (C₁-C₆) linéaire ou ramifié, leurs énantiomères, diastéréoisomères, N-oxydes, ainsi que leurs sels d'addition à un acide ou à une base pharmaceutiquement acceptable.

7. Composés de formule (I) selon l'une quelconque des revendications 1 à 6, **caractérisés en ce que** Rₐ, R_{b}, R_{c} et R_{d} représentent un atome d'hydrogène, leurs énantiomères, diastéréoisomères, N-oxydes, ainsi que leurs sels d'addition à un acide ou à une base pharmaceutiquement acceptable.

8. Composés de formule (I) selon l'une quelconque des revendications 1 à 7, **caractérisés en ce que** Rₑ représente un atome d'hydrogène, ou un groupement alkyle (C₁-C₆) linéaire ou ramifié, ou alkényle (C₂-C₆) linéaire ou ramifié, leurs énantiomères, diastéréoisomères, N-oxydes, ainsi que leurs sels d'addition à un acide ou à une base pharmaceutiquement acceptable.

9. Composés de formule (I) selon l'une quelconque des revendications 1 à 8, **caractérisés en ce qu'**ils représentent des composés de formule (I/A) : 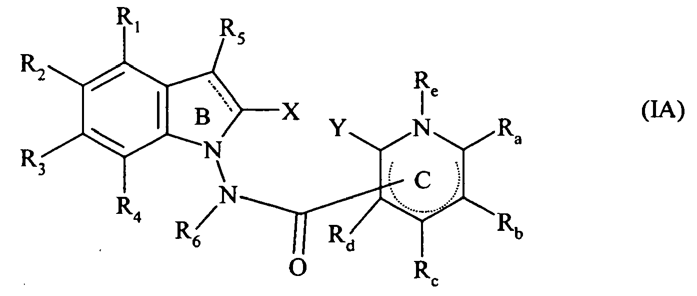 dans laquelle -----, X, Y, R₁, R₂, R₃, R₄, R₅, R₆, Rₐ, R_{b}, R_{c}, R_{d} et Rₑ sont tels que définis dans la formule (I), leurs énantiomères, diastéréoisomères, N-oxydes, ainsi que leurs sels d'addition à un acide ou à une base pharmaceutiquement acceptable.

10. Composés de formule (I) selon l'une quelconque des revendications 1 à 9, **caractérisés en ce qu'**ils représentent des composés de formule (I/B) : 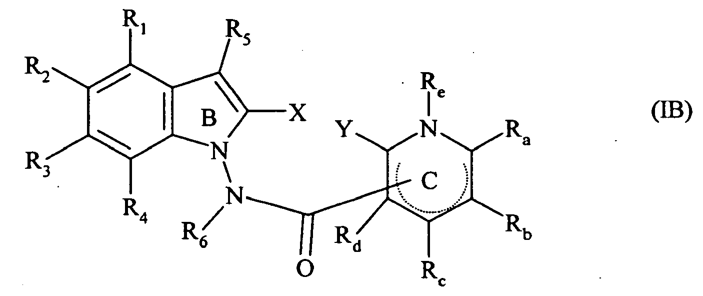 dans laquelle -----, X, Y, R₁, R₂, R₃, R₄, R₅, R₆, Rₐ, R_{b}, R_{c}, R_{d} et Rₑ sont tels que définis dans la formule (I), leurs énantiomères, diastéréoisomères, N-oxydes, ainsi que leurs sels d'addition à un acide ou à une base pharmaceutiquement acceptable.

11. Composés de formule (I) selon l'une quelconque des revendications 1 à 10, **caractérisés en ce qu'**ils représentent des composés de formule (I/C) : 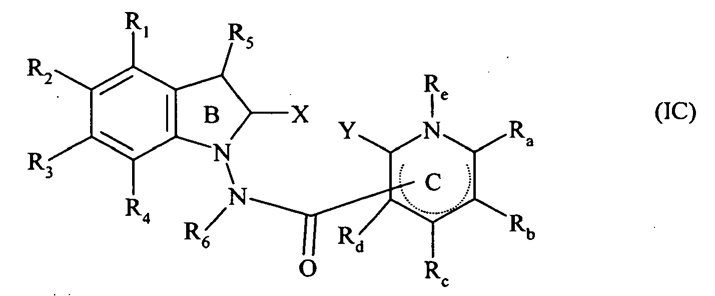 dans laquelle -----, X, Y, R₁, R₂, R₃, R₄, R₅, R₆, Rₐ, R_{b}, R_{c}, R_{d} et Rₑ sont tels que définis dans la formule (I), leurs énantiomères, diastéréoisomères, N-oxydes, ainsi que leurs sels d'addition à un acide ou à une base pharmaceutiquement acceptable.

12. Composés de formule (I) selon l'une quelconque des revendications 1 à 11, **caractérisés en ce qu'**ils représentent des composés de formule (I/D) : 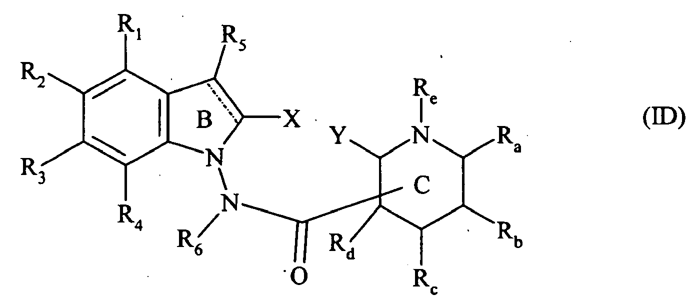 dans laquelle -----, X, Y, R₁, R₂, R₃, R₄, R₅, R₆, Rₐ, R_{b}, R_{c}, R_{d} et Rₑ sont tels que définis dans la formule (I), leurs énantiomères, diastéréoisomères, N-oxydes, ainsi que leurs sels d'addition à un acide ou à une base pharmaceutiquement acceptable.

13. Composés de formule (I) selon l'une quelconque des revendications 1 à 12, **caractérisés en ce qu'**ils représentent des composés de formule (I/E) : 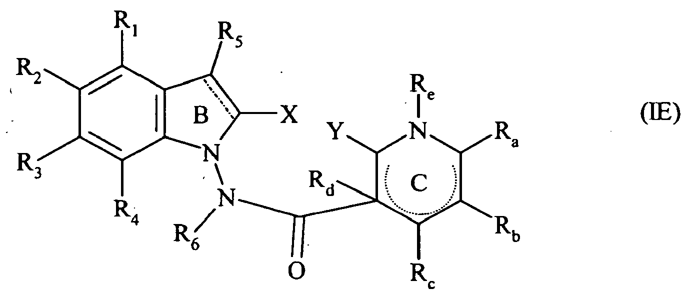 dans laquelle -----, X, Y, R₁, R₂, R₃, R₄, R₅, R₆, Rₐ, R_{b}, R_{c}, R_{d} et Rₑ sont tels que définis dans la formule (I), leurs énantiomères, diastéréoisomères, N-oxydes, ainsi que leurs sels d'addition à un acide ou à une base pharmaceutiquement acceptable.

14. Composés de formule (I) selon l'une quelconque des revendications 1 à 13, **caractérisés en ce qu'**ils représentent des composés de formule (I/F) : 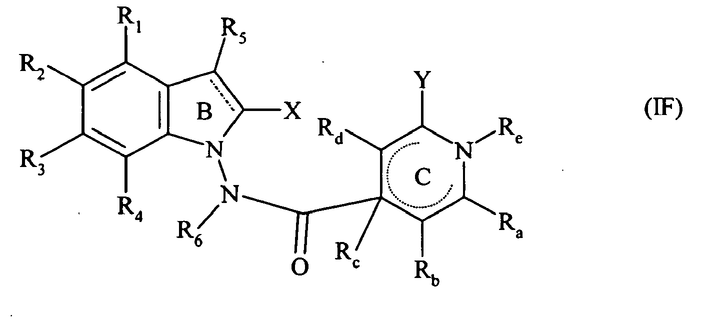 dans laquelle -----, X, Y, R₁, R₂, R₃, R₄, R₅, R₆, Rₐ, R_{b}, R_{c}, R_{d} et Rₑ sont tels que définis dans la formule (I), leurs énantiomères, diastéréoisomères, N-oxydes, ainsi que leurs sels d'addition à un acide ou à une base pharmaceutiquement acceptable.

15. Composés de formule (I) selon l'une quelconque des revendications 1 à 14, **caractérisés en ce qu'**ils représentent des composés de formule (I/G) : 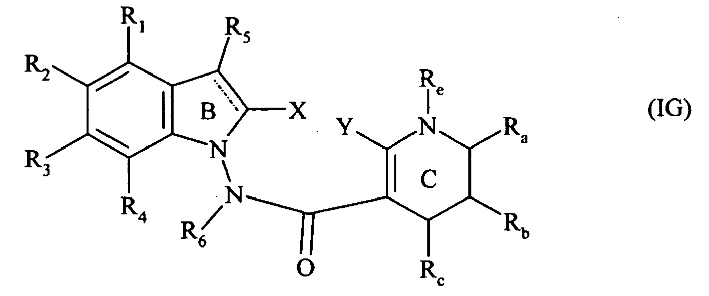 dans laquelle -----, X, Y, R₁, R₂, R₃, R₄, R₅, R₆, Rₐ, R_{b}, R_{c} et Rₑ sont tels que définis dans la formule (I), leurs énantiomères, diastéréoisomères, N-oxydes, ainsi que leurs sels d'addition à un acide ou à une base pharmaceutiquement acceptable.

16. Composés de formule (I) selon l'une quelconque des revendications 1 à 15, **caractérisés en ce qu'**ils représentent des composés de formule (I/H) : 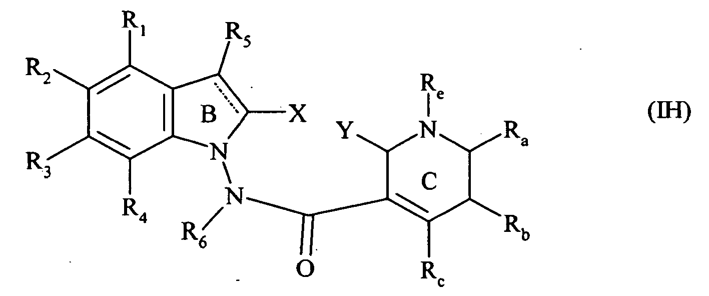 dans laquelle -----, X, Y, R₁, R₂, R₃, R₄, R₅, R₆, Rₐ, R_{b}, R_{c} et Rₑ sont tels que définis dans la formule (I), leurs énantiomères, diastéréoisomères, N-oxydes, ainsi que leurs sels d'addition à un acide ou à une base pharmaceutiquement acceptable.

17. Composés de formule (I) selon l'une quelconque des revendications 1 à 16, **caractérisés en ce qu'**ils représentent des composés de formule (I/J) : 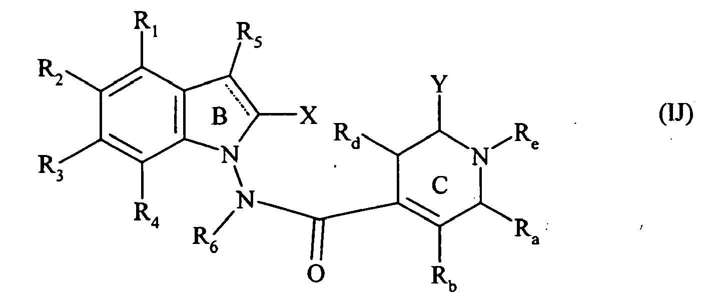 dans laquelle -----, X, Y, R₁, R₂, R₃, R₄, R₅, R₆, Rₐ, R_{b}, R_{d} et Rₑ sont tels que définis dans la formule (I), leurs énantiomères, diastéréoisomères, N-oxydes, ainsi que leurs sels d'addition à un acide ou à une base pharmaceutiquement acceptable.

18. Composés de formule (I) selon la revendication 1 qui sont le :
• *N*-(1*H*-indol-1-yl)-1-méthyl-1,2,5,6-tétrahydropyridine-3-carboxamide,
• *N*-(2,3-dihydro-1*H*-indol-1-yl)-1-méthyl-1,4,5,6-tétrahydropyridine-3-carboxamide,
• *N*-(5-fluoro-1*H*-indol-1-yl)-1-méthyl-1,2,5,6-tétrahydropyridine-3-carboxamide,
• *N*-(2,3-dihydro-1*H*-indol-1-yl)-1-méthyl-1,4,5,6-tétrahydropyridine-3-carboxamide,
• 1-[2-(diméthylamino)éthyl]-*N*-(1*H*-indol-1-yl)-1,2,5,6-tétrahydropyridine-3-carboxamide,
• *N*-(1*H*-indol-1-yl)-1-[2-(4-méthyl-1-pipérazinyl)éthyl]-3-pipéridinecarboxamide,
• *N*-(5-chloro-1*H*-indol-1-yl)-1-(2-hydroxyéthyl)-1,4,5,6-tétrahydropyridine-3-carboxamide,
• 4-(2-{3-[(1*H*-indol-1-ylamino)carbonyl]-1-pipéridinyl}éthyl)-pipérazine-1-carboxylate de *tert*-butyle,
• 1-[3-(diméthylammonium)propyl]-3-[(1*H*-indol-1-ylamino)carbonyl]pipéridinium,
• *N*-(1*H*-indol-1-yl)-1-[3-(1-pipéridinyl)propyl]-3-pipéridinecarboxamide,
• *N*-(1*H*-indol-1-yl)-1-[3-(4-méthyl-1-pipérazinyl)propyl]-3-pipéridinecarboxamide,
• *N*-(Indol-1-yl)-1-(2-pipéridin-1-yl-éthyl)-1,2,5,6-tétrahydropyridine-3-carboxamide,
• (±)-N-(indol-1-yl)-1-[2-[4-(1-méthylpipéridin-4-yl)pipérazin-1-yl)]éthyl]pipéridine-3-carboxamide,
• (±)-N-(indol-1-yl)-1-[3-[4-(2-hydroxyéthyl) pipérazin-1-yl)]propyl]pipéridine-3-carboxamide,
• (±)-N-(indol-1-yl)1-[4-(4-méthylpipérazin-1-yl)butyl]pipéridine-3-carboxamide,
• (±)-N-(Indol-1-yl)-1-allylpipéridine-3-carboxamide,
• (±)-N-(Indol-1-yl)-1-[4-(pipéridin-1-yl)but-2-èn-1-yl]-pipéridine-3-carboxamide,
• (R ou S) (-)-N-(Indol-1-yl)-1-[2-(pipéridin-1-yl)éthyl]pipéridine-3-carboxamide énantiomère 1,
• (R ou S) (+)-N-(Indol-1-yl)-1-[2-(pipéridin-1-yl)éthyl]pipéridine-3-carboxamide énantiomère 2.
leurs énantiomères, diastéréoisomères, N-oxydes, ainsi que leurs sels d'addition à un acide ou à une base pharmaceutiquement acceptable.

19. Procédé de préparation des composés de formule (I) selon la revendication 1, **caractérisé en ce qu'**on utilise comme produit de départ un composé de formule (II) : 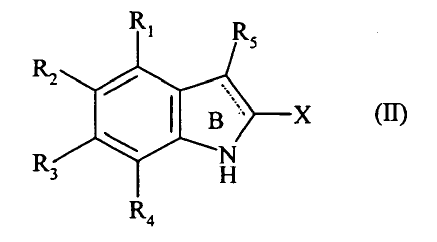 dans laquelle R₁, R₂, R₃, R₄, R₅ et X sont tels que définis dans la formule (I),
composé que l'on met en présence de diphénylphosphinylhydroxylamine, pour conduire au composé de formule (III) : 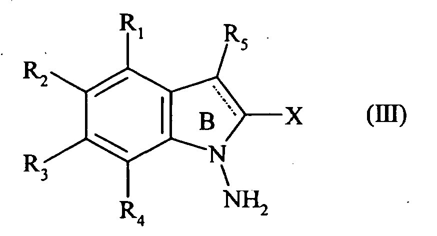 dans laquelle R₁, R₂, R₃, R₄, R₅ et X sont tels que définis précédemment,
composé de formule (III) qui est condensé avec un composé de formule (IV) : 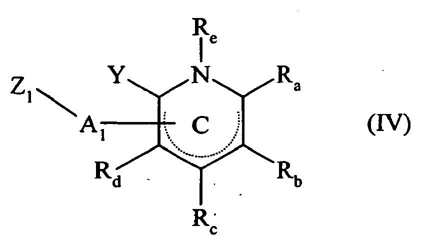 dans laquelle Rₐ, R_{b}, R_{c}, R_{d}, Rₑ et Y sont tels que définis dans la formule (I), A1 représente un groupement de formule -C(=Z)-, -CH₂-, -SO₂- dans lequel Z est tel que défini dans la formule (I) et Z₁ représente un groupement choisi parmi hydroxy, éthoxy ou méthoxy,
pour conduire au composé de formule (I/a), cas particulier des composés de formule (I) : 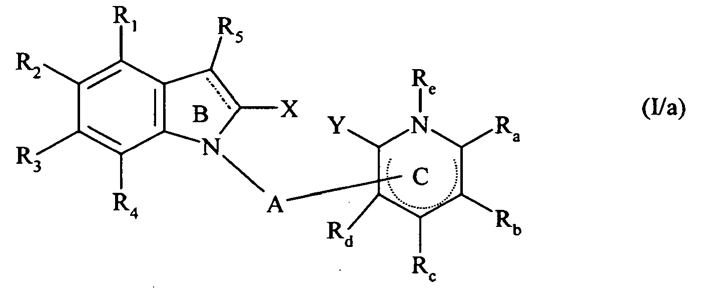 dans laquelle R₁, R₂, R₃, R₄, R₅, Rₐ, R_{b}, R_{c}, R_{d}, Rₑ, X et Y sont tels que définis précédemment et A est tel que défini dans la formule (I),
le composé de formule (I/a) formant l'ensemble des composés de l'invention qui sont purifiés, le cas échéant, selon une technique classique de purification, qui peuvent, si on le désire, être séparés en leurs différents isomères selon une technique classique de séparation et qui sont transformés, si on le souhaite, en leurs N-oxydes et, le cas échéant, en leurs sels d'addition à un acide ou à une base pharmaceutiquement acceptable.

20. Compositions pharmaceutiques contenant comme principe actif au moins un composé selon l'une quelconque des revendications 1 à 18, en combinaison avec un ou plusieurs excipients ou véhicules inertes, non toxiques, pharmaceutiquement acceptables.

21. Compositions pharmaceutiques selon la revendication 20 contenant au moins un principe actif, inducteur de tyrosine hydroxylases, selon l'une quelconque des revendications 1 à 18 et utiles dans le traitement des dépressions, de l'anxiété, des troubles de la mémoire au cours de la sénescence et/ou de maladies neurodégénératives, et dans le traitement palliatif de la maladie de Parkinson et pour l'adaptation au stress.

## Claims

1. Compounds of formula (I) : 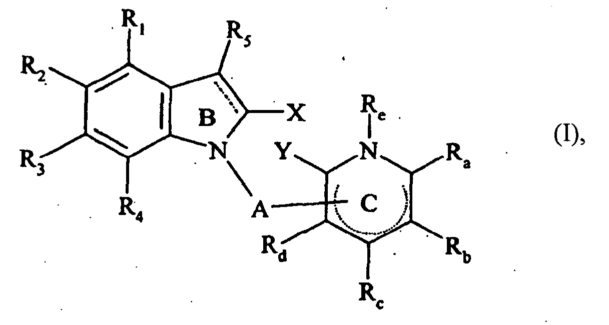 wherein :
• A represents a divalent radical : 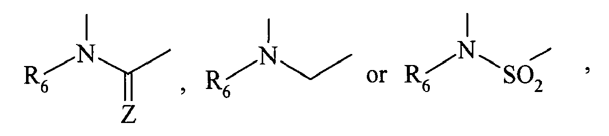 wherein :
Z represents an oxygen atom or sulphur atom,
R₆ represents :
a hydrogen atom,
a linear or branched (C₁-C₆)alkyl group, C(O)-AA wherein AA represents an amino acid radical, a linear or branched (C₁-C₆)alkoxy-carbonyl group, CHR'-O-C(O)-R" wherein R' represents a hydrogen atom or a linear or branched (C₁-C₆)alkyl group and R" represents a linear or branched (C₁-C₆)alkyl group,
a linear or branched (C₂-C₆)alkenyl group, an aryl group, an aryl-(C₁-C₆)alkyl group in which the alkyl moiety is linear or branched, a linear or branched (C₁-C₆)polyhaloalkyl group,
or a linear or branched (C₁-C₆)alkyl chain substituted by one or more halogen atoms, one or more hydroxy groups, linear or branched (C₁-C₆)alkoxy groups, or amino groups optionally substituted by one or two identical or different, linear or branched (C₁-C₆)alkyl groups,
• in the ring B
----- represents a single bond or a double bond,
• in the ring C
----- represents a single bond or a double bond, the ring C containing, at most, only one double bond,
• R₁, R₂, R₃ and R₄, which may be the same or different, each independently of the others, represent:
a hydrogen or halogen atom,
a linear or branched (C₁-C₆)alkyl group, a linear or branched (C₁-C₆)alkoxy group, a hydroxy group, a cyano group, a nitro group, a linear or branched (C₁-C₆)polyhaloalkyl group, an amino group (optionally substituted by one or two linear or branched (C1-C₆)alkyl and/or linear or branched (C₂-C₆)alkenyl groups, it being possible for the alkyl and alkenyl groups to be the same or different),
or a linear or branched (C₁-C₆)alkyl chain substituted by one or more halogen atoms, one or more hydroxy groups, linear or branched (C₁-C₆)alkoxy groups, or amino groups optionally substituted by one or two identical or different, linear or branched (C₁-C₆)alkyl groups,
• R₅ represents :
a hydrogen atom,
a linear or branched (C₁-C₆)alkyl group, an aminoalkyl group in which the alkyl moiety is a linear or branched chain of 1 to 6 carbon atoms, or a linear or branched (C₁-C₆)-hydroxyalkyl group,
• X and Y, which may be the same or different, each independently of the other, represent:
a hydrogen atom or a linear or branched (C₁-C₆)alkyl group,
• Rₐ, R_{b}, R_{c} and R_{d}, which may be the same or different, each independently of the others, represent:
a hydrogen or halogen atom,
a linear or branched (C₁-C₆)alkyl group, a hydroxy group, a linear or branched (C₁-C₆)alkoxy group, a cyano group, a nitro group, a linear or branched (C₁-C₆)poly-haloalkyl group, an amino group (optionally substituted by one or two identical or different, linear or branched (C₁-C₆)alkyl groups),
or a linear or branched (C₁-C₆)alkyl chain substituted by one or more groups selected from halogen, hydroxy, linear or branched (C₁-C₆)alkoxy, and amino optionally substituted by one or two identical or different, linear or branched (C₁-C₆)alkyl groups,
it being understood that when A is linked to the ring C at a carbon atom carrying one of the substituents Rₐ, R_{b}, R_{c}, R_{d} or Y and said linking carbon atom also carries a double bond, then the corresponding substituent Rₐ, R_{b}, R_{c}, R_{d} or Y is absent,
• Rₑ represents :
a hydrogen atom,
a linear or branched (C₁-C₆)alkyl group; an aryl-(C₁-C₆)alkyl group in which the alkyl moiety is linear or branched; a linear or branched (C₂-C₆)alkenyl group; a linear or branched (C₂-C₆)alkynyl group; a linear or branched (C₁-C₆)alkyl chain substituted by one or more groups selected from hydroxy, amino (optionally substituted by one or two identical or different, linear or branched (C₁-C₆)alkyl groups), linear or branched (C₁-C₆)alkoxy, and NR₇R₈ wherein R₇ and R₈, together with the nitrogen atom carrying them, form an optionally substituted, 4- to 8-membered heterocycle optionally containing one or more double bonds within the heterocycle and optionally containing within the cyclic system a second hetero atom selected from an oxygen atom and a nitrogen atom; or a linear or branched (C₂-C₆)alkenyl chain substituted by the same groups as the alkyl chain or a linear or branched (C₂-C₆)alkynyl chain substituted by the same groups as the alkyl chain,
their enantiomers, diastereoisomers, and N-oxides, and also addition salts thereof with a pharmaceutically acceptable acid or base,
it being understood that :
as an optionally substituted, 4- to 8-membered heterocycle optionally containing one or more double bonds within the heterocycle and optionally containing within the cyclic system a second hetero atom selected from an oxygen atom and a nitrogen atom, there may be mentioned, without implying any limitation, pyrrolidine, piperidine, azepane, piperazine and morpholine, those heterocycles optionally being substituted (including on the second nitrogen atom of piperazine) by one or more identical or different groups selected from linear or branched (C₁-C₆)alkyl, linear or branched (C₁-C₆)hydroxyalkyl, linear or branched (C₁-C₆)alkoxy-(C₁-C₆)alkyl, CO₂Rᵥ, CO₂-R_{w}-NRᵥR'ᵥ, CO₂-R_{w}-ORᵥ (wherein Rᵥ represents a hydrogen atom or a linear or branched (C₁-C₆)alkyl group, R'ᵥ is as defined for Rᵥ and R_{w} represents a linear or branched (C₁-C₆)alkylene chain), aryl, aryloxycarbonyl, linear or branched aryl-(C₁-C₆)alkoxy-carbonyl, optionally substituted cycloalkyl, optionally substituted cycloalkylalkyl, optionally substituted heterocycloalkyl, optionally substituted heterocycloalkylalkyl, and aminoalkyl in which the alkyl moiety is a linear or branched chain of 1 to 6 carbon atoms and the amino moiety optionally is substituted by one or two identical or different, linear or branched (C₁-C₆)alkyl groups,
aryl means a phenyl or naphthyl group, each optionally being substituted by one or more halogen atoms, nitro, amino, linear or branched (C₁-C₆)alkyl or linear or branched (C₁-C₆)-alkoxy groups,
cycloalkyl means a saturated, 4- to 8-membered, monocyclic group,
cycloalkylalkyl means a cycloalkyl-alkyl group wherein the alkyl group denotes a linear or branched chain of 1 to 6 carbon atoms and the cycloalkyl group denotes a saturated, 4- to 8-membered, monocyclic group,
heterocycloalkyl means a saturated, 4- to 8-membered, monocyclic group containing 1 or 2 hetero atoms selected from nitrogen, oxygen and sulphur,
heterocycloalkylalkyl means a heterocycloalkyl-alkyl group wherein the alkyl group denotes a linear or branched chain of 1 to 6 carbon atoms and the heterocycloalkyl group denotes a saturated, 4- to 8-membered, monocyclic group containing 1 or 2 hetero atoms selected from nitrogen, oxygen and sulphur,
the expression "optionally substituted" when referring to the groups cycloalkyl, cycloalkylalkyl, heterocycloalkyl and heterocycloalkylalkyl means that those groups may be substituted by one or more identical or different substituents selected from linear or branched (C₁-C₆)alkyl, linear or branched (C₁-C₆)hydroxyalkyl, linear or branched (C₁-C₆)alkoxy-(C₁-C₆)alkyl, carboxy, linear or branched (C₁-C₆)alkoxy-carbonyl and aminoalkyl in which the alkyl moiety is a linear or branched chain of 1 to 6 carbon atoms and the amino moiety optionally is substituted by one or two identical or different, linear or branched (C₁-C₆)alkyl groups,
an amino acid radical is understood to mean the radicals alanyl, arginyl, asparaginyl, α-aspartyl, cysteinyl, α-glutamyl, glutaminyl, glycyl, histidyl, isoleucyl, leucyl, lysyl, methionyl, phenylalanyl, prolyl, seryl, threonyl, tryptophyl, tyrosyl and valyl.

2. Compounds of formula (I) according to claim 1, **characterised in that** A represents a divalent radical : 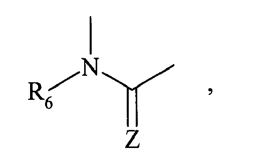 wherein R₆ is as defined for formula (I) and Z represents an oxygen atom, their enantiomers and diastereoisomers, and also addition salts thereof with a pharmaceutically acceptable acid or base.

3. Compounds of formula (I) according to either claim 1 or claim 2, **characterised in that** R₆ represents a hydrogen atom, their enantiomers, diastereoisomers, and N-oxides, and also addition salts thereof with a pharmaceutically acceptable acid or base.

4. Compounds of formula (I) according to any one of claims 1 to 3, **characterised in that** R₁, R₂, R₃ and R₄ represent a hydrogen atom, a halogen atom or a linear or branched (C₁-C₆)alkoxy group, their enantiomers, diastereoisomers, and N-oxides, and also addition salts thereof with a pharmaceutically acceptable acid or base.

5. Compounds of formula (I) according to any one of claims 1 to 4, **characterised in that** R₅ represents a hydrogen atom or a linear or branched (C₁-C₆)alkyl group, their enantiomers, diastereoisomers, and N-oxides, and also addition salts thereof with a pharmaceutically acceptable acid or base.

6. Compounds of formula (I) according to any one of claims 1 to 5, **characterised in that** X and Y represent a hydrogen atom or a linear or branched (C₁-C₆)alkyl group, their enantiomers, diastereoisomers, and N-oxides, and also addition salts thereof with a pharmaceutically acceptable acid or base.

7. Compounds of formula (I) according to any one of claims 1 to 6, **characterised in that** Rₐ, R_{b}, R_{c} and R_{d} represent a hydrogen atom, their enantiomers, diastereoisomers, and N-oxides, and also addition salts thereof with a pharmaceutically acceptable acid or base.

8. Compounds of formula (I) according to any one of claims 1 to 7, **characterised in that** Rₑ represents a hydrogen atom or a linear or branched (C₁-C₆)alkyl or linear or branched (C₂-C₆)alkenyl group, their enantiomers, diastereoisomers, and N-oxides, and also addition salts thereof with a pharmaceutically acceptable acid or base.

9. Compounds of formula (I) according to any one of claims 1 to 8, **characterised in that** they represent compounds of formula (I/A) : 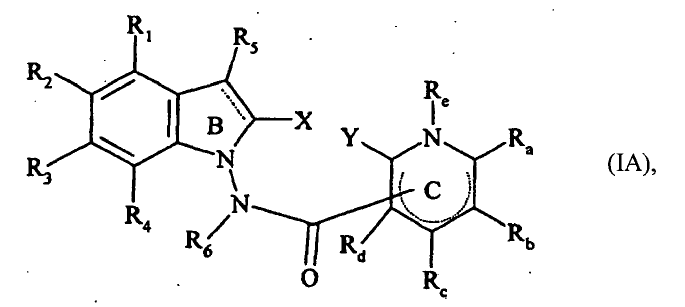 wherein -----, X, Y, R₁, R₂, R₃, R₄, Rₛ, R₆, Rₐ, R_{b}, R_{c}, R_{d} and Rₑ are as defined for formula (I), their enantiomers, diastereoisomers, and N-oxides, and also addition salts thereof with a pharmaceutically acceptable acid or base.

10. Compounds of formula (I) according to any one of claims 1 to 9, **characterised in that** they represent compounds of formula (I/B) : 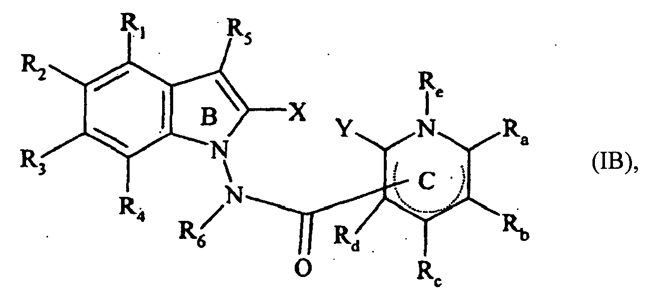 wherein -----, X, Y, R₁, R₂, R₃, R₄, R₅, R₆, Rₐ, R_{b}, R_{c}, R_{d} and Rₑ are as defined for formula (I), their enantiomers, diastereoisomers, and N-oxides, and also addition salts thereof with a pharmaceutically acceptable acid or base.

11. Compounds of formula (I) according to any one of claims 1 to 10, **characterised in that** they represent compounds of formula (I/C) : 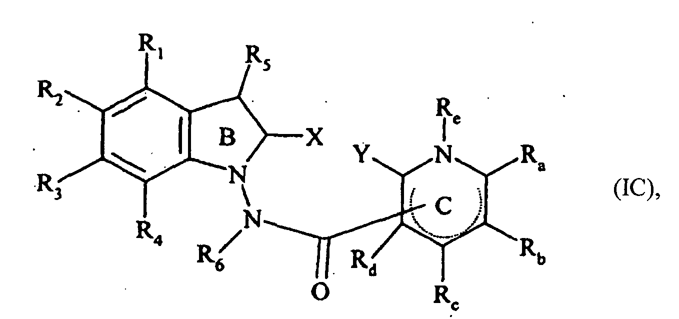 wherein -----, X, Y, R₁, R₂, R₃, R₄, R₅, R₆, Rₐ, R_{b}, R_{c}, R_{d} and Rₑ are as defined for formula (I), their enantiomers, diastereoisomers, and N-oxides, and also addition salts thereof with a pharmaceutically acceptable acid or base.

12. Compounds of formula (I) according to any one of claims 1 to 11, **characterised in that** they represent compounds of formula (I/D) : 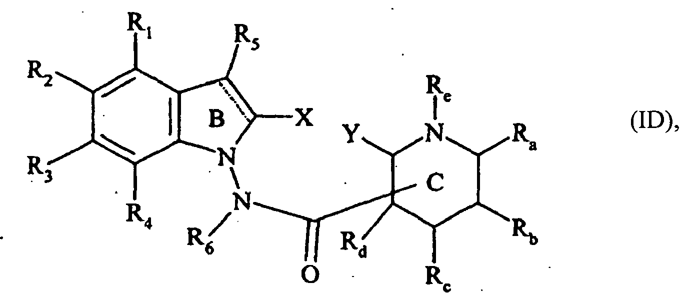 wherein -----, X, Y, R₁, R₂, R₃, R₄, R₅, R₆, Rₐ, R_{b}, R_{c}, R_{d} and Rₑ are as defined for formula (I), their enantiomers, diastereoisomers, and N-oxides, and also addition salts thereof with a pharmaceutically acceptable acid or base.

13. Compounds of formula (I) according to any one of claims 1 to 12, **characterised in that** they represent compounds of formula (I/E) : 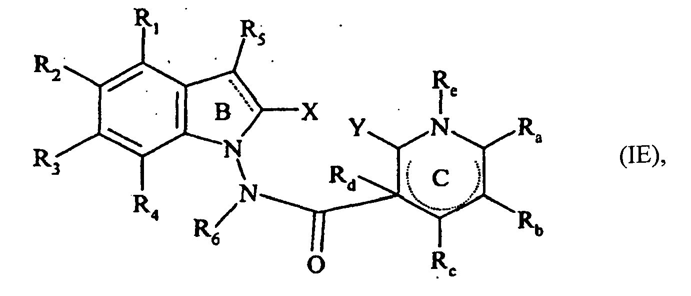 wherein -----, X, Y, R₁, R₂, R₃, R₄, R₅, R₆, Rₐ, R_{b}, R_{c}, R_{d} and Rₑ are as defined for formula (I), their enantiomers, diastereoisomers, and N-oxides, and also addition salts thereof with a pharmaceutically acceptable acid or base.

14. Compounds of formula (I) according to any one of claims 1 to 13, **characterised in that** they represent compounds of formula (I/F) : 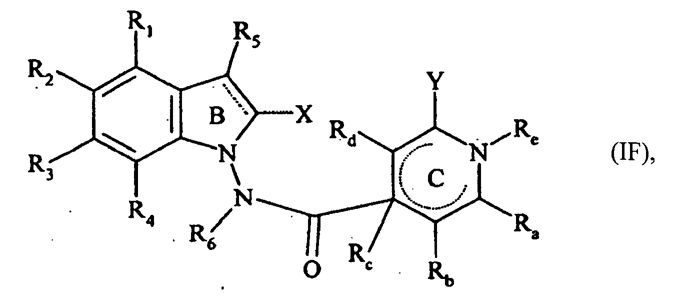 wherein -----, X, Y, R₁, R₂, R₃, R₄, R₅, R₆, Rₐ, R_{b}, R_{c}, R_{d} and Rₑ are as defined for formula (I), their enantiomers, diastereoisomers, and N-oxides, and also addition salts thereof with a pharmaceutically acceptable acid or base.

15. Compounds of formula (I) according to any one of claims 1 to 14, **characterised in that** they represent compounds of formula (I/G) : 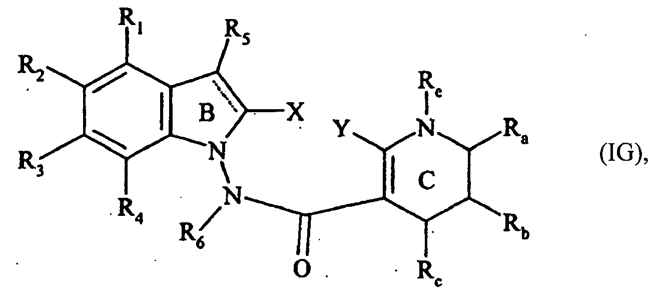 wherein -----, X, Y, R₁, R₂, R₃, R₄, R₅, R₆, Rₐ, R_{b}, R_{c} and Rₑ are as defined for formula (I), their enantiomers, diastereoisomers, and N-oxides, and also addition salts thereof with a pharmaceutically acceptable acid or base.

16. Compounds of formula (I) according to any one of claims 1 to 15, **characterised in that** they represent compounds of formula (I/H) : 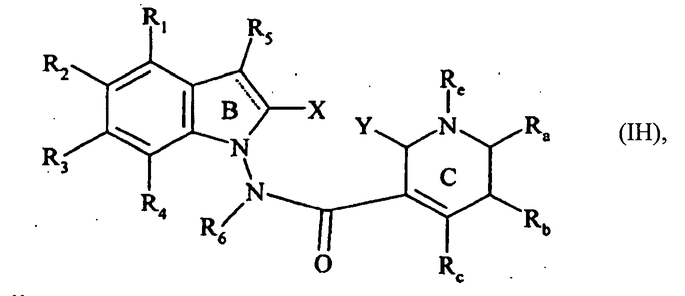 wherein -----, X, Y, R₁, R₂, R₃, R₄, R₅, R₆, Rₐ, R_{b}, R_{c} and Rₑ are as defined for formula (I), their enantiomers, diastereoisomers, and N-oxides, and also addition salts thereof with a pharmaceutically acceptable acid or base.

17. Compounds of formula (I) according to any one of claims 1 to 16, **characterised in that** they represent compounds of formula (I/J) : 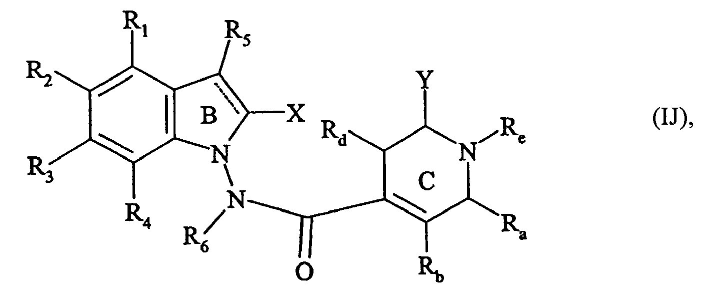 wherein -----, X, Y, R₁, R₂, R₃, R₄, R₅, R₆, Rₐ, R_{b}, R_{d} and Rₑ are as defined for formula (I), their enantiomers, diastereoisomers, and N-oxides, and also addition salts thereof with a pharmaceutically acceptable acid or base.

18. Compounds of formula (I) according to claim 1 which are :
• *N*-(1*H*-indol-1-yl)-1-methyl-1,2,5,6-tetrahydropyridine-3-carboxamide,
• *N*-(2,3-dihydro-1*H*-indol-1-yl)-1-methyl-1,4,5,6-tetrahydropyridine-3-carboxamide,
• *N*-(5-fluoro-1*H*-indol-1-yl)-1-methyl-1,2,5,6-tetrahydropyridine-3-carboxamide,
• *N*-(2,3-dihydro-1*H*-indol-1-yl)-1-methyl-1,4,5,6-tetrahydropyridine-3-carboxamide,
• 1-[2-(dimethylamino)ethyl]-*N*-(1*H*-indol-1-yl)-1,2,5,6-tetrahydropyridine-3-carboxamide,
• *N*-(1*H*-indol-1-yl)-1-[2-(4-methyl-1-piperazinyl)ethyl]-3-piperidinecarboxamide,
• *N*-(5-chloro-1*H*-indol-1-yl)-1-(2-hydroxyethyl)-1,4,5,6-tetrahydropyridine-3-carboxamide,
• *tert*-butyl 4-(2-(3-[(1*H*-indol-1-ylamino)carbonyl]-1-piperidyl)ethyl)piperazine-1-carboxylate,
• 1-[3-(dimethylammonium)propyl]-3-[(1*H*-indol-1-ylamino)carbonyl]piperidinium,
• *N*-(1*H*-indol-1-yl)-1-[3-(1-piperidyl)propyl]-3-piperidinecarboxamide,
• *N*-(1*H*-indol-1-yl)-1-[3-(4-methyl-1-piperazinyl)propyl]-3-piperidinecarboxamide,
• *N*-(indol-1-yl)-1-(2-piperidin-1-yl-ethyl)-1,2,5,6-tetrahydropyridine-3-carboxamide,
• (±)-N-(indol-1-yl)-1-[2-[4-(1-methylpiperidin-4-yl)piperazin-1-yl)]ethyl]piperidine-3-carboxamide,
• (±)-N-(indol-1-yl)-1-[3-[4-(2-hydroxyethyl)piperazin-1-yl)]propyl]piperidine-3-carboxamide,
• (±)-N-(indol-1-yl)-1-[4-(4-methylpiperazin-1-yl)butyl]piperidine-3-carboxamide,
• (±)-N-(indol-1-yl)-1-allylpiperidine-3-carboxamide,
• (±)-N-(indol-1-yl)-1-[4-(piperidin-1-yl)but-2-en-1-yl]piperidine-3-carboxamide,
• (R or S) (-)-N-(indol-1-yl)-1-[2-(piperidin-1-yl)ethyl]piperidine-3-carboxamide enantiomer 1,
• (R or S) (+)-N-(indol-1-yl)-l-[2-(piperidin-1-yl)ethyl]piperidine-3-carboxamide enantiomer 2,
their enantiomers, diastereoisomers, and N-oxides, and also addition salts thereof with a pharmaceutically acceptable acid or base.

19. Process for the preparation of compounds of formula (I) according to claim 1, **characterised in that** there is used as starting material a compound of formula (II) : 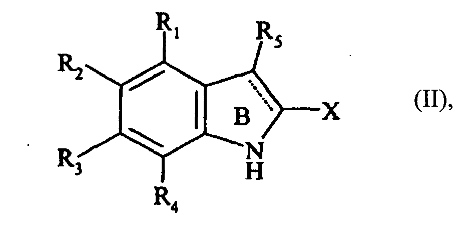 wherein R₁, R₂, R₃, R₄, R₅ and X are as defined for formula (I), which compound is placed in the presence of diphenylphosphinylhydroxylamine to yield the compound of formula (III) : 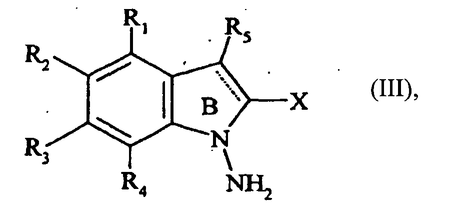 wherein R₁, R₂, R₃, R₄, R₅ and X are as defined hereinbefore,
which compound of formula (III) is condensed with a compound of formula (IV) : 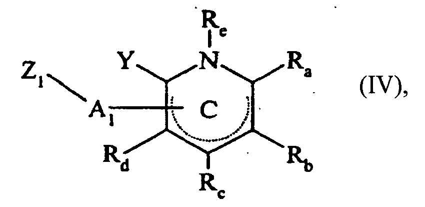 wherein Rₐ, R_{b}, R_{c}, R_{d}, Rₑ and Y are as defined for formula (I), A₁ represents a group of formula -C(=Z)-, -CH₂- or -SO₂- wherein Z is as defined for formula (I) and Z₁ represents a group selected from hydroxy, ethoxy and methoxy,
to yield the compound of formula (I/a), a particular case of the compounds of formula (I) : 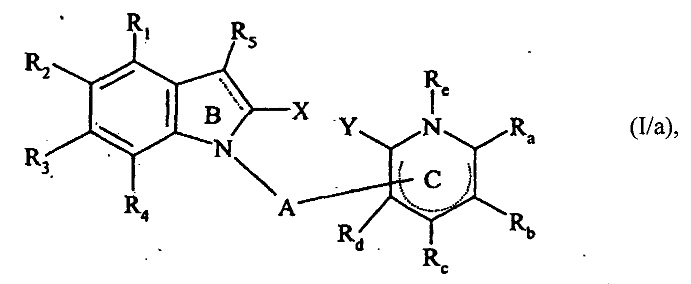 wherein R₁, R₂, R₃, R₄, R₅, Rₐ, R_{b}, R_{c}, R_{d}, Rₑ, X and Y are as defined hereinbefore and A is as defined for formula (I),
the compounds of formula (I/a) forming the entirety of the compounds of the invention, which are purified, if necessary, according to a conventional purification technique, may be separated, when desired, into their different isomers according to a conventional separation technique and are converted, when desired, into their N-oxides and, where appropriate, their addition salts with a pharmaceutically acceptable acid or base.

20. Pharmaceutical compositions comprising as active ingredient at least one compound according to any one of claims 1 to 18 in combination with one or more inert, non-toxic, pharmaceutically acceptable excipients or carriers.

21. Pharmaceutical compositions according to claim 20 comprising at least one active ingredient, an inducer of tyrosine hydroxylases, according to any one of claims 1 to 18 and for use in the treatment of depression, anxiety, disorders of memory in the course of ageing and/or neurodegenerative diseases, and in the palliative treatment of Parkinson's disease, and for adaptation to stress.

## Patentansprüche

1. Verbindungen der Formel (I): 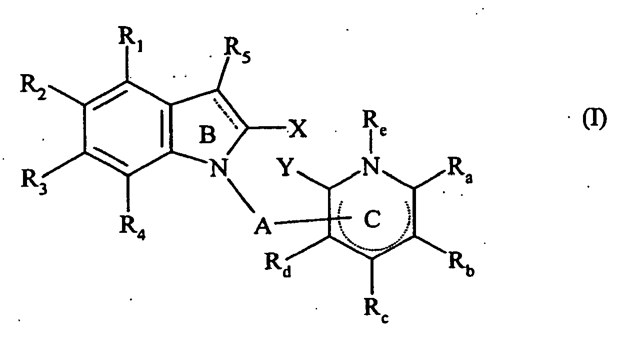 in der:
• A einen zweiwertigen Rest bedeutet: 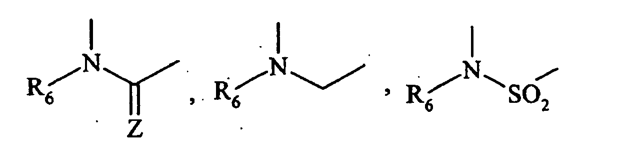
worin:
Z ein Sauerstoffatom oder ein Schwefelatom bedeutet,
R₆:
ein Wasserstoffatom,
eine geradkettige oder verzweigte (C₁-C₆)-Alkylgruppe, Gruppe C(O)-AA, worin AA einen Aminosäurerest darstellt, geradkettige oder verzweigte (C₁-C₆)-Alkoxycarbonylgruppe, Gruppe CHR'-O-C(O)-R", worin R' ein Wasserstoffatom oder eine geradkettige oder verzweigte (C₁-C₆)-Alkylgruppe und R" eine geradkettige oder verzweigte (C₁-C₆)-Alkylgruppe bedeuten,
geradkettige oder verzweigte (C₂-C₆)-Alkenylgruppe, Arylgruppe, geradkettige oder verzweigte Aryl-(C₁-C₆)-alkylgruppe, geradkettige oder verzweigte (C₁-C₆)-Polyhalogenalkylgruppe,
oder geradkettige oder verzweigte (C₁-C₆)-Alkylkette, die durch ein oder mehrere Halogenatome oder eine oder mehrere Hydroxygruppen, geradkettige oder verzweigte (C₁-C₆)-Alkoxygruppen oder gegebenenfalls durch eine oder zwei gleichartige oder verschiedenartige, geradkettige oder verzweigte (C₁-C₆)-Alkylgruppen substituierte Aminogruppe substituiert ist, bedeutet,
• in dem Ring B
----- für eine Einfachbindung oder eine Doppelbindung steht,
• in dem Ring C
----- für eine Einfachbindung oder eine Doppelbindung steht, wobei der Ring C höchstens eine einzige Doppelbindung enthält,
• R₁, R₂, R₃, R₄, die gleich oder verschieden sind, unabhängig voneinander:
ein Wasserstoffatom oder ein Halogenatom,
eine geradkettige oder verzweigte (C₁-C₆)-Alkylgruppe, geradkettige oder verzweigte (C₁-C₆)-Alkoxygruppe, Hydroxygruppe, Cyanogruppe, Nitrogruppe, geradkettige oder verzweigte (C₁-C₆)-Polyhalogenalkylgruppe, Aminogruppe (die gegebenenfalls durch eine oder zwei geradkettige oder verzweigte (C₁-C₆)-Alkylgruppen und/oder geradkettige oder verzweigte (C₂-C₆)-Alkenylgruppen substituiert ist, wobei die Alkyl- und Alkenylgruppe gleichartig oder verschieden sein können),
oder geradkettige oder verzweigte (C₁-C₆)-Alkylkette, die durch ein oder mehrere Halogenatome oder eine oder mehrere Hydroxygruppen, geradkettige oder verzweigte (C₁-C₆)-Alkoxygruppen oder gegebenenfalls durch eine oder zwei geradkettige oder verzweigte, gleichartige oder verschiedenartige (C₁-C₆)-Alkylgruppen substituierte Aminogruppe bedeutet,
• R₅:
ein Wasserstoffatom,
eine geradkettige oder verzweigte (C₁-C₆)-Alkylgruppe, geradkettige oder verzweigte (C₁-C₆)-Aminoalkylgruppe oder geradkettige oder verzweigte (C₁-C₆)-Hydroxyalkylgruppe bedeutet,
• X und Y, die gleichartig oder verschieden sind, unabhängig voneinander ein Wasserstoffatom oder eine geradkettige oder verzweigte (C₁-C₆)-Alkylgruppe bedeuten,
• Rₐ, R_{b}, R_{c} und R_{d}, die gleichartig oder verschieden sind, unabhängig voneinander:
ein Wasserstoffatom oder ein Halogenatom,
eine geradkettige oder verzweigte (C₁-C₆)-Alkylgruppe, Hydroxygruppe, geradkettige oder verzweigte (C₁-C₆)-Alkoxygruppe, Cyanogruppe, Nitrogruppe, geradkettige oder verzweigte (C₁-C₆)-Polyhalogenalkylgruppe, Aminogruppe (die gegebenenfalls durch eine oder zwei gleichartige oder verschiedenartige, geradkettige oder verzweigte (C₁-C₆)-Alkylgruppen substituiert ist),
geradkettige oder verzweigte (C₁-C₆)-Alkylkette, die durch eine oder mehrere Gruppen ausgewählt aus Halogen, Hydroxy, geradkettigem oder verzweigtem (C₁-C₆)-Alkoxy oder gegebenenfalls durch eine oder zwei gleichartige oder verschiedenartige, geradkettige oder verzweigte (C₁-C₆)-Alkylgruppen substituiertes Amino bedeuten,
mit der Maßgabe dass, wenn A über eines der Kohlenstoffatome, welches einen der Substituenten Rₐ, R_{b}, R_{c}, R_{d} oder Y trägt, an den Ring C gebunden ist und dieses Bindungs-Kohlenstoffatom auch eine Doppelbindung trägt, der entsprechende Substituent Rₐ, R_{b}, R_{c}, R_{d} oder Y nicht vorhanden ist,
• Rₑ:
ein Wasserstoffatom,
eine geradkettige oder verzweigte (C₁-C₆)-Alkylgruppe, geradkettige oder verzweigte Aryl-(C₁-C₆)-alkylgruppe, geradkettige oder verzweigte (C₂-C₆)-Alkenylgruppe, geradkettige oder verzweigte (C₂-C₆)-Alkinylgruppe, geradkettige oder verzweigte (C₁-C₆)-Alkylkette substituiert durch eine oder mehrere Gruppen ausgewählt aus Hydroxy, Amino (gegebenenfalls substituiert durch eine oder zwei gleichartige oder verschiedenartige, geradkettige oder verzweigte (C₁-C₆)-Alkylgruppen), geradkettigem oder verzweigtem (C₁-C₆)-Alkoxy oder NR₇R₈, worin R₇ und R₈ gemeinsam mit dem sie tragenden Stickstoffatom einen gegebenenfalls substituierten Heterocyclus mit 4 bis 8 Kettengliedern bilden, der gegebenenfalls eine oder mehrere Doppelbindungen im Heterocyclus aufweist und gegebenenfalls in dem cyclischen System ein zweites Heteroatom enthält ausgewählt aus dem Sauerstoffatom und dem Stickstoffatom, eine geradkettige oder verzweigte (C₂-C₆)-Alkenylkette, die durch die gleichen Gruppen substituiert ist wie die Alkylkette, oder eine geradkettige oder verzweigte (C₂-C₆)-Alkinylkette, die durch die gleichen Gruppen wie die Alkylkette substituiert ist, bedeutet,
deren Enantiomere, Diastereoisomere, N-Oxide sowie deren Additionssalze mit einer pharmazeutisch annehmbaren Säure oder Base,
wobei es sich versteht, dass man:
als gegebenenfalls substituierten Heterocyclus mit 4 bis 8 Kettengliedern, der gegebenenfalls eine oder mehrere Doppelbindungen im Heterocyclus enthält und gegebenenfalls in dem cyclischen System ein zweites Heteroatom aufweist ausgewählt aus dem Sauerstoffatom und dem Stickstoffatom in nicht einschränkender Weise nennen kann: Pyrrolidin, Piperidin, Azepan, Piperazin und Morpholin, wobei diese Heterocyclen gegebenenfalls substituiert sein können einschließlich an dem zweiten Stickstoffatom des Piperazins durch eine oder mehrere, gleichartige oder verschiedenartiger Gruppen ausgewählt aus geradkettigem oder verzweigtem (C₁-C₆)-Alkyl, geradkettigem oder verzweigtem (C₁-C₆)-Hydroxyalkyl, geradkettigem oder verzweigtem (C₁-C₆)-Alkoxy-(C₁-C₆)-alkyl, CO₂Rᵥ, CO₂-R_{w}-NRᵥR'ᵥ, CO₂-R_{w}-ORᵥ, worin Rᵥ ein Wasserstoffatom oder eine geradkettige oder verzweigte (C₁-C₆)-Alkylgruppe darstellt, R'ᵥ die gleichen Bedeutungen besitzt wie Rᵥ und R_{w} eine geradkettige oder verzweigte (C₁-C₆)-Alkylenkette, Aryl, Aryloxycarbonyl, geradkettiges oder verzweigtes Aryl-(C₁-C₆)-alkoxy, gegebenenfalls substituiertes Cycloalkyl, gegebenenfalls substituiertes Cycloalkylalkyl, gegebenenfalls substituiertes Heterocycloalkyl, gegebenenfalls substituiertes Heterocycloalkylalkyl oder geradkettiges oder verzweigtes (C₁-C₆)-Aminoalkyl, wobei der Aminorest gegebenenfalls durch eine oder zwei gleichartige oder verschiedenartige, geradkettige oder verzweigte (C₁-C₆)-Alkylgruppen substituiert ist, bedeutet,
man unter Aryl eine Phenyl- oder Naphthylgruppe versteht, wobei diese Gruppen gegebenenfalls durch ein oder mehrere Halogenatome, Nitrogruppen, Aminogruppen, geradkettige oder verzweigte (C₁-C₆)-Alkylgruppen oder geradkettige oder verzweigte (C₁-C₆)-Alkoxygruppen substituiert sein können,
man unter Cycloalkyl eine monocyclische gesättigte Gruppe versteht, die 4 bis 8 Kettenglieder aufweist,
man unter Cycloalkylalkyl die Cycloalkylalkylgruppe versteht, in der die Alkylgruppe für eine geradkettige oder verzweigte Kette mit 1 bis 6 Kohlenstoffatomen steht, und die Cycloalkylgruppe eine monocyclische gesättigte Gruppe mit 4 bis 8 Kettengliedern bedeutet,
man unter Heterocycloalkyl eine monocyclische gesättigte Gruppe versteht, die 4 bis 8 Kettenglieder aufweist und 1 oder 2 Heteroatome ausgewählt aus Stickstoff, Sauerstoff und Schwefel umfasst,
man unter Heterocycloalkylalkyl die Heterocycloalkyl-alkylgruppe versteht, in der die Alkylgruppe eine geradkettige oder verzweigte Kette mit 1 bis 6 Kohlenstoffatomen bedeutet und die Heterocycloalkylgruppe für eine monocyclische gesättigte Gruppe steht, die 4 bis 8 Kettenglieder aufweist und 1 oder 2 Heteroatome ausgewählt aus Stickstoff, Sauerstoff und Schwefel umfasst,
der Begriff "gegebenenfalls substituiert" im Hinblick auf die Cycloalkyl-, Cycloalkylalkyl-, Heterocycloalkyl- und Heterocycloalkylalkyl-gruppe bedeutet, dass diese Gruppen durch 1 oder mehrere, gleichartige oder verschiedenartige Substituenten substituiert sein können, ausgewählt aus geradkettigem oder verzweigtem (C₁-C₆)-Alkyl, geradkettigem oder verzweigtem (C₁-C₆)-Hydroxyalkyl, geradkettigem oder verzweigtem (C₁-C₆)-Alkoxy-(C₁-C₆)-alkyl, Carboxy, geradkettigem oder verzweigtem (C₁-C₆)-Alkoxycarbonyl und geradkettigem oder verzweigtem (C₁-C₆)-Aminoalkyl, wobei der Aminorest gegebenenfalls durch eine oder zwei gleichartige oder verschiedenartige, geradkettige oder verzweigte (C₁-C₆)-Alkylgruppen substituiert ist,
man unter einem Aminosäurerest die Reste Alanyl, Arginyl, Asparaginyl, α-Aspartyl, Cysteinyl, α-Glutamyl, Glutaminyl, Glycyl, Histidyl, Isoleucyl, Leucyl, Lysyl, Methionyl, Phenylalanyl, Prolyl, Seryl, Threonyl, Thryptophyl, Tyrosyl und Valyl versteht.

2. Verbindungen der Formel (I) nach Anspruch 1, **dadurch gekennzeichnet, dass** A den folgenden zweiwertigen Rest bedeutet: 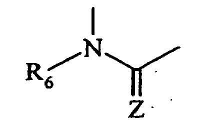 in dem R₆ die bezüglich der Formel (I) angegebenen Bedeutungen besitzt und Z ein Sauerstoffatom darstellt, deren Enantiomere, Diastereoisomere sowie ihre Additionssalze mit einer pharmazeutisch annehmbaren Säure oder Base.

3. Verbindungen der Formel (I) nach einem der Ansprüche 1 bis 2, **dadurch gekennzeichnet, dass** R₆ ein Wasserstoffatom bedeutet, deren Enantiomere, Diastereoisomere, N-Oxide sowie ihre Additionssalze mit einer pharmazeutisch annehmbaren Säure oder Base.

4. Verbindungen der Formel (I) nach einem der Ansprüche 1 bis 3, **dadurch gekennzeichnet, dass** R₁, R₂, R₃ und R₄ ein Wasserstoffatom, ein Halogenatom oder eine geradkettige oder verzweigte (C₁-C₆)-Alkoxygruppe bedeuten, deren Enantiomere, Diastereoisomere, N-Oxide sowie ihre Additionssalze mit einer pharmazeutisch annehmbaren Säure oder Base.

5. Verbindungen der Formel (I) nach einem der Ansprüche 1 bis 4, **dadurch gekennzeichnet, dass** R₅ ein Wasserstoffatom oder eine geradkettige oder verzweigte (C₁-C₆)-Alkylgruppe bedeutet, deren Enantiomere, Diastereoisomere, N-Oxide sowie ihre Additionssalze mit einer pharmazeutisch annehmbaren Säure oder Base.

6. Verbindungen der Formel (I) nach einem der Ansprüche 1 bis 5, **dadurch gekennzeichnet, dass** X und Y ein Wasserstoffatom oder eine geradkettige oder verzweigte (C₁-C₆)-Alkylgruppe bedeuten, deren Enantiomere, Diastereoisomere, N-Oxide sowie ihre Additionssalze mit einer pharmazeutisch annehmbaren Säure oder Base.

7. Verbindungen der Formel (I) nach einem der Ansprüche 1 bis 6, **dadurch gekennzeichnet, dass** Rₐ, R_{b}, R_{c} und R_{d} ein Wasserstoffatom bedeuten, deren Enantiomere, Diastereoisomere, N-Oxide sowie ihre Additionssalze mit einer pharmazeutisch annehmbaren Säure oder Base.

8. Verbindungen der Formel (I) nach einem der Ansprüche 1 bis 7, **dadurch gekennzeichnet, dass** Rₑ ein Wasserstoffatom oder eine geradkettige oder verzweigte (C₁-C₆)-Alkylgruppe oder eine geradkettige oder verzweigte (C₂-C₆)-Alkenylgruppe bedeutet, deren Enantiomere, Diastereoisomere, N-Oxide sowie ihre Additionssalze mit einer pharmazeutisch annehmbaren Säure oder Base.

9. Verbindungen der Formel (I) nach einem der Ansprüche 1 bis 8, **dadurch gekennzeichnet, dass** sie der Formel (I/A) entsprechen: 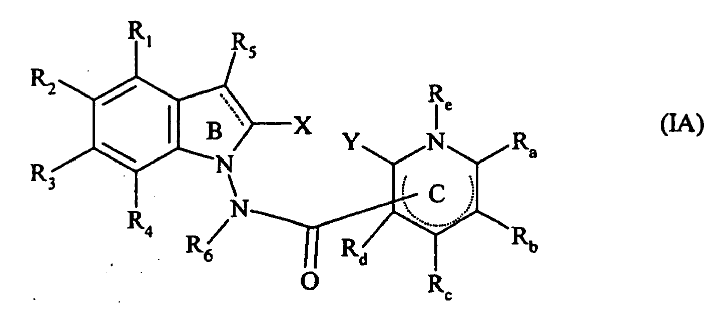 in der -----, X, Y, R₁, R₂, R₃, R₄, R₅, R₆, Rₐ. R_{b}, R_{c}, R_{d} und Rₑ die bezüglich der Formel (I) angegebenen Bedeutungen besitzen, deren Enantiomere, Diastereoisomere, N-Oxide sowie ihre Additionssalze mit einer pharmazeutisch annehmbaren Säure oder Base.

10. Verbindungen der Formel (I) nach einem der Ansprüche 1 bis 9, **dadurch gekennzeichnet, dass** sie der Formel (I/B) entsprechen: 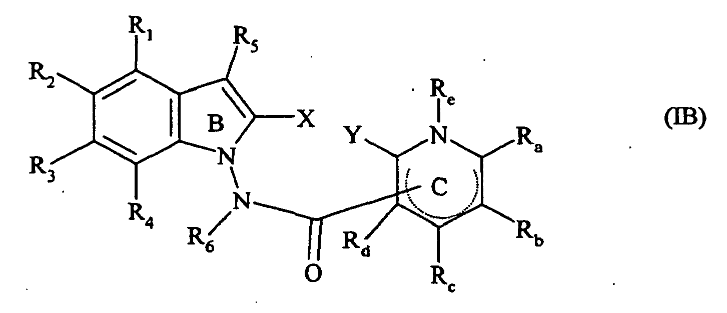 in der -----, X, Y, R₁, R₂, R₃, R₄, R₅, R₆, Rₐ, R_{b}, R_{c}, R_{d} und Rₑ die bezüglich der Formel (I) angegebenen Bedeutungen besitzen, deren Enantiomere, Diastereoisomere, N-Oxide sowie ihre Additionssalze mit einer pharmazeutisch annehmbaren Säure oder Base.

11. Verbindungen der Formel (I) nach einem der Ansprüche 1 bis 10, **dadurch gekennzeichnet, dass** sie der Formel (I/C) entsprechen: 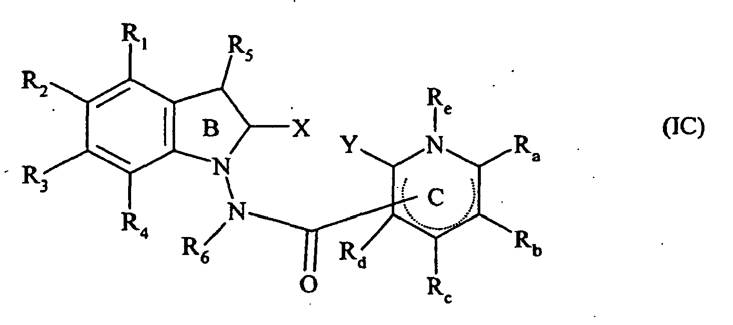 in der -----, X, Y, R₁, R₂, R₃, R₄, R₅, R₆, Rₐ, R_{b}, R_{c}, R_{d} und Rₑ die bezüglich der Formel (I) angegebenen Bedeutungen besitzen, deren Enantiomere, Diastereoisomere, N-Oxide sowie ihre Additionssalze mit einer pharmazeutisch annehmbaren Säure oder Base.

12. Verbindungen der Formel (I) nach einem der Ansprüche 1 bis 11, **dadurch gekennzeichnet, dass** sie der Formel (I/D) entsprechen: 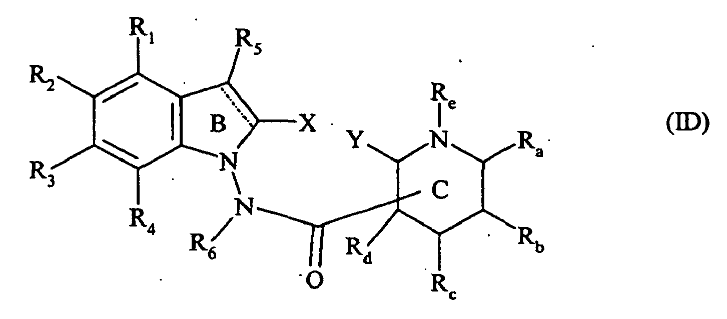 in der -----, X, Y, R₁, R₂, R₃, R₄, R₅, R₆, Rₐ. R_{b}, R_{c}, R_{d} und Rₑ die bezüglich der Formel (I) angegebenen Bedeutungen besitzen, deren Enantiomere, Diastereoisomere, N-Oxide sowie ihre Additionssalze mit einer pharmazeutisch annehmbaren Säure oder Base.

13. Verbindungen der Formel (I) nach einem der Ansprüche 1 bis 12, **dadurch gekennzeichnet, dass** sie der Formel (I/E) entsprechen: 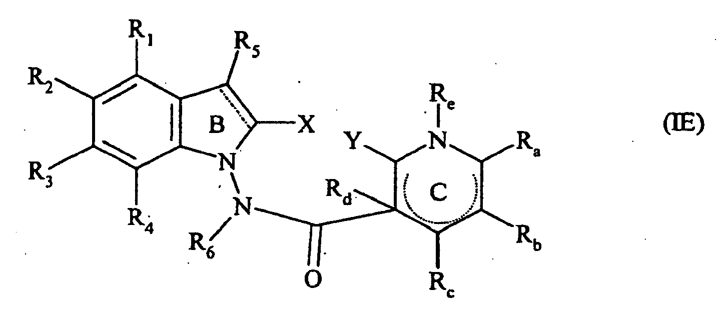 in der -----, X, Y, R₁, R₂, R₃, R₄, R₅, R₆, Rₐ, R_{b}, R_{c}, R_{d} und Rₑ die bezüglich der Formel (I) angegebenen Bedeutungen besitzen, deren Enantiomere, Diastereoisomere, N-Oxide sowie ihre Additionssalze mit einer pharmazeutisch annehmbaren Säure oder Base.

14. Verbindungen der Formel (I) nach einem der Ansprüche 1 bis 13, **dadurch gekennzeichnet, dass** sie der Formel (I/F) entsprechen: 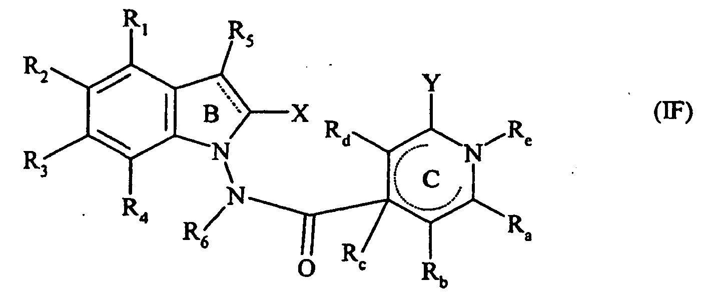 in der -----, X, Y, R₁, R₂, R₃, R₄, R₅, R₆, Rₐ, R_{b}, R_{c}, R_{d} und Rₑ die bezüglich der Formel (I) angegebenen Bedeutungen besitzen, deren Enantiomere, Diastereoisomere, N-Oxide sowie ihre Additionssalze mit einer pharmazeutisch annehmbaren Säure oder Base.

15. Verbindungen der Formel (I) nach einem der Ansprüche 1 bis 14, **dadurch gekennzeichnet, dass** sie der Formel (I/G) entsprechen: 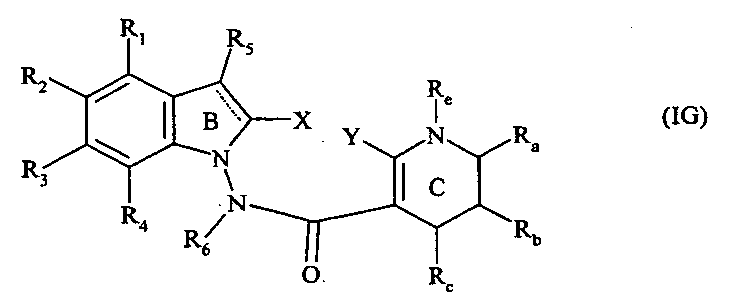 in der -----, X, Y, R₁, R₂, R₃, R₄, R₅, R₆, Rₐ, R_{b}, Rₑ. R_{d} und Rₑ die bezüglich der Formel (I) angegebenen Bedeutungen besitzen, deren Enantiomere, Diastereoisomere, N-Oxide sowie ihre Additionssalze mit einer pharmazeutisch annehmbaren Säure oder Base.

16. Verbindungen der Formel (I) nach einem der Ansprüche 1 bis 15, **dadurch gekennzeichnet, dass** sie der Formel (I/H) entsprechen: 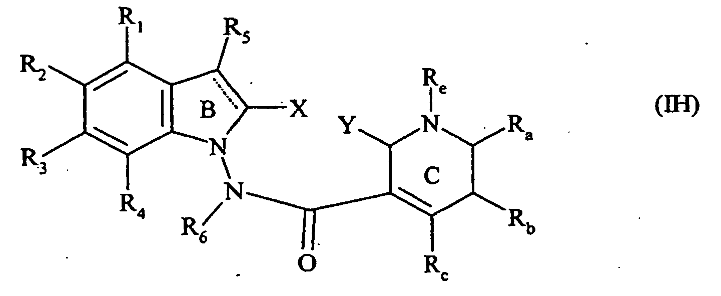 in der -----, X, Y, R₁, R₂, R₃, R₄, R₅, R₆, Rₐ, R_{b}, R_{c}, R_{d} und Rₑ die bezüglich der Formel (I) angegebenen Bedeutungen besitzen, deren Enantiomere, Diastereoisomere, N-Oxide sowie ihre Additionssalze mit einer pharmazeutisch annehmbaren Säure oder Base.

17. Verbindungen der Formel (I) nach einem der Ansprüche 1 bis 16, **dadurch gekennzeichnet, dass** sie der Formel (I/J) entsprechen: 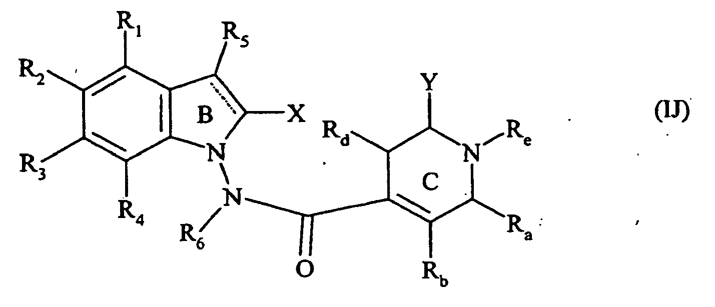 in der -----, X, Y, R₁, R₂, R₃, R₄, R₅, R₆, Rₐ, R_{b}, R_{c}, R_{d} und Rₑ die bezüglich der Formel (I) angegebenen Bedeutungen besitzen, deren Enantiomere, Diastereoisomere, N-Oxide sowie ihre Additionssalze mit einer pharmazeutisch annehmbaren Säure oder Base.

18. Verbindungen der Formel (I) nach Anspruch 1, nämlich:
• *N*-(1*H*-Indol-1-yl)-1-methyl-1,2,5,6-tetrahydropyridin-3-carboxamid,
• *N*-(2,3-Dihydro-1*H*-indol-1-yl)-1-methyl-1,4,5,6-tetrahydropyridin-3-carboxamid,
• *N*-(5-Fluor-1*H*-indol-1-yl)-1-methyl-1,2,5,6-tetrahydropyridin-3-carboxamid,
• *N*-(2,3-Dihydro-1*H*-indol-1-yl)-1-methyl-1,4,5,6-tetrahydropyridin-3-carboxamid,
• 1-[2-(Dimethylamino)-ethyl]-*N*-(1*H*-indol-1-yl)-1,2,5,6-tetrahydropyridin-3-carboxamid,
• *N*-(1*H*-Indol-1-yl)-1-[2-(4-methyl-1-piperazinyl)-ethyl]-3-piperidincarboxamid,
• *N*-(5-Chlor-1*H*-indol-1-yl)-1-(2-hydroxyethyl)-1,4,5,6-tetrahydropyridin-3-carboxamid,
• 4-(2-{3-[(1*H*-Indol-1-ylamino)-carbonyl]-1-piperidinyl}-ethyl)-piperazin-1-carbonsäure-*tert*.-butylester,
• 1-[3-(Dimethylammonium)-propyl]-3-[(1*H*-indol-1-ylamino)-carbonyl]-piperidinium,
• *N*-(1*H*-Indol-1-yl)-1-[3-(1-piperidinyl)-propyl]-3-piperidincarboxamid,
• *N*-(1H-Indol-1-yl)-1-[3-(4-methyl-1-piperazinyl)-propyl]-3-piperidincarboxamid,
• N-(Indol-1-yl)-1-(2-piperidin-1-yl-ethyl)-1,2,5,6-tetrahydropyridin-3-carboxamid,
• (±)-N-(Indol-1-yl)-1-[2-[4-(1-methylpiperidin-4-yl)-piperazin-1-yl]-ethyl]-piperidin-3-carboxamid,
• (±)-N-(Indol-1-yl)-1-[3-[4-(2-hydroxyethyl)-piperazin-1-yl)]-propyl]-piperidin-3-carboxamid,
• (±)-N-(Indol-1-yl)-1-[4-(4-mefhylpiperazin-1-yl)-butyl]-piperidin-3-carboxamid,
• (±)-N-(Indol-1-yl)-1-allylpiperidin-3-carboxamid,
• (±)-N-(Indol-1-yl)-1-[4-(piperidin-1-yl)-but-2-en-1-yl]-piperidin-3-carboxamid,
• (R oder S) (-)-N-(lndol-1-yl)-1-[2-(piperidin-l-yl)-ethyl]-piperidin-3-carboxamid Enantiomeres 1,
• (R oder S) (+)-N-(Indol-1-yl)-1-[2-(piperidin-1-yl)-ethyl]-piperidin-3-carboxamid Enantiomeres 2,
deren Enantiomere, Diastereoisomere, N-Oxide sowie ihre Additionssalze mit einer pharmazeutisch annehmbaren Säure oder Base.

19. Verfahren zur Herstellung der Verbindungen der Formel (I) nach Anspruch 1, **dadurch gekennzeichnet, dass** man als Ausgangsprodukt eine Verbindung der Formel (II) verwendet: 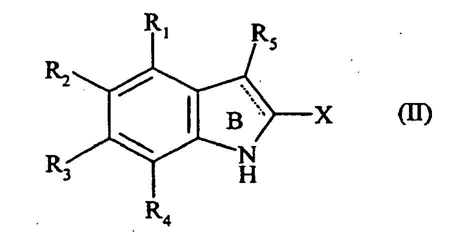 in der R₁, R₂, R₃, R₄. R₅ und X die bezüglich der Formel (I) angegebenen Bedeutungen besitzen,
welche Verbindung man in Gegenwart von Diphenylphosphinylhydroxylamin umsetzt zur Bildung der Verbindung der Formel (III): 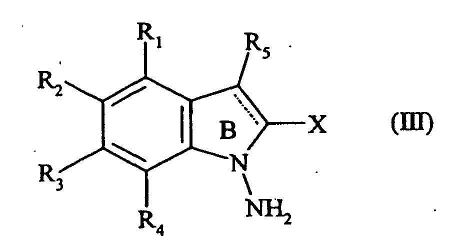 in der R₁, R₂, R₃, R₄, R₅ und X die oben angegebenen Bedeutungen besitzen,
welche Verbindung der Formel (III) mit einer Verbindung der Formel (IV) kondensiert wird: 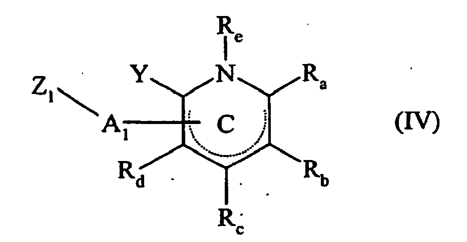 in der Rₐ, R_{b}, R_{c}, R_{d}, Rₑ und Y die bezüglich der Formel (I) angegebenen Bedeutungen besitzen, A₁ eine Gruppe der Formel -C(=Z)-, -CH₂-, -SO₂- darstellt, worin Z die bezüglich der Formel (I) angegebenen Bedeutungen besitzt und Z₁ eine Gruppe ausgewählt aus Hydroxy, Ethoxy oder Methoxy bedeutet,
zur Bildung der Verbindung der Formel (I/a), einem Sonderfall der Verbindungen der Formel (I): 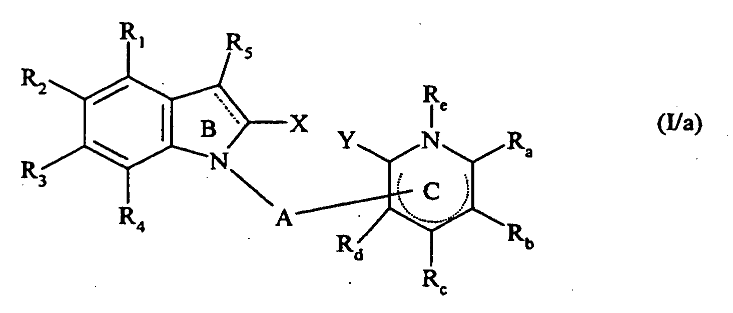 in der R₁, R₂, R₃, R₄, R₅, Rₐ, R_{b}, R_{c}, R_{d}, Rₑ, X und Y die oben angegebenen Bedeutungen besitzen, und A die bezüglich der Formel (I) angegebenen Bedeutungen besitzt,
welche Verbindungen der Formel (I/a), welche die Gesamtheit der erfindungsgemäßen Verbindungen bilden, gegebenenfalls mit Hilfe einer klassischen Reinigungsmethode gereinigt werden, gegebenenfalls mit Hilfe einer klassischen Trennmethode in ihre verschiedenen Isomeren aufgetrennt werden und die gewünschtenfalls in ihre N-Oxide umgewandelt werden können und gewünschtenfalls in ihre Additionssalze mit einer pharmazeutisch annehmbaren Säure oder Base überführt werden können.

20. Pharmazeutische Zubereitungen, enthaltend als Wirkstoff mindestens eine Verbindung nach einem der Ansprüche 1 bis 18 in Kombination mit einem oder mehreren inerten, nichttoxischen, pharmazeutisch annehmbaren Hilfsstoffen.

21. Pharmazeutische Zubereitungen nach Anspruch 20, enthaltend mindestens einen Tyrosinhydroxylase induzierenden Wirkstoff nach einem der Ansprüche 1 bis 18, die nützlich sind zur Behandlung von Depressionen, Angst, Gedächtnisstörungen infolge des Alterns und/oder von neurodegenerativen Erkrankungen und bei der palliativen Behandlung der Parkinsonschen Krankheit und zur Anpassung an Stress.
